# EUROPEAN PATENT APPLICATION

(11) **EP 3 028 743 A2**
(43) Date of publication of application: **08.06.2016**
(21) Application number: 15191023.9
(22) Date of filing: 05.03.2009
(51) Int. Cl.: A61P 35/00, A61K 31/195, A61K 45/06, A61K 31/00, A61K 31/407, A61K 31/138

(54) **COMPOSITIONS FOR MUCOSITIS AND ONCOLOGY THERAPIES**

(30) Priority: 05.03.2008 US 34122; 10.07.2008 US 79768; 05.08.2008 US 86437; 12.01.2009 US 144121
(62) Divisional of application: 09716791.0
(71) Applicant: Vicus Therapeutics, LLC, Morristown, NJ 07960 (US)
(72) Inventor: BASCOMB, Newell, Morristown, New Jersey 07960 (US); MAKI, John, Morristown, New Jersey 07960 (US); YOUNG, Frederic, Morristown, New Jersey 07960 (US)
(74) Representative: Wilkinson, Marc George

(57) **Abstract**

In alternative embodiments, this invention provides compositions and methods for treating cancer or any condition caused by dysfunctional cells, side effects from treatments for cancer or any condition caused by dysfunctional cells, e.g., mucositis therapies (e.g., for oral mucositis; digestive mucositis; esophageal mucositis; intestinal mucositis). In alternative embodiments, the invention provides cytoprotection products that may be used either alone or in combination with other medical therapies such as cancer chemotherapies and radiation therapies.

## Description

### FIELD OF THE INVENTION

This invention relates generally to medicine and pharmaceutical formulations. In alternative embodiments, this invention provides cancer and mucositis therapies (e.g., for oral mucositis; digestive mucositis; esophageal mucositis; intestinal mucositis) and cytoprotection products that may be used either alone or in combination with other medical therapies, such as cancer chemotherapies and radiation therapies.

### BACKGROUND

Oral mucositis is an adverse side effect of chemotherapy and radiation. Current cancer treatment methods commonly include chemotherapy and radiation therapy. These treatments are associated with adverse effects, even though they are used with the desire to extend patient survival and improve quality of life. For example, the severe adverse reactions to these therapies include increased patient morbidity and mortality. There are approximately 400,000 cases of treatment-induced damage to the oral cavity worldwide every year. Cytotoxicity from chemotherapy and radiotherapy often results in oral mucositis with associated illness and pain, including odynophagia (e.g. painful swallowing), dysgeusia (e.g. distortion and/or decrease of the sense of taste) and subsequent dehydration and malnutrition. Clinically, this toxicity is ranges from slight erythema (e.g. redness of the skin associated with capillary congestion) and edema of the oral mucosa (e.g. swelling due to an increase in interstitial fluid) to severe, focal or widespread ulceration, bleeding and exudation (e.g. oozing from sores).

### SUMMARY OF THE INVENTION

The invention provides cyto-protection products and therapies that may be used either alone or in combination with other medical therapies such as cancer chemotherapies and radiation therapies. The products and methods of the invention can be used to: treat or ameliorate cancer (including any tumor, benign or metastatic), or treat, abolish, ameliorate, diminish, improve, and/or inhibit unwanted side effects and disease state symptoms, e.g., of a mucositis, such as an oral mucositis, digestive mucositis, esophageal mucositis, and/or intestinal mucositis, or any other side effect resulting from a cancer treatment.

In alternative embodiments, the invention provides a therapeutic combination or combinations of drugs for an individual in need thereof comprising or consisting of:
(i) (a) a beta adrenergic receptor antagonist; (b) a non-steroidal anti-inflammatory drug (a NSAID); and (c) a therapeutic agent for the treatment of cancer;
(ii) (a) a macrolide or a composition comprising a macrolide ring, and (b) a therapeutic agent for the treatment of cancer;
(iii) (a) an immunosuppressant composition or pharmaceutical, and (b) a therapeutic agent for the treatment of cancer;
(iv) (a) a proton pump inhibitor (a PPI), and (b) a therapeutic agent for the treatment of cancer;
(v) any combination of the therapeutic combination of drugs of (i), (ii), (iii) and/or (iv); or
(vi) the combination of any of (i) to (v) further comprising a proton pump inhibitor (a PPI); a synthetic prostaglandin E₁ (PGE₁) analogue misoprostol; *N*-(4-hydroxyphenyl)acetamide (paracetamol or acetaminophen); 2-[4-(2-methylpropyl)phenyl]propanoic acid (ibuprofen); an anticonvulsant or antiseizure drug; an neuropathic pain analgesic; an opioid (opiate) painkiller (analgesic); an antibiotic; an antidepressant or antipsychotic; or, further comprising any combination thereof.

The therapeutic combination of claim 1, wherein (i) the non-steroidal anti-inflammatory drug (a NSAID) comprises (a) a cyclooxygenase (COX) (or prostaglandin synthase) inhibitor; or, (b) the COX inhibitor of (a), wherein the COX inhibitor comprises or consists of an etodolac or equivalent; a naproxen or equivalent; a celecoxib or equivalent; a rofecoxib or equivalent; a etoricoxib or equivalent; a valdecoxib or equivalent; a parecoxib or equivalent; a nabumetone or equivalent; a diclofenac (2-(2,6-dichloranilino) phenylacetic acid) or equivalent; or, a lumiracoxib or equivalent; (ii) the neuropathic pain analgesic comprises or consists of gabapentin or pregabalin; or (iii) the antisense or siRNA nucleic acid comprises or consists of oblimersen or GENASENSE™.

In alternative embodiments of the therapeutic combination(s) of the invention, the etodolac is LODINE™, LODINE SR™ or ECCOXOLAC™; or the celecoxib is CELEBREX™ or CELEBRA™; or the rofecoxib is VIOXX™, CEOXX™ or CEEOXX™; or the etoricoxib is ARCOXIA™, ALGIX™ or TAUXIB™; or the valdecoxib is BEXTRA™; the parecoxib is DYNASTAT™; the naproxen is XENOBID™, ALEVE™, ANAPROX™, MIRANAX™, NAPROGESIC™, NAPROSYN™, NAPRELAN™, PROXEN™ or SYNFLEX™; the nabumetone is RELAFEN™, RELIFEX™ or GAMBARAN™; or, the diclofenac is FLECTOR PATCH™, VOLTAREN™, VOLTAROL™, DICLON™, DICLOFLEX DIFEN™, DIFENE™, CATAFLAM™, PENNSAID™, PANAMOR™, RHUMALGAN™, MODIFENAC™, ABITREN™, OLFEN™, VOVERAN™, ARTHROTEC™, DEDOLOR™, DEFLAMAT™, VETAGESIC™ or ZOLTEROL™.

In alternative embodiments of the therapeutic combination(s) of the invention, the beta adrenergic receptor antagonist (a beta blocker) comprises propranolol or equivalent; or, the propranolol is INDERAL™, AVLOCARDYL™, DERALIN™, DOCITON™, INDERALICI™, INNOPRAN XL™, or SUMIAL™.

In alternative embodiments of the therapeutic combination(s) of the invention, the beta adrenergic receptor antagonist (a beta blocker) comprises propranolol or equivalent and the non-steroidal anti-inflammatory drug (a NSAID) comprises etodolac or equivalent.

In alternative embodiments of the therapeutic combination(s) of the invention, the therapeutic agent for the treatment of cancer comprises or consists of a monoclonal antibody, a peptide, a synthetic polypeptide or peptidomimetic, a nucleic acid, a synthetic nucleic acid, a lipid, a carbohydrate and/or a small molecule.

In alternative embodiments of the therapeutic combination(s) of the invention, the therapeutic agent for the treatment of cancer comprises or consists of a sorafenib or equivalent, or NEXAVAR™; a sunitinib or equivalent, or SUTENT™; an erlotinib or equivalent, or TARCEVA™; an imatinib or equivalent, or GLEEVEC™; a lapatinib or equivalent, or TYKERB™; a bevacizumab or equivalent, or AVASTIN™; a trastuzumab or equivalent, or HERCEPTIN™; a cetuximab or equivalent, or ERBITUX™; a bevacizumab or equivalent, or AVASTIN™ or BIBW 2992; a gefitinib or equivalent, or IRESSA™; a ranibizumab or equivalent, or LUCENTIS™; a pegaptanib or equivalent, or MACUGEN™; a dasatinib or equivalent, or BMS-354825™; a sunitinib or equivalent, or SUTENT™; a pazopanib or equivalent; a nilotinib or equivalent, or TASIGNA™; a panitumumab or equivalent, or VECTIBIX™; a bandetinib or equivalent; a brivanib or equivalent, or E7080™; thalidomide or equivalent, or THALOMID™; lenalidomide or equivalent, or REVLIMID™; bortezomib or equivalent, or VELCADE™; disulfiram or equivalent, or ANTABUSE™ or ANTABUS™; or epigallocatechin gallate (EGCG) or equivalent; a demecolcine,; an etoglucid or elsamitrucin, a lonidamine, a lucanthone, a mitotane or a mitoguazone or equivalent; or any combination thereof.

In alternative embodiments of the therapeutic combination(s) of the invention, the therapeutic agent for the treatment of cancer comprises or consists of a protein kinase inhibitor; and the protein kinase inhibitor can comprise or consist of a tyrosine kinase inhibitor or a serine/threonine kinase inhibitor.

In alternative embodiments of the therapeutic combination(s) of the invention, the therapeutic agent for the treatment of cancer comprises or consists of an angiogenesis inhibitor; and the angiogenesis inhibitor can comprise or consist of a vascular endothelial growth factor (VEGF)-mediated angiogenesis inhibitor.

In alternative embodiments of the therapeutic combination(s) of the invention, the therapeutic agent for the treatment of cancer comprises or consists of an inducer of apoptosis or a mitotic and anti-microtubule inhibitor (inhibition of microtubule function); and the inducer of apoptosis or mitotic or anti-microtubule inhibitor can comprise or consist of a raltitrexed or equivalent, or TOMUDEX™; a doxorubicin or equivalent, or ADRIAMYCIN™; a fluorouracil or 5-fluorouracil or equivalent; a paclitaxel or equivalent, or TAXOL™ or ABRAXANE™; a docetaxel or equivalent, or TAXOTERE™; a larotaxel, tesetaxel or ortataxel or equivalent; an epothilone or an epothilone A, B, C, D, E or F or equivalent; an ixabepilone (also known as azaepothilone B) or equivalent, or BMS-247550™; a vincristine (also known as leurocristine) or equivalent, or ONCOVIN™; a vinblastin, vinblastine, vindesine, vinflunine, vinorelbine or NAVELBINE™ or equivalent; or, any combination thereof.

In alternative embodiments of the therapeutic combination(s) of the invention, the therapeutic agent for the treatment of cancer comprises or consists of an alkylating agent; and the alkylating agent can comprise or consist of a cisplatin or equivalent; a cisplatinum or equivalent; a *cis*-diamminedichloridoplatinum(II) (CDDP) or equivalent; a carboplatin or equivalent; a oxaloplatin or equivalent; a cyclophosphamide (cytophosphane) or equivalent, or ENDOXAN™, CYTOXAN™, NEOSAR™ or REVIMMUNE™; a mechlorethamine or equivalent; a chlormethine or equivalent; a mustine or equivalent; a nitrogen mustard or equivalent; a chlorambucil or equivalent, or LEUKERAN™; or, a combination thereof.

In alternative embodiments of the therapeutic combination(s) of the invention, the therapeutic agent for the treatment of cancer comprises or consists of a topoisomerase inhibitor; and the topoisomerase inhibitor can comprise or consist of an etoposide or equivalent, or EPOSIN™, ETOPOPHOS™, VEPESID™ or VP-16™; an amsacrine or equivalent; a topotecan or equivalent, or HYCAMTIN™; a teniposide or equivalent, or VUMON™ or VM-26™; an epipodophyllotoxin or equivalent; a camptothecin or equivalent; an irinotecan or equivalent, or CAMPTOSAR™; or, a combination thereof.

In alternative embodiments of the therapeutic combination(s) of the invention, the therapeutic agent for the treatment of cancer comprises or consists of an antibiotic, e.g., a glycopeptide antibiotic; and the glycopeptide antibiotic can comprise or consist of a bleomycin or equivalent or a bleomycin A₂ or B₂ or equivalent; a mitomycin or a mitomycin C or equivalent, a plicamycin (also known as mithramycin) or equivalent, or MITHRACIN™; or, a combination thereof.

In alternative embodiments of the therapeutic combination(s) of the invention, the therapeutic agent for the treatment of cancer comprises or consists of a steroid receptor inhibitor or steroid inhibitor (an anti-steroid); and the steroid receptor inhibitor can comprise or consist of an estrogen receptor modulator (a SERM), and the estrogen receptor modulator can comprise or consist of a tamoxifen or equivalent, or NOLVADEX™, ISTUBAL™ or VALODEX™. In alternative embodiments the steroid inhibitor or an anti-steroid comprises or consists of a finasteride or equivalent, or PROSCAR™, PROPECIA™, FINCAR™, FINPECIA™, FINAX™, FINAST™, FINARA™, FINALO™, PROSTERIDE™, GEFINA™, APPECIA™, FINASTERID IVAX™, FINASTERID or ALTERNOVA™.

In one embodiment of the therapeutic combination(s) of the invention, the therapeutic agent for the treatment of cancer comprises or consists of a matrix metalloproteinase (MMP) inhibitor, or comprises or consists of an inhibitor of a collagenase, a gelatinase and/or a stromelysin enzyme; and the matrix metalloproteinase (MMP) inhibitor can comprise or consist of batimastat, prinomastat or marimastat.

In alternative embodiments of the therapeutic combination(s) of the invention, the macrolide or composition comprising a macrolide ring comprises or consists of a macrolide antibiotic; or the macrolide or composition comprising a macrolide ring can comprise or consist of a clarithromycin or equivalent, or BIAXIN™, KLARICID™, KLABAX™, CLARIPEN™, CLARIDAR™, FROMILID™ or CLACID™; an azithromycin or equivalent, or ZITHROMAX™, ZITROMAX™ or SUMAMED™; a dirithromycin or equivalent; an erythromycin or equivalent; a roxithromycin or equivalent, or ROXO™, SURLID™, RULIDE™, BIAXSIG™, ROXAR™, ROXIMYCIN™ or COROXIN™; a telithromycin or equivalent or KETEK™; a josamycin or equivalent; a kitasamycin or equivalent; a midecamycin or equivalent; oleandomycin or equivalent; a roxithromycin or equivalent, or ROXO™, SURLID™, RULIDE™, BIAXSIG™, ROXAR™, ROXIMYCIN™ or COROXIN™; a troleandomycin or equivalent; or a tylosin or equivalent; or, any combination thereof.

In alternative embodiments of the therapeutic combination(s) of the invention, the immunosuppressant composition or pharmaceutical comprises or consists of a sirolimus or equivalent (also known as rapamycin), or RAPAMUNE™; a tacrolimus or equivalent, or FK-506™ or FUJIMYCIN™; a ciclosporin (or cyclosporine or cyclosporin) or equivalent; or a cortisone or equivalent.

In alternative embodiments of the therapeutic combination(s) of the invention, the proton pump inhibitor (a PPI) comprises or consists of an H₂-receptor antagonist (H₂RA); and the H₂-receptor antagonist (H₂RA) can comprise or consist of a cimetidine or equivalent, or TAGAMET™, TAGAMET HB™ or TAGAMET HB200™; a ranitidine or equivalent, or TRITEC™ or ZANTAC™; a famotidine or equivalent, or PEPCIDINE™ or PEPCID™; a nizatidine or equivalent, or TAZAC™ or AXID™, or the proton pump inhibitor (PPI) can comprise or consist of a benzimidazole compound or structure, or an imidazopyridine compound or structure; e.g., the imidazopyridine compound or structure can comprise or consist of a zolpidem or equivalent, or AMBIEN™, AMBIEN CR™, IVEDAL™, NYTAMEL™, STILNOCT™, STILNOX™, ZOLDEM™, ZOLNOD™ or ZOLPIHEXAL™; an alpidem (also called ananxyl) or equivalent; a saripidem or equivalent; necopidem or equivalent.

In alternative embodiments of the therapeutic combination(s) of the invention, one, two, three, four or five or more drugs of the therapeutic combination are formulated as separate compositions; or one, two, three, four or five or more drugs of the therapeutic combination are formulated into one composition or drug formulation (e.g., one, two, three, four or five or more or more drugs of the therapeutic combination are formulated or tableted together).

In alternative embodiments of the therapeutic combination(s) of the invention, the beta adrenergic receptor antagonist, or a beta blocker or equivalent, or a propranolol or equivalent; the non-steroidal anti-inflammatory drug, or a NSAID or equivalent, or an etodolac or equivalent; and the therapeutic agent for the treatment of cancer, are formulated (e.g., tableted) or in different compositions or formulations, or, are formulated in the same composition or formulation, or are formulated (e.g., tableted) or together.

In alternative embodiments of the therapeutic combination(s) of the invention, one, two, three, four or five or more or all of the drugs of the therapeutic combination are formulated (e.g., tableted) or packaged individually, or are packaged together, or packaged in any combination, in a single package, a plurality of packages or packettes, or a blister packet, lidded blister or blister card or packets, or a shrink wrap. For example, the beta adrenergic receptor antagonist, or a beta blocker or equivalent, or a propranolol or equivalent; the non-steroidal anti-inflammatory drug, or a NSAID or equivalent, or an etodolac or equivalent; and the therapeutic agent for the treatment of cancer, can be packaged individually in a single package, a plurality of packages or packettes, or a blister packet, lidded blister or blister card or packets, or a shrink wrap.

In alternative embodiments of the therapeutic combination(s) of the invention, one, two, three, four or five or more or all of the drugs of the therapeutic combination are formulated (e.g., tableted) or packaged together or in any combination in a single package, a plurality of packages or packettes, or a blister packet, lidded blister or blister card or packets, or a shrink wrap. In one embodiment, one, two, three, four or five or more or all of the drugs are released upon opening of the single package, plurality of packages or packettes, blister packet, lidded blister, blister card or packets or shrink wrap. For example, the beta adrenergic receptor antagonist, or a beta blocker or equivalent, or a propranolol or equivalent; the non-steroidal anti-inflammatory drug, or a NSAID or equivalent, or an etodolac or equivalent; and the therapeutic agent for the treatment of cancer can be packaged together in a single package, a plurality of packages or packettes, or a blister packet, lidded blister or blister card or packets, or a shrink wrap, and one, two, three, four or five or more or all of the drugs can be released upon opening of the single package, plurality of packages or packettes, blister packet, lidded blister, blister card or packets or shrink wrap.

In alternative embodiments of the therapeutic combination(s) of the invention, one, two, three, four or five or more or all of the drugs of the therapeutic combination are formulated (e.g., tableted) or manufactured as a parenteral formulation, an aqueous solution, a liposome, an injectable solution, a tablet, a pill, a lozenge, a capsule, a caplet, a patch, a spray, an inhalant, a powder, a freeze-dried powder, an inhalant, a patch, a gel, a geltab, a nanosuspension, a nanoparticle, a nanoliposome, a microgel, a pellet, a suppository or any combination thereof.

In alternative embodiments of the therapeutic combination(s) of the invention, one, two, three, four or five or more or all of the drugs of the therapeutic combination are formulated (e.g., tableted) or manufactured together in one parenteral formulation, one aqueous solution, one liposome, one injectable solution, one freeze-dried powder, one feed, one food, one food supplement, one pellet, one lozenge, one liquid, one elixir, one aerosol, one inhalant, one adhesive, one spray, one powder, one freeze-dried powder, one patch, one tablet, one pill, one capsule, one gel, one geltab, one lozenge, one caplet, one nanosuspension, one nanoparticle, one nanoliposome, one microgel or one suppository.

In alternative embodiments of the therapeutic combination(s) of the invention:
(a) the dosage of etodolac ranges from about 200 mg to 400 mg a day, or, about 10, 15, 20, 25, 30, 35, 40, 45, 50, 75, 80, 85, 90, 100, 150, 200, 250, 300, 350, 400, 450, 500, 600, 700, 800, 900 or 1000 mg or more; or
(b) the dosage of propranolol ranges from 10 to 320 mg per day based on heart rate and blood pressure of the individual, or, about 10, 15, 20, 25, 30, 35, 40, 45, 50, 75, 80, 85, 90, 100, 150, 200, 250, 300, 350, 400, 450, 500, 600, 700, 800, 900 or 1000 mg or more.

In alternative embodiments of the therapeutic combination(s) of the invention:
(a) the dosage of clarithromycin comprises or consists of a 250 mg immediate release formulation or tablet every 12 hours, a 500 mg immediate release formulation or tablet every 12 hours, or a 1000 mg extended release formulation or tablet once daily, or any combination thereof;
(b) the dosage of roxithromycin comprises or consists of a 300 mg formulation or tablet once a day, or a 150 mg formulation or tablet twice a day, or any combination thereof;
(c) the dosage of rapamycin comprises or consists of 2 mg/day to 20 mg/day, based on tolerability and liver and kidney side effects;
(d) the dosage of rapamycin of (c), wherein the dose is increased (doubled) every week; or
(e) the dosage of rapamycin of (d), wherein the dose is doubled every week.

In alternative embodiments of the therapeutic combination(s) of the invention, the drug combination is packaged in dosages that match a chrono-dosing regimen to match an optimal dose for the time of day, the desired effect, the patient, the patient's condition, and the like. For example, the beta adrenergic receptor antagonist or a beta blocker or equivalent, or a propranolol or equivalent; the non-steroidal anti-inflammatory drug or NSAID or equivalent, or etodolac or equivalent; and the therapeutic agent for the treatment of cancer, can be packaged in dosages that match a chrono-dosing regimen to match an optimal dose for the time of day, the desired effect, the patient, the patient's condition, and the like.

In alternative embodiments of the therapeutic combination(s) of the invention, the beta adrenergic receptor antagonist or beta blocker or equivalent or propranolol or equivalent; the non-steroidal anti-inflammatory drug or NSAID or equivalent or etodolac or equivalent; and the therapeutic agent for the treatment of cancer, are packaged in dosages that match a chrono-dosing regimen comprising:
(a) in the AM, 20 mg beta adrenergic receptor antagonist (a beta blocker), e.g., a propranolol or equivalent, 200mg NSAID, e.g., an etodolac or equivalent; in the afternoon, 10 mg beta blocker, 200 mg NSAID, e.g., an etodolac or equivalent; in the PM, 10 mg beta blocker, 400 mg NSAID;
(b) in the AM 40 mg beta adrenergic receptor antagonist (a beta blocker), e.g., a propranolol or equivalent, 200 mg NSAID, e.g., an etodolac or equivalent; in the afternoon 20 mg beta blocker, 200 mg NSAID; in the evening, 20 mg propranolol, 400 mg NSAID;
(c) in the AM 80 mg beta adrenergic receptor antagonist (a beta blocker), e.g., a propranolol or equivalent, 200 mg NSAID; in the afternoon 40 mg beta blocker, 200 mg NSAID, in the evening 40 mg, NSAID; or
(d) a dose escalation comprising a regimen of (a) to (b) to (c).

In alternative embodiments of the therapeutic combination(s) of the invention, the beta adrenergic receptor antagonist or beta blocker or equivalent or propranolol or equivalent; the non-steroidal anti-inflammatory drug or NSAID or equivalent or etodolac or equivalent; and the therapeutic agent for the treatment of cancer, are packaged in dosages that match a chrono-dosing regimen comprising:
Start: AM, 20 mg propranolol, 200mg etodolac; afternoon, 10 mg propranolol, 200 mg etodolac; PM 5 mg propranolol, 400 mg etodolac;
Dose Escalation 1: AM 40 mg propranolol, 200 mg etodolac; afternoon 20 mg propranolol, 200 mg etodolac; evening, 10 mg propranolol, 400 mg etodolac;
Dose escalation 2: AM 80 mg propranolol, 200 mg etodolac; afternoon 40 mg propranolol, 200 mg etodolac, evening 20 mg, etodolac.

In alternative embodiments of the therapeutic combination(s) of the invention, the therapeutic drug combination is formulated for administration once a day, b.i.d. (twice a day), t.i.d (three times a day), four times a day, five times a day, or hourly, or weekly, or biweekly, or monthly; or in any desired dosage for any desired administration regimen. For example, the beta adrenergic receptor antagonist (a beta blocker) or propranolol or equivalent; the non-steroidal anti-inflammatory drug or NSAID or etodolac or equivalent; and the therapeutic agent for the treatment of cancer, can be formulated for administration once a day, b.i.d. or t.i.d, or weekly, or biweekly, or monthly.

In alternative embodiments, the therapeutic combination(s) of drugs are formulated for administration intravenously, topically, orally, by inhalation, by infusion, by injection, by inhalation, intraperitoneally, intramuscularly, subcutaneously, intra-aurally, for intra-articular administration, for intra-mammary administration, for topical administration or for absorption through epithelial or mucocutaneous linings; or are formulated for administration using any route or combination of routes of administration.

In alternative embodiments, the therapeutic combination(s) of drugs further comprise instructions for use, e.g., in the treatment, amelioration and/or prevention of a cancer, a cachexia, a cancer cachexia, a mucositis, an oral mucositis, a digestive mucositis, an esophageal mucositis, an intestinal mucositis and/or an anorexia. In alternative embodiments, the therapeutic combination(s) of drugs of the invention are formulated for and/or are used for the treatment, amelioration and/or prevention of a cancer, a cachexia, a cancer cachexia, a mucositis, an oral mucositis, a digestive mucositis, an esophageal mucositis, an intestinal mucositis and/or an anorexia.

In alternative embodiments, the therapeutic combination(s) of drugs of the invention are formulated for and/or are used for the treatment, amelioration and/or prevention of a cachexia defined as at least two of the symptoms selected from the group consisting of: 1) a hyper-inflammatory state, 2) altered hormone levels and cytokine levels; 3) decreased heart rate variability; 4) weight loss, and 5) increased heart rate, wherein optionally the increased heart rate is having a sustained elevated heart rate of at least about 6 bpm. The cachexia can be defined by an individual having at least a sustained elevated heart rate of at least about 6 bpm and weight loss.

In one embodiment, the invention provides pharmaceutical compositions or formulations comprising a therapeutic combination of drugs of the invention; and the therapeutic combination can further comprise or further consist of a pharmaceutically acceptable excipient. In alternative embodiments the pharmaceutical composition(s) or formulation(s) are formulated or manufactured as a feed, a food, a food or feed concentrate, a pellet, a lozenge, a liquid, a lotion, an implant, a nanoparticle, an elixir, an aerosol, a spray, an aerosol, an inhalant, a powder, a tablet, a pill, a capsule, a gel, a geltab, a nanosuspension, a nanoparticle, a patch, a microgel and/or a suppository.

In alternative embodiments, the invention provides uses of a therapeutic combination of the invention in the manufacture of a medicament or pharmaceutical composition for treating, ameliorating or preventing a trauma, condition or disease comprising: a cancer or dysfunctional cell condition; any side effect from the treatment of cancer, chronic Systemic Inflammatory Response State (SIRS); chronic systemic inflammatory stress; burns, chronic obstructive pulmonary disease; congestive heart failure; chronic kidney disease; surgery; sepsis; ageing; acute respiratory distress syndrome; acute lung injury; infection; a CNS disorder or injury; anemia; immunosuppression; insulin resistance; anorexia; anxiety; sleep disturbances; weakness; fatigue; gastrointestinal distress; sleep disturbances; wake disturbances; pain; listlessness; shortness of breath; lethargy; depression; malaise; or, a combination thereof. In alternative embodiments, the trauma, condition or disease comprises a maladaptive nutritional state secondary to the SIRS, or the maladaptive nutritional state comprises cachexia, and optionally the cachexia comprises cachexia secondary to cancer. In alternative embodiments, the cancer or dysfunctional cell condition comprises (is) any metastatic or benign tumor, and the use is for treating (killing, eliminating, stopping the growth and/or metastasis of) cancer stem cells or cancer cells from: lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma or pituitary adenoma, and any combination thereof.

In alternative embodiments of the use(s) of the invention, wherein the cachexia is defined as at least two of the symptoms selected from the group consisting of: 1) a hyper-inflammatory state, 2) altered hormone levels and cytokine levels; 3) decreased heart rate variability; 4) weight loss, and 5) increased heart rate, wherein optionally the increased heart rate is having a sustained elevated heart rate of at least about 6 bpm; or, the cachexia is defined by an individual having at least a sustained elevated heart rate of at least about 6 bpm and weight loss.

In alternative embodiments of the use(s) of the invention, the CNS disorder comprises Parkinson's disease or Alzheimer's disease.

In alternative embodiments the invention provides methods for treating, preventing and/or ameliorating a trauma, condition or disease comprising a cancer or dysfunctional cell condition or a mucositis, any side effect from the treatment of a cancer or dysfunctional cell condition, chronic Systemic Inflammatory Response State (SIRS), chronic systemic inflammatory stress; burns, chronic obstructive pulmonary disease; congestive heart failure; chronic kidney disease; surgery; cancer; sepsis; ageing; acute respiratory distress syndrome; acute lung injury; infection; a CNS disorder or injury; anemia; immunosuppression; insulin resistance; anorexia; anxiety; sleep disturbances; weakness; fatigue; gastrointestinal distress; sleep disturbances; wake disturbances; pain; listlessness; shortness of breath; lethargy; depression; malaise; or, a combination thereof, the method comprising the steps of:
(a) providing a therapeutic combination of the invention, or the pharmaceutical composition or formulation of the invention; and,
(b) administering a therapeutically effective amount of the therapeutic combination or the pharmaceutical composition or formulation of step (a), thereby treating or ameliorating the side effect, trauma, condition or disease.

In alternative embodiments, the condition or disease comprises a maladaptive nutritional state secondary to the SIRS; or, the maladaptive nutritional state comprises cachexia or anorexia, or a cachexia secondary to cancer. In alternative embodiments the cancer or dysfunctional cell condition comprises (is) any metastatic or benign tumor, and the use is for treating (killing, eliminating, stopping the growth and/or metastasis of) cancer stem cells or cancer cells from: lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, a neoplasm of the central nervous system (CNS), primary CNS lymphoma, spinal axis tumors, brain stem glioma or pituitary adenoma, and any combination thereof.

In alternative embodiments the cachexia is defined as at least two of the symptoms selected from the group consisting of: 1) a hyper-inflammatory state, 2) altered hormone levels and cytokine levels; 3) decreased heart rate variability; 4) weight loss, and 5) increased heart rate, wherein optionally the increased heart rate is having a sustained elevated heart rate of at least about 6 bpm; or, the cachexia is defined by an individual having at least a sustained elevated heart rate of at least about 6 bpm and weight loss.

In alternative embodiments the mucositis is a mucositis caused by a chemotherapy and/or a radiation therapy; or an oral mucositis, a digestive mucositis, an esophageal mucositis or an intestinal mucositis.

In alternative embodiments the invention provides composition(s) or a product or products of manufacture comprising a therapeutic combination of the invention, and/or the pharmaceutical composition or formulation of the invention. The pharmaceutical composition or formulation of the invention can comprise (e.g., be manufactured as) a blister pack, clamshell or tray; and in one aspect the therapeutic combination is formulated for unit dosage administration to an individual in need thereof at the same time, and each unit dosage is contained within one blister in the blister pack, or compartment in the clamshell or tray. The pharmaceutical composition or formulation of the invention can comprise (e.g., be manufactured as) a caplet, a lozenge, a pill, capsule, tablet, tab, geltab or implant, wherein the therapeutic combination is formulated for unit dosage administration to an individual in need thereof at the same time.

The pharmaceutical composition or formulation of the invention can be manufactured in unit dosages, wherein in alternative embodiments each unit dosage is contained within one, two or three or more caplet(s), lozenge(s), pill(s), capsule(s), tablet(s), tab(s), geltab(s) or implant(s).

In alternative embodiments the invention provides a nanoparticle, microparticle, nanoliposome or liposome comprising a therapeutic combination of the invention, or the pharmaceutical composition or formulation of the invention.

In alternative embodiments the invention provides a kit or kits comprising a therapeutic combination of the invention, or the pharmaceutical composition or formulation of the invention. In alternative embodiments the kits comprise at least one blister pack, lidded blister or blister card or packets, or a shrink wrap, comprising the therapeutic combination or the pharmaceutical composition.

In alternative embodiments the invention provides a kit or kits for the treatment, amelioration or prevention of a mucositis, a cancer or any dysfunctional cell condition, a side effect from the treatment of a cancer or dysfunctional cell condition, a chronic Systemic Inflammatory Response State (SIRS) or a maladaptive nutritional state in a patient population, the kit comprising a therapeutic combination of the invention, or the pharmaceutical composition or formulation of the invention, and instructions for use of the therapeutic combination or pharmaceutical composition. In alternative embodiments of the kits the mucositis is a mucositis secondary to radiotherapy (radiation therapy) and/or chemotherapy, or the mucositis is an oral mucositis, a digestive mucositis, an esophageal mucositis, an intestinal mucositis. In alternative embodiments of the kits the maladaptive nutritional state comprises cachexia, and optionally the cachexia comprises cachexia secondary to cancer. In alternative embodiments the cachexia is defined:
(a) as having (being associated with) at least two of the symptoms selected from the group consisting of: 1) a hyper-inflammatory state, 2) altered hormone levels and/or cytokine levels; 3) decreased heart rate variability; 4) weight loss, and 5) sustained increased heart rate, wherein optionally the sustained increased heart rate is having a sustained elevated heart rate of at least about 6 bpm; or
(b) by an individual having at least a sustained elevated heart rate of at least about 6 bpm and weight loss.

In alternative embodiments, the cancer or dysfunctional cell condition comprises (is) any metastatic or benign tumor, and the intended use is for treating (killing, eliminating, stopping the growth and/or metastasis of), ameliorating and/or preventing the formation (generation) of cancer stem cells or cancer cells from: lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma or pituitary adenoma, and any combination thereof.

In alternative embodiments the invention provides products of manufacture comprising a blister package, a lidded blister or a blister card or packet, a clamshell, a tray or a shrink wrap comprising a therapeutic combination of the invention, or the pharmaceutical composition or formulation of the invention. In alternative embodiments the products of manufacture can comprise a blister package, a lidded blister or a blister card or packet, a clamshell, a tray or a shrink wrap comprising a therapeutic combination of the invention, or the pharmaceutical composition or formulation of the invention, wherein the therapeutic combination or pharmaceutical composition or formulation are manufactured and/or formulated for at least two, three, four or five or more dosage administrations; or the therapeutic combination or pharmaceutical composition or formulation are manufactured and/or formulated for once a day, or bid (twice a day), or tid (three times a day), or four times a day, administration.

In alternative embodiments of the products of manufacture of the invention the therapeutic combination or pharmaceutical composition or formulation are formulated (e.g., manufactured) as one dosage administration in the morning and one dosage administration in the evening; or are formulated as one dosage administration in the morning, one dosage mid-day and one dosage administration in the evening. In one aspect, the dosage schedule provides a relatively higher dose of one drug in the morning (the AM) than in the evening, and a relatively higher dose of another medication in the evening than in the morning.

In alternative embodiments, the products of manufacture or formulations of the invention comprise a therapeutic combination of the invention or the pharmaceutical composition or formulation of the invention, and a nutritional supplement, or food supplement or feed supplement.

In alternative embodiments the invention provides methods for treating, ameliorating or preventing mucositis, a cachexia and/or a chronic Systemic Inflammatory Response State (SIRS), wherein a therapeutic combination of the invention, or the pharmaceutical composition or formulation of the invention, is administered to an individual in need thereof; and in alternative embodiments the therapeutic combination or the pharmaceutical composition are formulated for multiple administrations, e.g., at least two administrations, one in the morning and one in the evening, wherein the dosage schedule provides a relatively higher dose of beta blocker in the morning (the AM) than in the evening, and a relatively higher dose of an anti-inflammatory medication in the evening than in the morning.

In alternative embodiments of the methods of the invention, the administration regimen comprises at least two dosages of beta adrenergic receptor antagonist (a beta blocker) drug and at least two dosages of non-steroidal anti-inflammatory drug (a NSAID), and in alternative embodiments further comprises administration of an anti-anxiety drug, a synthetic prostaglandin E₁ (PGE₁) analogue misoprostol; *N*-(4-hydroxyphenyl)acetamide (paracetamol or acetaminophen); 2-[4-(2-methylpropyl)phenyl]propanoic acid (ibuprofen); an anticonvulsant or antiseizure drug; an neuropathic pain analgesic; an opioid (opiate) painkiller (analgesic); an antibiotic; an antidepressant or antipsychotic; or, further comprising any combination thereof; and in alternative embodiments the drugs are organized or labeled in one or more blister packages, one or more lidded blisters or one or more blister cards or packets, one or more clamshells, one or more trays or one or more shrink wraps for usage by an individual for at least two or more administrations, e.g., one in the morning and one in the evening; and in one aspect the dosage schedule provides a relatively higher dose of beta blocker in the morning (the AM) than in the evening, and a relatively higher dose of an anti-anxiety and/or an anti-inflammatory medication in the evening than in the morning.

In alternative embodiments of the methods of the invention, the administration regimen comprises doses of propranolol given in 20 or 40 mg tablets immediate release on a bid basis, and in the first dose week the doses for propranolol are 20 mg in the morning and 20 mg at bedtime, and after 1 week the dosage is adjusted to 20 mg of the immediate release product in the morning and 60 mg of the extended release at bedtime, and optionally if after an additional week the subject shows no improvement or has not obtained a 20% reduction in heart rate, without decreasing heart rate below 60 bpm or blood pressure below 90/60, the dose is adjusted to 40 mg of the immediate release propranolol in the morning and 120 mg of the extended release propranolol at bedtime.

In alternative embodiments of the methods of the invention, the administration regimen comprises doses of etodolac are given in 200 mg capsules or 500 mg tablets on a bid basis, and doses for etodolac are started at 200 mg in the morning and at bedtime, and after 1 week the dosage are adjusted to 200 mg in the morning and 500 mg at bedtime.

In alternative embodiments of the invention provides a blister pack or a plurality of blister packettes, a blister package, a lidded blister or a blister card or packet, a clamshell, a tray or a shrink wrap, comprising a therapeutic combination of the invention, or the pharmaceutical composition or formulation of the invention. In alternative embodiments of the blister pack or a plurality of blister packettes, a blister package, a lidded blister or a blister card or packet, a clamshell, a tray or a shrink wrap, the therapeutic combination of drugs are arranged or clustered in the blister pack or a plurality of blister packettes: (a) in a chrono-dosing arrangement or pattern; or (b) individually.

In alternative embodiments of the invention provides a paper, plastic or cellophane package or a plurality of packettes comprising a therapeutic combination of the invention, or the pharmaceutical composition or formulation of the invention. In alternative embodiments the invention provides of the paper, plastic or cellophane package or a plurality of packettes the therapeutic combination of drugs are arranged or clustered in the paper, plastic or cellophane package or a plurality of packettes: (a) in a chrono-dosing arrangement or pattern; or (b) individually.

In alternative embodiments of the invention a drug combination of the invention is formulated, packaged or designed for drug regimen compliance of a cancer patient population, a pediatric or geriatric population, or a mentally compromised patient population.

In alternative embodiments of the invention drug combination(s) of the invention are formulated, packaged or designed for drug regimen compliance of a cancer patient population having mild or severe mental retardation, slow cognition, dementia, senility, Alzheimer's disease, traumatic brain injury, chemical brain damage, mental diseases (e.g., dissociative disorder, obsessive-compulsive disorder, delusional disorder, schizophrenia, mania, panic disorder, depression, dyslexia, any learning disability and the like) post-traumatic stress disorder, traumatic war neurosis, post-traumatic stress syndrome (PTSS), physical disability (e.g., blindness).

In alternative embodiments of the blister pack or a plurality of blister packettes, a blister package, a lidded blister or a blister card or packet, a clamshell, a tray or a shrink wrap of the invention, or the paper, plastic or cellophane package or a plurality of packettes of the invention, wherein the drug combination is formulated, packaged or designed for drug regimen compliance of a cancer patient population, a pediatric or geriatric population, or a mentally compromised patient population. In alternative embodiments of a blister pack or a plurality of blister packettes, a blister package, a lidded blister or a blister card or packet, a clamshell, a tray or a shrink wrap of the invention, or a paper, plastic or cellophane package or a plurality of packettes of the invention, the drug combination is formulated, packaged or designed for drug regimen compliance of a cancer patient population having mild or severe mental retardation, slow cognition, dementia, senility, Alzheimer's disease, traumatic brain injury, chemical brain damage, mental diseases (e.g., dissociative disorder, obsessive-compulsive disorder, delusional disorder, schizophrenia, mania, panic disorder, depression, dyslexia, any learning disability and the like) post-traumatic stress disorder, traumatic war neurosis, post-traumatic stress syndrome (PTSS), physical disability (e.g., blindness).

In alternative embodiments the invention provides a food or food supplement comprising the therapeutic combination of the invention, or the pharmaceutical composition or formulation of the invention. In alternative embodiments the invention provides feed or feed supplements comprising the therapeutic combination of the invention, or the pharmaceutical composition or formulation of the invention. In alternative embodiments the invention provides nutraceuticals comprising the therapeutic combination of the invention, or the pharmaceutical composition or formulation of the invention.

The invention provides compositions or product of manufactures comprising:
(a) a combination of compounds comprising or consisting of
   (i) at least two different compounds, each selected from a separate group selected from the group consisting of Group A, Group B, Group C, Group D, Group E, Group F, Group G, Group H, Group I, Group J, Group K, Group L and Group M, as set forth in Table 1, or,
   (ii) at least two different compounds, wherein at least two of the compounds are selected from the same group selected from the group consisting of Group A, Group B, Group C, Group D, Group E, Group F, Group G, Group H, Group I, Group J, Group K, Group L and Group M,
   wherein
   Group A comprises geranylgeranyl compounds (acyclic polyisoprenoids);
   Group B comprises Angiotensin Converting Enzyme Inhibitors (ACE Inhibitors or ACE-Is);
   Group C comprises Angiotensin Receptor Blockers (ARBs);
   Group D comprises Peroxisome Proliferator-Activated Receptor (PPAR) ligands;
   Group E comprises Non-Steroidal Anti-Inflammatory Drugs (NSAIDs);
   Group F comprises compositions for chemotherapy or radiation therapy (chemotherapy compositions include e.g., biologics such as proteins (e.g., peptides, antibodies, cytokines and the like) and small molecules such as alkylating agents);
   Group G comprises TNF inhibitors;
   Group H comprises deferrioxamine or deferoxamine;
   Group I comprises polyunsaturated fatty acids;
   Group J comprises eflornithine (α-difluoromethylornithine or DFMO);
   Group K comprises superoxide dismutases (SOD), catalases, superoxide dismutase (SOD)-catalase conjugates or complexes, peroxiredoxins and peroxidases;
   Group L comprises activators of a heat shock response;
   Group M comprises drugs that reduce an inflammatory response or a progressive tissue damage response;
(b) the composition or product of manufacture of (a) comprising or consisting of
   (i) at least three, four, five, six, seven, eight, nine or ten or more different compounds, each selected from a separate group selected from the group consisting of Group A, Group B, Group C, Group D, Group E, Group F, Group G, Group H, Group I, Group J, Group K, Group L and Group M, as set forth in Table 1, or,
   (ii) at least three, four, five, six, seven, eight, nine or ten or more different compounds, wherein at least three, four, five, six, seven, eight, nine or ten or more of the compounds are selected from the same group selected from the group consisting of Group A, Group B, Group C, Group D, Group E, Group F, Group G, Group H, Group I, Group J, Group K, Group L and Group M,
(c) the composition or product of manufacture of (a) or (b), wherein the combination of compounds comprise or consist of at least one, three, four, five, six, seven, eight, nine or ten or more compounds selected from at least one, three, four, five, six, seven, eight, nine, ten, eleven, twelve or all of the following groups: Group A, Group B, Group C, Group D, Group E, Group F, Group G, Group H, Group I, Group J, Group K, Group L, and/or Group M;
(d) the composition or product of manufacture of (c), wherein the combination of compounds comprises or consists of at least one compound from each of
   Group A and Group B, Group A and Group C, Group A and Group D, Group A and Group E, Group A and Group F, Group A and Group G, Group A and Group H, Group A and Group I, Group A and Group J, Group A and Group K, Group A and Group L, Group A and Group M, Group B and Group C,
   Group B and Group C, Group B and Group D, Group B and Group E, Group B and Group F, Group B and Group G, Group B and Group H, Group B and Group I, Group B and Group J, Group B and Group K, Group B and Group L, Group B and Group M,
   Group C and Group D, Group C and Group E, Group E and Group D; Group C and Group E, Group C and Group F, Group C and Group G, Group B and Group H, Group C and Group I, Group C and Group J, Group C and Group K, Group C and Group L, Group C and Group M,
   Group D and Group E, Group D and Group F, Group D and Group G, Group D and Group H, Group D and Group I, Group D and Group J, Group D and Group K, Group D and Group L, Group D and Group M,
   Group E and Group F, Group E and Group G, Group E and Group H, Group E and Group I, Group E and Group J, Group E and Group K, Group E and Group L, Group E and Group M,
   Group F and Group G, Group F and Group H, Group F and Group I, Group F and Group J, Group F and Group K, Group F and Group L, Group F and Group M,
   Group G and Group H, Group G and Group I, Group G and Group J, Group G and Group K, Group G and Group L, Group G and Group M,
   Group H and Group I, Group H and Group J, Group H and Group K, Group H and Group L, Group H and Group M,
   Group I and Group J, Group I and Group K, Group I and Group L, Group I and Group M,
   Group J and Group K, Group J and Group L, Group J and Group M, or
   Group K and Group L, Group L and Group M;
(e) the composition or product of manufacture of any of (a) to (d), wherein the combination of compounds comprises or consists of a geranylgeranyl acetone (GGA) or an analog of geranylgeranyl acetone and a non-steroidal anti-inflammatory drug (NSAID), or teprenone or SELBEX™ and a non-steroidal anti-inflammatory drug (NSAID);
(f) the composition or product of manufacture of (e), wherein the combination of compounds comprises or consists of (i) a GGA or an analog of GGA or a teprenone or a SELBEX™ and (ii) etodolac, aspirin, diclofenac, deflunisal, fenoprofen, flurbiprofen, ibuprofen, indomethacin, ketoprofen, ketorolac tromethamine, meclofenamate, mefenamic acid, meloxicam, nabumetone, naproxen, oxaprozin, piroxicam, salsalate, sulindac, tolmetin, a COX2-selective NSAID, celecoxib, rofecoxib, etoricoxib, valdecoxib, parecoxib, meloxicam or lumiracoxib;
(g) the composition or product of manufacture of (f), wherein the combination of compounds comprises or consists of a GGA or an analog of GGA or a teprenone or a SELBEX™ and: VOLTAREN™, CATAFLAM™, VOLTAREN-XR™, DOLOBID™, LODINE™, NALFON™, ANSAID™, FROBEN™, MOTRIN™, INDOCIN™, INDOCIN -SR™, ORUDIS™, ORUVAIL™, TORADOL™, MECLOMEN™, PONSTEL™, MOBICOX™, MOBIC™, RELIFEX™, RELAFEN™, ALEVE™, ANAPROX™, MIRANNAX™, NAPROGESIC™, NAPROSYN™, NAPRELAN™, SYNFLEX™, DAYPRO™ or DURAPROX™, FELDENE™, DISALCID™, SALFLEX™, CLINORIL™, TOLECTIN™, CELEBRA™, CELEBREX™, VIOXX™, CEOXX™, ARCOXIA™, BEXTRA™, DYNASTAT™, MOBIC™ or PREXIGE™;
(h) the composition or product of manufacture of any of (a) to (d), wherein the combination of compounds comprises or consists of a geranylgeranyl acetone (GGA) or an analog of geranylgeranyl acetone and an angiotensin converting enzyme (ACE) inhibitor, or teprenone or SELBEX™ and an angiotensin converting enzyme (ACE) inhibitor;
(i) the composition or product of manufacture of (h), wherein the combination of compounds comprises or consists of a geranylgeranyl acetone (GGA) or an analog of geranylgeranyl acetone or teprenone or SELBEX™ and: benazepril; captopril; cilazapril; enalapril; enalaprilat; fosinopril, fosinoprilat, imidapril (imidaprilum), lisinopril; moexipril; perindopril (coversyl); quinapril; ramipril; or, trandolapril;
(j) the composition or product of manufacture of (i), wherein the combination of compounds comprises or consists of a geranylgeranyl acetone (GGA) or an analog of geranylgeranyl acetone or teprenone or SELBEX™ and: LOTENSIN™, CAPOTIN™, RENITEC™, VASOTEC™, MONOPRIL™, UNIVASC™, PERDIX™, ACCUPRIL™, TRITACE™, RAMACE™, ALTACE™, or MAVIK™;
(k) the composition or product of manufacture of any of (a) to (d), wherein the combination of compounds comprises or consists of a geranylgeranyl acetone (GGA) or an analog of geranylgeranyl acetone and a pentoxifylline, or 1-(5-oxohexyl)-3, 7-dimethylxanthine, or TRENTAL™;
(l) the composition or product of manufacture of any of (a) to (d), wherein the combination of compounds comprises or consists of a geranylgeranyl acetone (GGA) or an analog of geranylgeranyl acetone and a desferrioxamine (deferoxamine), or DESFERAL™, DESFERAN™, DESFERAL™, DESFEREX™, DESFERIN™, or DESFERRIN™;
(m) the composition or product of manufacture of any of (a) to (d), wherein the combination of compounds comprises or consists of a geranylgeranyl acetone (GGA) or an analog of geranylgeranyl acetone and a polyunsaturated fatty acid;
(n) the composition or product of manufacture of (m), wherein the combination of compounds comprises or consists of a geranylgeranyl acetone (GGA) or an analog of geranylgeranyl acetone and a ω-3 fatty acid or omega-3 fatty acid, a ω-6 fatty acid or omega-6 fatty acid, or a as co-9 fatty acid or omega-9 fatty acid;
(o) the composition or product of manufacture of (m), wherein the combination of compounds comprises or consists of a geranylgeranyl acetone (GGA) or an analog of geranylgeranyl acetone and α-linolenic acid (ALA), eicosapentaenoic acid (EPA), docosahexaenoic acid (DHA), linoleic acid (LA), oleic acid and/or erucic acid;
(p) the composition of any of (a) to (d), wherein the combination of compounds comprises or consists of geranylgeranyl acetone (GGA) or an analog of geranylgeranyl acetone and an eflornithine, or α-difluoromethylornithine or DFMO, or ORNIDYL™;
(q) the composition or product of manufacture of any of (a) to (d), wherein the combination of compounds comprises or consists of geranylgeranyl acetone (GGA) or an analog of geranylgeranyl acetone and a superoxide dismutase (SOD);
(r) the composition or product of manufacture of any of (a) to (d), wherein the combination of compounds comprises or consists of geranylgeranyl acetone (GGA) or an analog of geranylgeranyl acetone and an activator of a heat shock response;
(s) the composition or product of manufacture of (r), wherein the combination of compounds comprises or consists of geranylgeranyl acetone (GGA) or an analog of geranylgeranyl acetone and geranylgeranylacetone or gernate, zinc, tin, salicylates, dexamethasone, cocaine, nicotine, alpha-adrenergic agonist, ppar-gamma agonist, a geldanamycin, biomolecular-geldanamycin, cyclopentanone, a prostanoid, a prostaglandin, a thromboxane, a prostacyclins, enprostil, paracetamol or acetaminophen (N-(4-hydroxyphenyl)-acetamide), ketotiphen, levamisole, diazepam, VALIUM™, bromocriptine, PARLODEL™, or dopamine (4-(2-aminoethyl)benzene-1,2-diol);
(t) the composition or product of manufacture of any of (a) to (s), wherein the combination of compounds comprises or consists of at least one compound selected from each of three groups selected from the group consisting of Group A, Group B, Group C, Group D, Group E, Group F, Group G, Group H, Group I, Group J, Group K, Group L and Group M;
(u) the composition or product of manufacture of (t), wherein the combination of compounds comprises or consists of at least one compound selected from Group A, Group B and Group C; Group A, Group B and Group D; Group A, Group B and Group E; Group A, Group D and Group E; Group B, Group C and Group D; Group B, Group C and Group E; Group C, Group E and Group F;
(v) the composition or product of manufacture of any of (a) to (u) formulated as a pharmaceutical composition;
(w) the composition or product of manufacture of (v) formulated for administration intravenously, topically, orally, by inhalation, by infusion, by injection, intraperitoneally, intramuscularly, subcutaneously, intra-aurally, by intra-articular administration, by intra-mammary administration, rectally, by topical administration or by absorption through epithelial and/or mucocutaneous linings;
(x) the composition or product of manufacture of (w) formulated as an aqueous suspension, a solid, a liquid, a powder, an emulsion, a lyophilized powder, a spray, a cream, a lotion, a controlled release formulation, a tablet, a pill, a gel, a liposome, on a patch, in an implant, on a tape, a dragee, a capsule, a lozenge, a gel, a syrup, a slurry and/or a suspension, or the composition is formulated with a solid excipient, a carbohydrate, a protein filler, a sugar, a lactose, a sucrose, a mannitol, a sorbitol, a starch, a cellulose, a methyl cellulose, a hydroxypropylmethyl-cellulose, a sodium carboxymethylcellulose, a gum, an arabic gum, a tragacanth, a gelatin, a collagen, a disintegrating or solubilizing agent, a cross-linked polyvinyl pyrrolidone, an agar, an alginic acid or alginic salt, or a sodium alginate;
(y) the composition or product of manufacture of any of (a) through (x), wherein the composition or product of manufacture comprises the combination of geranyl geranyl acetone (GGA) and captopril; GGA and CAPOTIN™; an analog of GGA and captopril; an analog of and CAPOTIN™; teprenone (CAS Registry Number 6809-52-5) and captopril; teprenone and CAPOTIN™; SELBEX™ and CAPOTIN™; or VT-211™; or
(z) the composition or product of manufacture of any of (a) through (x), wherein the composition or product of manufacture comprises the combination of geranyl geranyl acetone (GGA) and etodolac; GGA and LODINE™); an analog of GGA and etodolac; an analog of GGA and LODINE™; teprenone (CAS Registry Number 6809-52-5) and etodolac; teprenone and LODINE™; SELBEX™ and etodolac; SELBEX™ and LODINE™); or VT-212™.

One embodiment of the composition or product of manufacture comprises or consists of a blister pack, clamshell or tray, wherein the combination of drugs is formulated for unit dosage administration to an individual in need thereof at the same time, and each unit dosage is contained within one blister in the blister pack, or compartment in the clamshell or tray. The composition or product of manufacture can comprise or consist of a pill, capsule, tablet, tab, geltab or implant, wherein the combination of drugs is formulated for unit dosage administration to an individual in need thereof at the same time, and each unit dosage is contained within one pill, capsule, tablet, tab, geltab or implant.

The invention provides nanoparticles or microparticles comprising the composition or product of manufacture of this invention.

The invention provides uses of the composition or product of manufacture of this invention, for the manufacture of a medicament.

The invention provides uses of the composition or product of manufacture of this invention, for the manufacture of a medicament for to treat, ameliorate, diminish, improve and/or inhibit unwanted side effects and/or disease state symptoms associated with or caused by anger; anemia; anorexia; anorexia-cachexia; anxiety; atrophy or muscle atrophy; cachexia; cancer cachexia; cancer and any conditions caused by dysfunctional cells; cognitive impairment; cytoprotection deficiency; depression or despair; difficulties with activities of daily living; discomfort; emesis; erectile or sexual dysfunction or sexual disinterest; excessive sympathoneural drive; fatigue; febrile neutropenia; frustration; hair loss; heart failure; infection, bacterial infection, viral infection, mycobacterium infection, yeast infection, protozoan infection, inflammation; intolerance to a medical therapy; lack of appetite; lack of energy; lack of motivation; mucositis; oral mucositis; digestive mucositis; esophageal mucositis; intestinal mucositis; myeloprotection deficiency; neutropenia; rash; pain; reduced physical activity; wasting; worrying; pruritis; xerostomia; cardiotoxicity; ototoxicity; nephrotoxicity; peripheral neuropathy and/or stress or anxiety related to any of the above.

The invention provides methods (therapies) for treating or ameliorating a cancer or dysfunctional cell condition, including any metastatic or benign tumor, including cancer stem cells or cancer cells from lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma or pituitary adenoma, and any combination thereof.

The invention provides methods (therapies) for treating, ameliorating, diminishing, improving and/or inhibiting unwanted side effects and/or disease state symptoms associated with or caused by
(i) anger; anemia; anorexia; anorexia-cachexia; anxiety; atrophy or muscle atrophy; cachexia; cancer cachexia; cancer and any conditions caused by dysfunctional cells; cognitive impairment; cytoprotection deficiency; depression or despair; difficulties with activities of daily living; discomfort; emesis; erectile or sexual dysfunction or sexual disinterest; excessive sympathoneural drive; fatigue; febrile neutropenia; frustration; hair loss; heart failure; infection, bacterial infection, viral infection, mycobacterium infection, yeast infection, protozoan infection, inflammation; intolerance to a medical therapy; lack of appetite; lack of energy; lack of motivation; oral mucositis; digestive mucositis; esophageal mucositis; intestinal mucositis; myeloprotection deficiency; neutropenia; rash; pain; reduced physical activity; wasting; worrying; pruritis; xerostomia; cardiotoxicity; ototoxicity; nephrotoxicity; peripheral neuropathy and/or stress or anxiety related to any of the above,
(ii) mucositis or oral mucositis;
(iii) radiation therapy or chemotherapy; and/or
(iv) radiation therapy for cancer or cancer chemotherapy;
the method comprising
(a) administering to an individual in need thereof an effective amount of at least one composition or product of manufacture of this invention,
   thereby treating, ameliorating, diminishing, improving and/or inhibiting the unwanted side effects and/or disease state symptoms associated with or caused by
   (i) anger; anemia; anorexia; anorexia-cachexia; anxiety; atrophy or muscle atrophy; cachexia; cancer cachexia; cancer and any conditions caused by dysfunctional cells; cognitive impairment; cyto-protection deficiency; depression or despair; difficulties with activities of daily living; discomfort; emesis; erectile or sexual dysfunction or sexual disinterest; excessive sympathoneural drive; fatigue; febrile neutropenia; frustration; hair loss; heart failure; infection, bacterial infection, viral infection, mycobacterium infection, yeast infection, protozoan infection, inflammation; intolerance to a medical therapy; lack of appetite; lack of energy; lack of motivation; oral mucositis; digestive mucositis; esophageal mucositis; intestinal mucositis; myeloprotection deficiency; neutropenia; rash; pain; reduced physical activity; wasting; worrying; pruritis; xerostomia; cardiotoxicity; ototoxicity; nephrotoxicity; peripheral neuropathy and/or stress or anxiety related to any of the above,
   (ii) mucositis or oral mucositis;
   (iii) radiation therapy or chemotherapy; the administration can be before, during and/or after the radiation therapy and/or cancer chemotherapy; or
   (iv) radiation therapy for cancer or cancer chemotherapy; the administration can be before, during and/or after the radiation therapy and/or cancer chemotherapy;
(b) the method of (a), wherein the administered composition comprises a geranylgeranyl compound, a geranylgeranyl acetone (GGA) or an analog of geranylgeranyl acetone (GGA), at a dosage regimen of between about 0.10 mg to about 20.00 gm per day, or between about 30 mg to 3 gm per day; or
(c) the method of (a), wherein the administered composition comprises an angiotensin converting enzyme inhibitor at a dosage regimen of between about 0.10 mg to about 10.00 gm per day, or between about 10 mg to 450 mg per day.

The invention provides methods, treatments, therapies comprising administering to an individual in need thereof an effective amount of at least one composition or product of manufacture of this invention.

The invention provides methods, treatments, therapies comprising administering to an individual in need thereof an effective amount of at least one composition or product of manufacture of this invention, in conjunction (together) with a drug therapy, a chemotherapy or a radiation therapy. The administration can be before, during and/or after the radiation therapy and/or cancer chemotherapy.

The invention provides kits comprising (a) at least one composition or product of manufacture of this invention; or (b) the kit of (a), further comprising instructions to practice a method, treatment or therapy of this invention.

In alternative embodiments, this invention provides products and therapies that comprise use of compositions, e.g., preparations, formulations, kits and other products of manufacture (e.g., blister packs), comprising combinations of beneficial ingredients that provide cytoprotection to living cells and tissues, including tissue compartments and/or organs and/or organ systems; in different embodiments of this invention said cytoprotection may be measured at the cellular level and/or at a tissue compartment level and/or at the organ level and/or at the organ system level.

The invention provides compositions, e.g., pharmaceuticals comprising combinations of drugs, and methods comprising use of combinations of drugs, for treating, ameliorating, preventing or improving unwanted side effects (e.g., of a treatment, such as a radiotherapy or chemotherapy treatment; the composition can be administered before, during and/or after a radiation therapy and/or cancer chemotherapy), unwanted conditions, unwanted states and disease symptoms. In alternative embodiments, this invention provides products of manufacture, including preparations, products, multi-drug formulations, kits, multi-drug preparations, and other products of manufacture (e.g., multi-drug blister packs), and methods of using them, comprising use of combinations of beneficial ingredients for treating, preventing ameliorating, or improving any unwanted side effects (e.g., of a treatment, such as a radiotherapy or chemotherapy treatment; the administration can be before, during and/or after the radiation therapy and/or cancer chemotherapy), unwanted conditions, unwanted states and disease symptoms, as well as any combination of the unwanted side effects, conditions, states and disease symptoms e.g., as presented herein.

In alternative embodiments, unwanted side effects, conditions, states and disease symptoms treated, ameliorated, prevented or improved by compositions or methods of this invention include, e.g., 1/ anger; 2/ anemia; 3/ anorexia; 4/ anorexia-cachexia; 5/ anxiety; 6/ atrophy (e.g. muscle atrophy); 7/ cachexia, including cancer cachexia; 8/ cancer and any conditions caused by dysfunctional cells; 9/ cardiotoxicity; 10/ cognitive impairment; 11/ cytoprotection deficiency; 12/ depression; 13/ despair; 14/ delayed emesis; 15/ diarrhea; 16/ difficulties with activities of daily living; 17/ discomfort; 18/ emesis; 19/ erectile or sexual dysfunction or sexual disinterest; 20/ excessive sympathoneural drive; 21/ fatigue; 22/ febrile neutropenia; 23/ frustration; 24/ hair loss; 25/ heart failure; 26/ infection (in different aspects, infection types provided herein include bacterial, viral, mycobacterium, yeast and protozoan infections, and any combination thereof); 27/ inflammation; 28/ intolerance to a medical therapy; 29/ lack of appetite; 30/ lack of energy; 31/ lack or motivation; 32/ mucositis (e.g., oral, digestive, esophageal or intestinal mucositis); 33/ myeloprotection deficiency; 34/ myelosupression (including neutropenia); 35/ nausea; 36/ nephrotoxicity; 37/ neutropenia; 38/ oral mucositis; 39/ ototoxicity; 40/ pain; 41/ peripheral neuropathy; 42/ reduced physical activity; 43/ toxicity (including cyto-toxicity) from any chemotherapy or radiation or surgical trauma (the administration can be before, during and/or after the surgical trauma, radiation therapy and/or cancer chemotherapy); 44/ wasting; 45/ worrying; 46/ stress or anxiety related to any of the above.

The invention provides compositions, including products of manufacture, preparations, , e.g., formulations, kits, preparations, and other products of manufacture such as blister packs, and methods of using them, comprising use of combinations of beneficial ingredients for obtaining therapeutic benefits including for example: 1) to allow or enable or facilitate the tolerance and use of higher amounts and/or doses and/or longer durations of a medical therapy (e.g. cancer therapies or radiation therapies) than could be used without this invention; 2) to allow or enable or facilitate the tolerance and use of longer durations of a medical therapy (e.g. cancer therapies or radiation therapies) than could be used without this invention; 3) to reduce occurrences of (drug) resistance to a medical therapy (e.g. cancer chemotherapy or radiation therapy; the administration can be before, during and/or after the radiation therapy and/or cancer chemotherapy); 4) to enable dosage intensification of a medical therapy (e.g. cancer chemotherapy or radiation therapy); 5) to enable increasing the frequency of a medical therapy (e.g. cancer chemotherapy or radiation therapy); 6) to enhance patient response rates; 7) to increase patient survival; 8) to induce a tissue protective state; 9) to induce tissue regeneration; and 10) to induce tissue repair.

The invention provides compositions and methods for treating, preventing or improving unwanted side effects (e.g., of a treatment, such as a radiotherapy or chemotherapy treatment), unwanted conditions, unwanted states and disease symptoms arising from cancer or non-cancer medical needs, including non-cancer related indications and problems related to: 1/ Host versus graft post bone marrow transplantation (including pathobiology and treatment problems); 2/ Cyto-toxicity and GI damage secondary to surgical trauma; and 3/ acute lung and GI injury secondary to avian influenza, other infections and traumas (including from biowarfare agents).

The invention provides products of manufacture such as packages, kits, containers, blister packages, clamshells, trays, shrink wraps and the like, comprising at least two different pharmaceutically active ingredients, where each ingredient is manufactured in a separate pill, tablet, capsule, package, kit or container; or, all or a subset of the combinations of ingredients are manufactured in a separate package or container. In alternative aspects, the package, kit or container comprises a blister package, a clamshell, a tray, a shrink wrap and the like, comprising separate pills, tablets, capsules, packages, kits or containers for each of the at least two different pharmaceutically active ingredients.

In one embodiment, practicing the invention allows use of higher amounts and/or doses and/or longer durations of a medical therapy (e.g., a drug therapy, a cancer therapy or a radiation therapy) than the amounts and and/or doses or durations of a medical or drug therapy that could be tolerated and used without the benefit of this invention. Accordingly, this invention provides compositions and methods that can raise the upper range of the therapeutic window of a medical or a drug treatment.

The invention provides compositions, products and therapies; and in separate aspects, such a composition or product or therapy may comprise or consist of one or more members selected from any of Group A, Group B, Group C, Group D, Group E, Group F, Group G, Group H, Group I, Group J, Group K, Group L, and Group M, as shown in Table 1, where: of Group A comprises or consists of use of geranylgeranyl compounds, including for example, geranylgeranyl acetone (GGA) and analogs of geranylgeranyl acetone (GGA); Group B comprises or consists of use of Angiotensin Converting Enzyme Inhibitors (ACE Inhibitors or ACE-Is); Group C comprises or consists of use of Angiotensin Receptor Blockers (ARBs); Group D comprises or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises or consists of use of non-steroidal anti-inflammatory drugs (NSAIDs); Group F comprises or consists of NCCN therapies; Group G comprises use of or consists of use of TNF inhibitors, e.g. pentoxifylline, or 1-(5-oxohexyl)-3, 7-dimethylxanthine (e.g., TRENTAL™); Group H comprises or consists of use of deferrioxamine; Group I comprises or consists of polyunsaturated fatty acids, such as e.g. omega-3 fatty acids, including for example eicosapentaenoic acid (EPA), and docosahexaenoic acid (DHA); Group J comprises or consists of α-difluoromethylornithine; Group K comprises or consists of superoxide dismutase (SOD) and similar antioxidant compounds; Group L comprises or consists of activators of heat shock response; and Group M comprises or consists of drugs that reduce the inflammatory/progressive tissue damage response.

The invention provides compositions comprising or consisting of any combination of at least two different drugs or therapeutic combinations, e.g., at least two members as set forth in Table 1, e.g., from two to about one hundred members as set forth in Table 1. In separate embodiments, this invention provides a therapy (e.g. a product or a "method for treating") comprising or consisting of all combinations of members selected from any of Group A, Group B, Group C, Group D, Group E, Group F, Group G, Group H, Group I, Group J, Group K, Group L, and/or Group M, as set forth in Table 1.

In alternative embodiments, this invention provides therapies (including compositions and methods) comprising or consisting of every possible combination and permutation of one member, two members, 3 members, 4 members, five members, etc., and up to and including at least 100 (one hundred) members selected from any of Group A, Group B, Group C, Group D, Group E, Group F, Group G, Group H, Group I, Group J, Group K, Group L, and Group M, as shown in Table 1, e.g., where: of Group A comprises or consists of, or Group A comprises use of or consists of use of, geranylgeranyl compounds, including for example, geranylgeranyl acetone (GGA) and analogs of geranylgeranyl acetone (GGA); Group B comprises use of or consists of use of Angiotensin Converting Enzyme Inhibitors (ACE Inhibitors or ACE-Is); Group C comprises use of or consists of use of Angiotensin Receptor Blockers (ARBs); Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises use of or consists of use of non-steroidal anti-inflammatory drugs (NSAIDs); Group F consists of NCCN therapies; Group G comprises use of or consists of use of TNF inhibitors, e.g. pentoxifylline, or 1-(5-oxohexyl)-3, 7-dimethylxanthine (e.g., TRENTAL™); Group H comprises use of or consists of use of deferrioxamine; Group I comprises polyunsaturated fatty acids, such as e.g. omega-3 fatty acids, including for example eicosapentaenoic acid (EPA), and docosahexaenoic acid (DHA); Group J comprises α-difluoromethylornithine; Group K comprises superoxide dismutase (SOD) and similar antioxidant compounds; Group L comprises activators of heat shock response; and Group M comprises drugs that reduce the inflammatory/progressive tissue damage response.

In separate embodiments, this invention provides a therapy (including compositions and methods) comprising or consisting of at least two different members from only one of Group A, Group B, Group C, Group D, Group E, Group F, Group G, Group H, Group I, Group J, Group K, Group L, and Group M, as shown in Table 1 (i.e., at least two different members from a single group), where: of Group A comprises or consists of, or Group A comprises or consists of use of, geranylgeranyl compounds, including for example, geranylgeranyl acetone (GGA) and analogs of geranylgeranyl acetone (GGA); Group B comprises or consists of use of Angiotensin Converting Enzyme Inhibitors (ACE Inhibitors or ACE-Is); Group C comprises or consists of use of Angiotensin Receptor Blockers (ARBs); Group D comprises or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises or consists of use of non-steroidal anti-inflammatory drugs (NSAIDs); Group F comprises or consists of NCCN therapies; Group G comprises or consists of use of TNF inhibitors, e.g. pentoxifylline, or 1-(5-oxohexyl)-3, 7-dimethylxanthine (e.g., TRENTAL™); Group H comprises or consists of use of deferrioxamine; Group I comprises or consists of polyunsaturated fatty acids, such as e.g. omega-3 fatty acids, including for example eicosapentaenoic acid (EPA), and docosahexaenoic acid (DHA); Group J comprises or consists of α-difluoromethylornithine; Group K comprises or consists of superoxide dismutase (SOD) and similar antioxidant compounds; Group L comprises or consists of activators of heat shock response; and Group M comprises or consists of drugs that reduce the inflammatory/progressive tissue damage response.

In alternative embodiments, this invention provides a therapy (including compositions and methods) (that may be called a "double or 2-member combination therapy") comprising or consisting of all combinations of at least two members selected from any of Group A, Group B, Group C, Group D, Group E, Group F, Group G, Group H, Group I, Group J, Group K, Group L, and Group M, as shown in Table 1, where: of Group A comprises or consists of, or Group A comprises or consists of use of, geranylgeranyl compounds, including for example, geranylgeranyl acetone (GGA) and analogs of geranylgeranyl acetone (GGA); Group B comprises or consists of use of Angiotensin Converting Enzyme Inhibitors (ACE Inhibitors or ACE-Is); Group C comprises or consists of use of Angiotensin Receptor Blockers (ARBs); Group D comprises or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises or consists of use of non-steroidal anti-inflammatory drugs (NSAIDs); Group F comprises or consists of NCCN therapies; Group G comprises or consists of use of TNF inhibitors, e.g. pentoxifylline, or 1-(5-oxohexyl)-3, 7-dimethylxanthine (e.g., TRENTAL™); Group H comprises or consists of use of deferrioxamine; Group I comprises or consists of polyunsaturated fatty acids, such as e.g. omega-3 fatty acids, including for example eicosapentaenoic acid (EPA), and docosahexaenoic acid (DHA); Group J comprises or consists of α-difluoromethylornithine; Group K comprises or consists of superoxide dismutase (SOD) and similar antioxidant compounds; Group L comprises or consists of activators of heat shock response; and Group M comprises or consists of drugs that reduce the inflammatory/progressive tissue damage response, where said members selected to comprise the "double combination therapy" may all be selected from the same Group (e.g. Group A) or may be selected from multiple Groups (e.g. Group A and Group B).

In alternative embodiments, this invention also provides a therapy (including compositions and methods) (that may be called a "triple or 3-member combination therapy") comprising or consisting of all combinations of at least three members selected from any of Group A, Group B, Group C, Group D, Group E, Group F, Group G, Group H, Group I, Group J, Group K, Group L, and Group M, as shown in Table 1, where: of Group A comprises or consists of, or Group A comprises or consists of use of, geranylgeranyl compounds, including for example, geranylgeranyl acetone (GGA) and analogs of geranylgeranyl acetone (GGA); Group B comprises or consists of use of Angiotensin Converting Enzyme Inhibitors (ACE Inhibitors or ACE-Is); Group C comprises or consists of use of Angiotensin Receptor Blockers (ARBs); Group D comprises or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises or consists of use of non-steroidal anti-inflammatory drugs (NSAIDs); Group F consists of NCCN therapies; Group G comprises or consists of use of TNF inhibitors, e.g. pentoxifylline, or 1-(5-oxohexyl)-3, 7-dimethylxanthine (e.g., TRENTAL™); Group H comprises or consists of use of deferrioxamine; Group I comprises or consists of polyunsaturated fatty acids, such as e.g. omega-3 fatty acids, including for example eicosapentaenoic acid (EPA), and docosahexaenoic acid (DHA); Group J comprises or consists of α-difluoromethylornithine; Group K comprises or consists of superoxide dismutase (SOD) and similar antioxidant compounds; Group L comprises or consists of activators of heat shock response; and Group M comprises or consists of drugs that reduce the inflammatory/progressive tissue damage response, where said members selected to comprise the "triple combination therapy" may all be selected from the same Group (e.g. Group A) or may be selected from multiple Groups (e.g. Group A, Group B, and Group E).

In alternative embodiments, this invention also provides a therapy (including compositions and methods) (that may be called a "quadruple or 4-member combination therapy") comprising or consisting of all combinations of at least four members selected from any of Group A, Group B, Group C, Group D, Group E, Group F, Group G, Group H, Group I, Group J, Group K, Group L, and Group M, as shown in Table 1, where: of Group A comprises or consists of, or Group A comprises or consists of use of, geranylgeranyl compounds, including for example, geranylgeranyl acetone (GGA) and analogs of geranylgeranyl acetone (GGA); Group B comprises or consists of use of Angiotensin Converting Enzyme Inhibitors (ACE Inhibitors or ACE-Is); Group C comprises or consists of use of Angiotensin Receptor Blockers (ARBs); Group D comprises or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises or consists of use of non-steroidal anti-inflammatory drugs (NSAIDs); Group F comprises or consists of use of NCCN therapies; Group G comprises or consists of use of TNF inhibitors, e.g. pentoxifylline, or 1-(5-oxohexyl)-3, 7-dimethylxanthine (e.g., TRENTAL™); Group H comprises or consists of use of deferrioxamine; Group I comprises or consists of polyunsaturated fatty acids, such as e.g. omega-3 fatty acids, including for example eicosapentaenoic acid (EPA), and docosahexaenoic acid (DHA); Group J comprises or consists of α-difluoromethylornithine; Group K comprises or consists of superoxide dismutase (SOD) and similar antioxidant compounds; Group L comprises or consists of activators of heat shock response; and Group M comprises or consists of drugs that reduce the inflammatory/ progressive tissue damage response, where said members selected to comprise the "quadruple combination therapy" may all be selected from the same Group (e.g. Group A) or may be selected from multiple Groups (e.g. Group A, Group B, Group E, and Group F).

In alternative embodiments, this invention also provides a therapy (including compositions and methods) (that may be called a "quintuple or pentuple or 5-member combination therapy") comprising or consisting of all combinations of at least five members selected from any of Group A, Group B, Group C, Group D, Group E, Group F, Group G, Group H, Group I, Group J, Group K, Group L, and Group M, as shown in Table 1, where: of Group A comprises or consists of, or Group A comprises or consists of use of, geranylgeranyl compounds, including for example, geranylgeranyl acetone (GGA) and analogs of geranylgeranyl acetone (GGA); Group B comprises or consists of use of Angiotensin Converting Enzyme Inhibitors (ACE Inhibitors or ACE-Is); Group C comprises or consists of use of Angiotensin Receptor Blockers (ARBs); Group D comprises or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises or consists of use of non-steroidal anti-inflammatory drugs (NSAIDs); Group F consists of NCCN therapies; Group G comprises or consists of use of TNF inhibitors, e.g. pentoxifylline, or 1-(5-oxohexyl)-3, 7-dimethylxanthine (e.g., TRENTAL™); Group H comprises or consists of use of deferrioxamine; Group I comprises or consists of polyunsaturated fatty acids, such as e.g. omega-3 fatty acids, including for example eicosapentaenoic acid (EPA), and docosahexaenoic acid (DHA); Group J comprises or consists of α-difluoromethylornithine; Group K comprises or consists of superoxide dismutase (SOD) and similar antioxidant compounds; Group L comprises or consists of activators of heat shock response; and Group M comprises or consists of drugs that reduce the inflammatory/progressive tissue damage response, where said members selected to comprise the "quintuple combination therapy" may all be selected from the same Group (e.g. Group A) or may be selected from multiple Groups (e.g. Group A, Group B, Group E, and Group F).

In alternative embodiments, this invention also provides a therapy (including compositions and methods) (that may be called a "sextuple or hextuple or 6-member combination therapy") comprising all combinations of at least six members selected from any of Group A, Group B, Group C, Group D, Group E, Group F, Group G, Group H, Group I, Group J, Group K, Group L, and Group M, as shown in Table 1, where: of Group A comprises or consists of, or Group A comprises or consists of use of, geranylgeranyl compounds, including for example, geranylgeranyl acetone (GGA) and analogs of geranylgeranyl acetone (GGA); Group B comprises or consists of use of Angiotensin Converting Enzyme Inhibitors (ACE Inhibitors or ACE-Is); Group C comprises or consists of use of Angiotensin Receptor Blockers (ARBs); Group D comprises or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises or consists of use of non-steroidal anti-inflammatory drugs (NSAIDs); Group F comprises or consists of NCCN therapies; Group G comprises or consists of use of TNF inhibitors, e.g. pentoxifylline, or 1-(5-oxohexyl)-3, 7-dimethylxanthine (e.g., TRENTAL™); Group H comprises or consists of use of deferrioxamine; Group I comprises or consists of polyunsaturated fatty acids, such as e.g. omega-3 fatty acids, including for example eicosapentaenoic acid (EPA), and docosahexaenoic acid (DHA); Group J comprises or consists of α-difluoromethylornithine; Group K comprises or consists of superoxide dismutase (SOD) and similar antioxidant compounds; Group L comprises or consists of activators of heat shock response; and Group M comprises or consists of drugs that reduce the inflammatory/progressive tissue damage response, where said members selected to comprise the "sextuple combination therapy" may all be selected from the same Group (e.g. Group A) or may be selected from multiple Groups (e.g. Group A, Group B, Group E, and Group F).

In alternative embodiments, this invention also provides a therapy (including compositions and methods) (that may be called a "septuple or 7-member combination therapy") comprising or consisting of all combinations of at least seven members selected from any of Group A, Group B, Group C, Group D, Group E, Group F, Group G, Group H, Group I, Group J, Group K, Group L, and/or Group M, as shown in Table 1, where: of Group A comprises or consists of, or Group A comprises or consists of use of, geranylgeranyl compounds, including for example, geranylgeranyl acetone (GGA) and analogs of geranylgeranyl acetone (GGA); Group B comprises or consists of use of Angiotensin Converting Enzyme Inhibitors (ACE Inhibitors or ACE-Is); Group C comprises or consists of use of Angiotensin Receptor Blockers (ARBs); Group D comprises or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises or consists of use of non-steroidal anti-inflammatory drugs (NSAIDs); Group F comprises or consists of NCCN therapies; Group G comprises or consists of use of TNF inhibitors, e.g. pentoxifylline, or 1-(5-oxohexyl)-3, 7-dimethylxanthine (e.g., TRENTAL™); Group H comprises or consists of use of deferrioxamine; Group I comprises or consists of polyunsaturated fatty acids, such as e.g. omega-3 fatty acids, including for example eicosapentaenoic acid (EPA), and docosahexaenoic acid (DHA); Group J comprises or consists of α-difluoromethylornithine; Group K comprises or consists of superoxide dismutase (SOD) and similar antioxidant compounds; Group L comprises or consists of activators of heat shock response; and Group M comprises or consists of drugs that reduce the inflammatory/progressive tissue damage response, where said members selected to comprise the "septuple combination therapy" may all be selected from the same Group (e.g. Group A) or may be selected from multiple Groups (e.g. Group A, Group B, Group E, and Group F).

In alternative embodiments, this invention also provides a therapy (including compositions and methods) (that may be called an "octuple or 8-member combination therapy") comprising or consisting of all combinations of at least eight members selected from any of Group A, Group B, Group C, Group D, Group E, Group F, Group G, Group H, Group I, Group J, Group K, Group L, and/or Group M, as shown in Table 1, where: of Group A comprises or consists of, or Group A comprises or consists of use of, geranylgeranyl compounds, including for example, geranylgeranyl acetone (GGA) and analogs of geranylgeranyl acetone (GGA); Group B comprises or consists of use of Angiotensin Converting Enzyme Inhibitors (ACE Inhibitors or ACE-Is); Group C comprises or consists of use of Angiotensin Receptor Blockers (ARBs); Group D comprises or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises or consists of use of non-steroidal anti-inflammatory drugs (NSAIDs); Group F comprises or consists of NCCN therapies; Group G comprises or consists of use of TNF inhibitors, e.g. pentoxifylline, or 1-(5-oxohexyl)-3, 7-dimethylxanthine (e.g., TRENTAL™); Group H comprises or consists of use of deferrioxamine; Group I comprises or consists of polyunsaturated fatty acids, such as e.g. omega-3 fatty acids, including for example eicosapentaenoic acid (EPA), and docosahexaenoic acid (DHA); Group J comprises or consists of α-difluoromethylornithine; Group K comprise or consists of s superoxide dismutase (SOD) and similar antioxidant compounds; Group L comprises or consists of activators of heat shock response; and Group M comprises or consists of drugs that reduce the inflammatory/progressive tissue damage response, where said members selected to comprise the "octuple combination therapy" may all be selected from the same Group (e.g. Group A) or may be selected from multiple Groups (e.g. Group A, Group B, Group E, and Group F).

In alternative embodiments, this invention also provides a therapy (including compositions and methods) (that may be called a "nonuple or 9-member combination therapy") comprising or consisting of all combinations of at least nine members selected from any of Group A, Group B, Group C, Group D, Group E, Group F, Group G, Group H, Group I, Group J, Group K, Group L, and/or Group M, as shown in Table 1, where: of Group A comprises or consists of, or Group A comprises or consists of use of, geranylgeranyl compounds, including for example, geranylgeranyl acetone (GGA) and analogs of geranylgeranyl acetone (GGA); Group B comprises or consists of use of Angiotensin Converting Enzyme Inhibitors (ACE Inhibitors or ACE-Is); Group C comprises or consists of use of Angiotensin Receptor Blockers (ARBs); Group D comprises or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises or consists of use of non-steroidal anti-inflammatory drugs (NSAIDs); Group F comprises or consists of NCCN therapies; Group G comprises or consists of use of TNF inhibitors, e.g. pentoxifylline, or 1-(5-oxohexyl)-3, 7-dimethylxanthine (e.g., TRENTAL™); Group H comprises or consists of use of deferrioxamine; Group I comprises or consists of polyunsaturated fatty acids, such as e.g. omega-3 fatty acids, including for example eicosapentaenoic acid (EPA), and docosahexaenoic acid (DHA); Group J comprises or consists of α-difluoromethylornithine; Group K comprises or consists of superoxide dismutase (SOD) and similar antioxidant compounds; Group L comprises or consists of activators of heat shock response; and Group M comprises or consists of drugs that reduce the inflammatory/progressive tissue damage response, where said members selected to comprise the "nonuple combination therapy" may all be selected from the same Group (e.g. Group A) or may be selected from multiple Groups (e.g. Group A, Group B, Group E, and Group F).

In alternative embodiments, this invention also provides a therapy (including compositions and methods) (that may be called a "decuple or 10-member combination therapy") comprising or consisting of all combinations of at least ten members selected from any of Group A, Group B, Group C, Group D, Group E, Group F, Group G, Group H, Group I, Group J, Group K, Group L, and/or Group M, as shown in Table 1, where: of Group A comprises or consists of, or Group A comprises or consists of use of, geranylgeranyl compounds, including for example, geranylgeranyl acetone (GGA) and analogs of geranylgeranyl acetone (GGA); Group B comprises or consists of use of Angiotensin Converting Enzyme Inhibitors (ACE Inhibitors or ACE-Is); Group C comprises or consists of use of Angiotensin Receptor Blockers (ARBs); Group D comprises or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises or consists of use of non-steroidal anti-inflammatory drugs (NSAIDs); Group F comprises or consists of NCCN therapies; Group G comprises or consists of use of TNF inhibitors, e.g. pentoxifylline, or 1-(5-oxohexyl)-3, 7-dimethylxanthine (e.g., TRENTAL™); Group H comprises or consists of use of deferrioxamine; Group I comprises or consists of polyunsaturated fatty acids, such as e.g. omega-3 fatty acids, including for example eicosapentaenoic acid (EPA), and docosahexaenoic acid (DHA); Group J comprises or consists of α-difluoromethylornithine; Group K comprise or consists of s superoxide dismutase (SOD) and similar antioxidant compounds; Group L comprises or consists of activators of heat shock response; and Group M comprises or consists of drugs that reduce the inflammatory/progressive tissue damage response, where said members selected to comprise the "decuple combination therapy" may all be selected from the same Group (e.g. Group A) or may be selected from multiple Groups (e.g. Group A, Group B, Group E, and Group F).

### "Double Combinations Therapies" (All "Combination Possibilities")

The invention provides compositions and therapies (methods) comprising or consisting of use of any combination of at least one compound from at least two Groups (from Table 1), and below are 78 non-limiting exemplary combinations of this invention.
Example 2-1. Any combination comprising or consisting of One Member of Group A and One Member of Group B (see Table 1). In alternative aspects, a therapy provided herein comprises or consists of or consists of at least one member selected from Group A, and at least one member selected from Group B, as shown in Table 1, where: of Group A comprises or consists of, or Group A comprises or consists of use of, geranylgeranyl compounds, including for example, geranylgeranyl acetone (GGA) and analogs of geranylgeranyl acetone (GGA); and Group B comprises or consists of use of Angiotensin Converting Enzyme Inhibitors (ACE Inhibitors or ACE-Is).
Example 2-2. Any combination comprising or consisting of One Member of Group A and One Member of Group C (see Table 1). In alternative aspects, a therapy provided herein comprises or consists of at least one member selected from Group A, and at least one member selected from Group C, as shown in Table 1, where: of Group A comprises or consists of, or Group A comprises or consists use of, geranylgeranyl compounds, including for example, geranylgeranyl acetone (GGA) and analogs of geranylgeranyl acetone (GGA); and Group C comprises or consists use of Angiotensin Receptor Blockers (ARBs).
Example 2-3. Any combination comprising or consisting of One Member of Group A and One Member of Group D (see Table 1). In alternative aspects, a therapy provided herein comprises or consists at least one member selected from Group A, and at least one member selected from Group D, as shown in Table 1, where: of Group A comprises or consists of, or Group A comprises or consists use of, geranylgeranyl compounds, including for example, geranylgeranyl acetone (GGA) and analogs of geranylgeranyl acetone (GGA); and Group D comprises or consists use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands).
Example 2-4. Any combination comprising or consisting of One Member of Group A and One Member of Group E (see Table 1). In alternative aspects, a therapy provided herein comprises or consists at least one member selected from Group A, and at least one member selected from Group E, as shown in Table 1, where: of Group A comprises or consists of, or Group A comprises or consists use of, geranylgeranyl compounds, including for example, geranylgeranyl acetone (GGA) and analogs of geranylgeranyl acetone (GGA); and Group E comprises or consists use of non-steroidal anti-inflammatory drugs (NSAIDs).
Example 2-5. Any combination comprising or consisting of One Member of Group A and One Member of Group F (see Table 1). In alternative aspects, a therapy provided herein comprises or consists of at least one member selected from Group A, and at least one member selected from Group F, as shown in Table 1, where: of Group A comprises or consists of, or Group A comprises use of or consists of use of, geranylgeranyl compounds, including for example, geranylgeranyl acetone (GGA) and analogs of geranylgeranyl acetone (GGA); and Group F consists of NCCN therapies.
Example 2-6. Any combination comprising or consisting of One Member of Group A and One Member of Group G (see Table 1). In alternative aspects, a therapy provided herein comprises or consists of at least one member selected from Group A, and at least one member selected from Group G, as shown in Table 1, where: of Group A comprises or consists of, or Group A comprises use of or consists of use of, geranylgeranyl compounds, including for example, geranylgeranyl acetone (GGA) and analogs of geranylgeranyl acetone (GGA); and Group G comprises use of or consists of use of TNF inhibitors, e.g. pentoxifylline, or 1-(5-oxohexyl)-3, 7-dimethylxanthine (e.g., TRENTAL™).
Example 2-7. Any combination comprising or consisting of One Member of Group A and One Member of Group H (see Table 1). In alternative aspects, a therapy provided herein comprises or consists of at least one member selected from Group A, and at least one member selected from Group H, as shown in Table 1, where: of Group A comprises or consists of, or Group A comprises use of or consists of use of, geranylgeranyl compounds, including for example, geranylgeranyl acetone (GGA) and analogs of geranylgeranyl acetone (GGA); and Group H comprises use of or consists of use of deferrioxamine.
Example 2-8. Any combination comprising or consisting of One Member of Group A and One Member of Group I (see Table 1). In alternative aspects, a therapy provided herein comprises or consists of at least one member selected from Group A, and at least one member selected from Group I, as shown in Table 1, where: of Group A comprises or consists of, or Group A comprises use of or consists of use of, geranylgeranyl compounds, including for example, geranylgeranyl acetone (GGA) and analogs of geranylgeranyl acetone (GGA); and Group I comprises polyunsaturated fatty acids, such as e.g. omega-3 fatty acids, including for example eicosapentaenoic acid (EPA), and docosahexaenoic acid (DHA).
Example 2-9. Any combination comprising or consisting of One Member of Group A and One Member of Group J (see Table 1). In alternative aspects, a therapy provided herein comprises or consists of at least one member selected from Group A, and at least one member selected from Group J, as shown in Table 1, where: of Group A comprises or consists of, or Group A comprises use of or consists of use of, geranylgeranyl compounds, including for example, geranylgeranyl acetone (GGA) and analogs of geranylgeranyl acetone (GGA); and Group J comprises α-difluoromethylornithine.
Example 2-10. Any combination comprising or consisting of One Member of Group A and One Member of Group K (see Table 1). In alternative aspects, a therapy provided herein comprises or consists of at least one member selected from Group A, and at least one member selected from Group K, as shown in Table 1, where: of Group A comprises or consists of, or Group A comprises use of or consists of use of, geranylgeranyl compounds, including for example, geranylgeranyl acetone (GGA) and analogs of geranylgeranyl acetone (GGA); and Group K comprises superoxide dismutase (SOD) and similar antioxidant compounds.
Example 2-11. Any combination comprising or consisting of One Member of Group A and One Member of Group L (see Table 1). In alternative aspects, a therapy provided herein comprises or consists of at least one member selected from Group A, and at least one member selected from Group L, as shown in Table 1, where: of Group A comprises or consists of, or Group A comprises use of or consists of use of, geranylgeranyl compounds, including for example, geranylgeranyl acetone (GGA) and analogs of geranylgeranyl acetone (GGA); and Group L comprises activators of heat shock response.
Example 2-12. Any combination comprising or consisting of One Member of Group A and One Member of Group M (see Table 1). In alternative aspects, a therapy provided herein comprises or consists of at least one member selected from Group A, and at least one member selected from Group M, as shown in Table 1, where: of Group A comprises or consists of, or Group A comprises use of or consists of use of, geranylgeranyl compounds, including for example, geranylgeranyl acetone (GGA) and analogs of geranylgeranyl acetone (GGA); and Group M comprises drugs that reduce the inflammatory/progressive tissue damage response.
Example 2-13. Any combination comprising or consisting of One Member of Group B and One Member of Group C (see Table 1). In alternative aspects, a therapy provided herein comprises or consists of at least one member selected from Group B, and at least one member selected from Group C, as shown in Table 1, where: Group B comprises use of or consists of use of Angiotensin Converting Enzyme Inhibitors (ACE Inhibitors or ACE-Is); and Group C comprises use of or consists of use of Angiotensin Receptor Blockers (ARBs).
Example 2-14. Any combination comprising or consisting of One Member of Group B and One Member of Group D (see Table 1). In alternative aspects, a therapy provided herein comprises or consists of at least one member selected from Group B, and at least one member selected from Group D, as shown in Table 1, where: Group B comprises use of or consists of use of Angiotensin Converting Enzyme Inhibitors (ACE Inhibitors or ACE-Is); and Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands).
Example 2-15. Any combination comprising or consisting of One Member of Group B and One Member of Group E (see Table 1). In alternative aspects, a therapy provided herein comprises or consists of at least one member selected from Group B, and at least one member selected from Group E, as shown in Table 1, where: Group B comprises use of or consists of use of Angiotensin Converting Enzyme Inhibitors (ACE Inhibitors or ACE-Is); and Group E comprises use of or consists of use of non-steroidal anti-inflammatory drugs (NSAIDs).
Example 2-16. Any combination comprising or consisting of One Member of Group B and One Member of Group F (see Table 1). In alternative aspects, a therapy provided herein comprises or consists of at least one member selected from Group B, and at least one member selected from Group F, as shown in Table 1, where: Group B comprises use of or consists of use of Angiotensin Converting Enzyme Inhibitors (ACE Inhibitors or ACE-Is); and Group F consists of NCCN therapies.
Example 2-17. Any combination comprising or consisting of One Member of Group B and One Member of Group G (see Table 1). In alternative aspects, a therapy provided herein comprises or consists of at least one member selected from Group B, and at least one member selected from Group G, as shown in Table 1, where: Group B comprises use of or consists of use of Angiotensin Converting Enzyme Inhibitors (ACE Inhibitors or ACE-Is); and Group G comprises use of or consists of use of TNF inhibitors, e.g. pentoxifylline, or 1-(5-oxohexyl)-3, 7-dimethylxanthine (e.g., TRENTAL™).
Example 2-18. Any combination comprising or consisting of One Member of Group B and One Member of Group H (see Table 1). In alternative aspects, a therapy provided herein comprises or consists of at least one member selected from Group B, and at least one member selected from Group H, as shown in Table 1, where: Group B comprises use of or consists of use of Angiotensin Converting Enzyme Inhibitors (ACE Inhibitors or ACE-Is); and Group H comprises use of or consists of use of deferrioxamine.
Example 2-19. Any combination comprising or consisting of One Member of Group B and One Member of Group I (see Table 1). In alternative aspects, a therapy provided herein comprises or consists of at least one member selected from Group B, and at least one member selected from Group I, as shown in Table 1, where: Group B comprises use of or consists of use of Angiotensin Converting Enzyme Inhibitors (ACE Inhibitors or ACE-Is); and Group I comprises polyunsaturated fatty acids, such as e.g. omega-3 fatty acids, including for example eicosapentaenoic acid (EPA), and docosahexaenoic acid (DHA).
Example 2-20. Any combination comprising or consisting of One Member of Group B and One Member of Group J (see Table 1). In alternative aspects, a therapy provided herein comprises or consists of at least one member selected from Group B, and at least one member selected from Group J, as shown in Table 1, where: Group B comprises use of or consists of use of Angiotensin Converting Enzyme Inhibitors (ACE Inhibitors or ACE-Is); and Group J comprises α-difluoromethylornithine.
Example 2-21. Any combination comprising or consisting of One Member of Group B and One Member of Group K (see Table 1). In alternative aspects, a therapy provided herein comprises or consists of at least one member selected from Group B, and at least one member selected from Group K, as shown in Table 1, where: Group B comprises use of or consists of use of Angiotensin Converting Enzyme Inhibitors (ACE Inhibitors or ACE-Is); and Group K comprises superoxide dismutase (SOD) and similar antioxidant compounds.
Example 2-22. Any combination comprising or consisting of One Member of Group B and One Member of Group L (see Table 1). In alternative aspects, a therapy provided herein comprises or consists of at least one member selected from Group B, and at least one member selected from Group L, as shown in Table 1, where: Group B comprises use of or consists of use of Angiotensin Converting Enzyme Inhibitors (ACE Inhibitors or ACE-Is); and Group L comprises activators of heat shock response.
Example 2-23. Any combination comprising or consisting of One Member of Group B and One Member of Group M (see Table 1). In alternative aspects, a therapy provided herein comprises or consists of at least one member selected from Group B, and at least one member selected from Group M, as shown in Table 1, where: Group B comprises use of or consists of use of Angiotensin Converting Enzyme Inhibitors (ACE Inhibitors or ACE-Is); and Group M comprises drugs that reduce the inflammatory/progressive tissue damage response.
Example 2-24. Any combination comprising or consisting of One Member of Group C and One Member of Group D (see Table 1). In alternative aspects, a therapy provided herein comprises or consists of at least one member selected from Group C, and at least one member selected from Group D, as shown in Table 1, where: Group C comprises use of or consists of use of Angiotensin Receptor Blockers (ARBs); and Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands).
Example 2-25. Any combination comprising or consisting of One Member of Group C and One Member of Group E (see Table 1). In alternative aspects, a therapy provided herein comprises or consists of at least one member selected from Group C, and at least one member selected from Group E, as shown in Table 1, where: Group C comprises use of or consists of use of Angiotensin Receptor Blockers (ARBs); and Group E comprises use of or consists of use of non-steroidal anti-inflammatory drugs (NSAIDs).
Example 2-26. Any combination comprising or consisting of One Member of Group C and One Member of Group F (see Table 1). In alternative aspects, a therapy provided herein comprises or consists of at least one member selected from Group C, and at least one member selected from Group F, as shown in Table 1, where: Group C comprises use of or consists of use of Angiotensin Receptor Blockers (ARBs); and Group F consists of NCCN therapies.
Example 2-27. Any combination comprising or consisting of One Member of Group C and One Member of Group G (see Table 1). In alternative aspects, a therapy provided herein comprises or consists of at least one member selected from Group C, and at least one member selected from Group G, as shown in Table 1, where: Group C comprises use of or consists of use of Angiotensin Receptor Blockers (ARBs); and Group G comprises use of or consists of use of TNF inhibitors, e.g. pentoxifylline, or 1-(5-oxohexyl)-3, 7-dimethylxanthine (e.g., TRENTAL™).
Example 2-28. Any combination comprising or consisting of One Member of Group C and One Member of Group H (see Table 1). In alternative aspects, a therapy provided herein comprises or consists of at least one member selected from Group C, and at least one member selected from Group H, as shown in Table 1, where: Group C comprises use of or consists of use of Angiotensin Receptor Blockers (ARBs); and Group H comprises use of or consists of use of deferrioxamine.
Example 2-29. Any combination comprising or consisting of One Member of Group C and One Member of Group I (see Table 1). In alternative aspects, a therapy provided herein comprises or consists of at least one member selected from Group C, and at least one member selected from Group I, as shown in Table 1, where: Group C comprises use of or consists of use of Angiotensin Receptor Blockers (ARBs); and Group I comprises polyunsaturated fatty acids, such as e.g. omega-3 fatty acids, including for example eicosapentaenoic acid (EPA), and docosahexaenoic acid (DHA).
Example 2-30. Any combination comprising or consisting of One Member of Group C and One Member of Group J (see Table 1). In alternative aspects, a therapy provided herein comprises or consists of at least one member selected from Group C, and at least one member selected from Group J, as shown in Table 1, where: Group C comprises use of or consists of use of Angiotensin Receptor Blockers (ARBs); and Group J comprises α-difluoromethylornithine.
Example 2-31. Any combination comprising or consisting of One Member of Group C and One Member of Group K (see Table 1). In alternative aspects, a therapy provided herein comprises or consists of at least one member selected from Group C, and at least one member selected from Group K, as shown in Table 1, where: Group C comprises use of or consists of use of Angiotensin Receptor Blockers (ARBs); and Group K comprises superoxide dismutase (SOD) and similar antioxidant compounds.
Example 2-32. Any combination comprising or consisting of One Member of Group C and One Member of Group L (see Table 1). In alternative aspects, a therapy provided herein comprises or consists of at least one member selected from Group C, and at least one member selected from Group L, as shown in Table 1, where: Group C comprises use of or consists of use of Angiotensin Receptor Blockers (ARBs); and Group L comprises activators of heat shock response.
Example 2-33. Any combination comprising or consisting of One Member of Group C and One Member of Group M (see Table 1). In alternative aspects, a therapy provided herein comprises or consists of at least one member selected from Group C, and at least one member selected from Group M, as shown in Table 1, where: Group C comprises use of or consists of use of Angiotensin Receptor Blockers (ARBs); and Group M comprises drugs that reduce the inflammatory/progressive tissue damage response.
Example 2-34. Any combination comprising or consisting of One Member of Group D and One Member of Group E (see Table 1). In alternative aspects, a therapy provided herein comprises or consists of at least one member selected from Group D, and at least one member selected from Group E, as shown in Table 1, where: Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); and Group E comprises use of or consists of use of non-steroidal anti-inflammatory drugs (NSAIDs).
Example 2-35. Any combination comprising or consisting of One Member of Group D and One Member of Group F (see Table 1). In alternative aspects, a therapy provided herein comprises or consists of at least one member selected from Group D, and at least one member selected from Group F, as shown in Table 1, where: Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); and Group F consists of NCCN therapies.
Example 2-36. Any combination comprising or consisting of One Member of Group D and One Member of Group G (see Table 1). In alternative aspects, a therapy provided herein comprises or consists of at least one member selected from Group D, and at least one member selected from Group G, as shown in Table 1, where: Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); and Group G comprises use of or consists of use of TNF inhibitors, e.g. pentoxifylline, or 1-(5-oxohexyl)-3, 7-dimethylxanthine (e.g., TRENTAL™).
Example 2-37. Any combination comprising or consisting of One Member of Group D and One Member of Group H (see Table 1). In alternative aspects, a therapy provided herein comprises or consists of at least one member selected from Group D, and at least one member selected from Group H, as shown in Table 1, where: Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); and Group H comprises use of or consists of use of deferrioxamine.
Example 2-38. Any combination comprising or consisting of One Member of Group D and One Member of Group I (see Table 1). In alternative aspects, a therapy provided herein comprises or consists of at least one member selected from Group D, and at least one member selected from Group I, as shown in Table 1, where: Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); and Group I comprises polyunsaturated fatty acids, such as e.g. omega-3 fatty acids, including for example eicosapentaenoic acid (EPA), and docosahexaenoic acid (DHA).
Example 2-39. Any combination comprising or consisting of One Member of Group D and One Member of Group J (see Table 1). In alternative aspects, a therapy provided herein comprises or consists of at least one member selected from Group D, and at least one member selected from Group J, as shown in Table 1, where: Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); and Group J comprises α-difluoromethylornithine.
Example 2-40. Any combination comprising or consisting of One Member of Group D and One Member of Group K (see Table 1). In alternative aspects, a therapy provided herein comprises or consists of at least one member selected from Group D, and at least one member selected from Group K, as shown in Table 1, where: Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); and Group K comprises superoxide dismutase (SOD) and similar antioxidant compounds.
Example 2-41. Any combination comprising or consisting of One Member of Group D and One Member of Group L (see Table 1). In alternative aspects, a therapy provided herein comprises or consists of at least one member selected from Group D, and at least one member selected from Group L, as shown in Table 1, where: Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); and Group L comprises activators of heat shock response.
Example 2-42. Any combination comprising or consisting of One Member of Group D and One Member of Group M (see Table 1). In alternative aspects, a therapy provided herein comprises or consists of at least one member selected from Group D, and at least one member selected from Group M, as shown in Table 1, where: Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); and Group M comprises drugs that reduce the inflammatory/progressive tissue damage response.
Example 2-43. Any combination comprising or consisting of One Member of Group E and One Member of Group F (see Table 1). In alternative aspects, a therapy provided herein comprises or consists of at least one member selected from Group E, and at least one member selected from Group F, as shown in Table 1, where: Group E comprises use of or consists of use of non-steroidal anti-inflammatory drugs (NSAIDs); and Group F consists of NCCN therapies.
Example 2-44. Any combination comprising or consisting of One Member of Group E and One Member of Group G (see Table 1). In alternative aspects, a therapy provided herein comprises or consists of at least one member selected from Group E, and at least one member selected from Group G, as shown in Table 1, where: Group E comprises use of or consists of use of non-steroidal anti-inflammatory drugs (NSAIDs); and Group G comprises use of or consists of use of TNF inhibitors, e.g. pentoxifylline, or 1-(5-oxohexyl)-3, 7-dimethylxanthine (e.g., TRENTAL™).
Example 2-45. Any combination comprising or consisting of One Member of Group E and One Member of Group H (see Table 1). In alternative aspects, a therapy provided herein comprises or consists of at least one member selected from Group E, and at least one member selected from Group H, as shown in Table 1, where: Group E comprises use of or consists of use of non-steroidal anti-inflammatory drugs (NSAIDs); and Group H comprises use of or consists of use of deferrioxamine.
Example 2-46. Any combination comprising or consisting of One Member of Group E and One Member of Group I (see Table 1). In alternative aspects, a therapy provided herein comprises or consists of at least one member selected from Group E, and at least one member selected from Group I, as shown in Table 1, where: Group E comprises use of or consists of use of non-steroidal anti-inflammatory drugs (NSAIDs); and Group I comprises polyunsaturated fatty acids, such as e.g. omega-3 fatty acids, including for example eicosapentaenoic acid (EPA), and docosahexaenoic acid (DHA).
Example 2-47. Any combination comprising or consisting of One Member of Group E and One Member of Group J (see Table 1). In alternative aspects, a therapy provided herein comprises or consists of at least one member selected from Group E, and at least one member selected from Group J, as shown in Table 1, where: Group E comprises use of or consists of use of non-steroidal anti-inflammatory drugs (NSAIDs); and Group J comprises α-difluoromethylornithine.
Example 2-48. Any combination comprising or consisting of One Member of Group E and One Member of Group K (see Table 1). In alternative aspects, a therapy provided herein comprises or consists of at least one member selected from Group E, and at least one member selected from Group K, as shown in Table 1, where: Group E comprises use of or consists of use of non-steroidal anti-inflammatory drugs (NSAIDs); and Group K comprises superoxide dismutase (SOD) and similar antioxidant compounds.
Example 2-49. Any combination comprising or consisting of One Member of Group E and One Member of Group L (see Table 1). In alternative aspects, a therapy provided herein comprises or consists of at least one member selected from Group E, and at least one member selected from Group L, as shown in Table 1, where: Group E comprises use of or consists of use of non-steroidal anti-inflammatory drugs (NSAIDs); and Group L comprises activators of heat shock response.
Example 2-50. Any combination comprising or consisting of One Member of Group E and One Member of Group M (see Table 1). In alternative aspects, a therapy provided herein comprises or consists of at least one member selected from Group E, and at least one member selected from Group M, as shown in Table 1, where: Group E comprises use of or consists of use of non-steroidal anti-inflammatory drugs (NSAIDs); and Group M comprises drugs that reduce the inflammatory/progressive tissue damage response.
Example 2-51. Any combination comprising or consisting of One Member of Group F and One Member of Group G (see Table 1). In alternative aspects, a therapy provided herein comprises or consists of at least one member selected from Group F, and at least one member selected from Group G, as shown in Table 1, where: Group F consists of NCCN therapies; and Group G comprises use of or consists of use of TNF inhibitors, e.g. pentoxifylline, or 1-(5-oxohexyl)-3, 7-dimethylxanthine (e.g., TRENTAL™).
Example 2-52. Any combination comprising or consisting of One Member of Group F and One Member of Group H (see Table 1). In alternative aspects, a therapy provided herein comprises or consists of at least one member selected from Group F, and at least one member selected from Group H, as shown in Table 1, where: Group F consists of NCCN therapies; and Group H comprises use of or consists of use of deferrioxamine.
Example 2-53. Any combination comprising or consisting of One Member of Group F and One Member of Group I (see Table 1). In alternative aspects, a therapy provided herein comprises or consists of at least one member selected from Group F, and at least one member selected from Group I, as shown in Table 1, where: Group F consists of NCCN therapies; and Group I comprises polyunsaturated fatty acids, such as e.g. omega-3 fatty acids, including for example eicosapentaenoic acid (EPA), and docosahexaenoic acid (DHA).
Example 2-54. Any combination comprising or consisting of One Member of Group F and One Member of Group J (see Table 1). In alternative aspects, a therapy provided herein comprises or consists of at least one member selected from Group F, and at least one member selected from Group J, as shown in Table 1, where: Group F consists of NCCN therapies; and Group J comprises α-difluoromethylornithine.
Example 2-55. Any combination comprising or consisting of One Member of Group F and One Member of Group K (see Table 1). In alternative aspects, a therapy provided herein comprises or consists of at least one member selected from Group F, and at least one member selected from Group K, as shown in Table 1, where: Group F consists of NCCN therapies; and Group K comprises superoxide dismutase (SOD) and similar antioxidant compounds.
Example 2-56. Any combination comprising or consisting of One Member of Group F and One Member of Group L (see Table 1). In alternative aspects, a therapy provided herein comprises or consists of at least one member selected from Group F, and at least one member selected from Group L, as shown in Table 1, where: Group F consists of NCCN therapies; and Group L comprises activators of heat shock response.
Example 2-57. Any combination comprising or consisting of One Member of Group F and One Member of Group M (see Table 1). In alternative aspects, a therapy provided herein comprises or consists of at least one member selected from Group F, and at least one member selected from Group M, as shown in Table 1, where: Group F consists of NCCN therapies; and Group M comprises drugs that reduce the inflammatory/progressive tissue damage response.
Example 2-58. Any combination comprising or consisting of One Member of Group G and One Member of Group H (see Table 1). In alternative aspects, a therapy provided herein comprises or consists of at least one member selected from Group G, and at least one member selected from Group H, as shown in Table 1, where: Group G comprises use of or consists of use of TNF inhibitors, e.g. pentoxifylline; and Group H comprises use of or consists of use of deferrioxamine.
Example 2-59. Any combination comprising or consisting of One Member of Group G and One Member of Group I (see Table 1). In alternative aspects, a therapy provided herein comprises or consists of at least one member selected from Group G, and at least one member selected from Group I, as shown in Table 1, where: Group G comprises use of or consists of use of TNF inhibitors, e.g. pentoxifylline; and Group I comprises polyunsaturated fatty acids, such as e.g. omega-3 fatty acids, including for example eicosapentaenoic acid (EPA), and docosahexaenoic acid (DHA).
Example 2-60. Any combination comprising or consisting of One Member of Group G and One Member of Group J (see Table 1). In alternative aspects, a therapy provided herein comprises or consists of at least one member selected from Group G, and at least one member selected from Group J, as shown in Table 1, where: Group G comprises use of or consists of use of TNF inhibitors, e.g. pentoxifylline; and Group J comprises α-difluoromethylornithine.
Example 2-61. Any combination comprising or consisting of One Member of Group G and One Member of Group K (see Table 1). In alternative aspects, a therapy provided herein comprises or consists of at least one member selected from Group G, and at least one member selected from Group K, as shown in Table 1, where: Group G comprises use of or consists of use of TNF inhibitors, e.g. pentoxifylline; and Group K comprises superoxide dismutase (SOD) and similar antioxidant compounds.
Example 2-62. Any combination comprising or consisting of One Member of Group G and One Member of Group L (see Table 1). In alternative aspects, a therapy provided herein comprises or consists of at least one member selected from Group G, and at least one member selected from Group L, as shown in Table 1, where: Group G comprises use of or consists of use of TNF inhibitors, e.g. pentoxifylline; and Group L comprises activators of heat shock response.
Example 2-63. Any combination comprising or consisting of One Member of Group G and One Member of Group M (see Table 1). In alternative aspects, a therapy provided herein comprises or consists of at least one member selected from Group G, and at least one member selected from Group M, as shown in Table 1, where: Group G comprises use of or consists of use of TNF inhibitors, e.g. pentoxifylline; and Group M comprises drugs that reduce the inflammatory/progressive tissue damage response.
Example 2-64. Any combination comprising or consisting of One Member of Group H and One Member of Group I (see Table 1). In alternative aspects, a therapy provided herein comprises or consists of at least one member selected from Group H, and at least one member selected from Group I, as shown in Table 1, where: Group H comprises use of or consists of use of deferrioxamine; and Group I comprises polyunsaturated fatty acids, such as e.g. omega-3 fatty acids, including for example eicosapentaenoic acid (EPA), and docosahexaenoic acid (DHA).
Example 2-65. Any combination comprising or consisting of One Member of Group H and One Member of Group J (see Table 1). In alternative aspects, a therapy provided herein comprises or consists of at least one member selected from Group H, and at least one member selected from Group J, as shown in Table 1, where: Group H comprises use of or consists of use of deferrioxamine; and Group J comprises α-difluoromethylornithine.
Example 2-66. Any combination comprising or consisting of One Member of Group H and One Member of Group K (see Table 1). In alternative aspects, a therapy provided herein comprises or consists of at least one member selected from Group H, and at least one member selected from Group K, as shown in Table 1, where: Group H comprises use of or consists of use of deferrioxamine; and Group K comprises superoxide dismutase (SOD) and similar antioxidant compounds.
Example 2-67. Any combination comprising or consisting of One Member of Group H and One Member of Group L (see Table 1). In alternative aspects, a therapy provided herein comprises or consists of at least one member selected from Group H, and at least one member selected from Group L, as shown in Table 1, where: Group H comprises use of or consists of use of deferrioxamine; and Group L comprises activators of heat shock response.
Example 2-68. Any combination comprising or consisting of One Member of Group H and One Member of Group M (see Table 1). In alternative aspects, a therapy provided herein comprises or consists of at least one member selected from Group H, and at least one member selected from Group M, as shown in Table 1, where: Group H comprises use of or consists of use of deferrioxamine; and Group M comprises drugs that reduce the inflammatory/progressive tissue damage response.
Example 2-69. Any combination comprising or consisting of One Member of Group I and One Member of Group J (see Table 1). In alternative aspects, a therapy provided herein comprises or consists of at least one member selected from Group I, and at least one member selected from Group J, as shown in Table 1, where: Group I comprises polyunsaturated fatty acids, such as e.g. omega-3 fatty acids, including for example eicosapentaenoic acid (EPA), and docosahexaenoic acid (DHA); and Group J comprises α-difluoromethylornithine.
Example 2-70. Any combination comprising or consisting of One Member of Group I and One Member of Group K (see Table 1). In alternative aspects, a therapy provided herein comprises or consists of at least one member selected from Group I, and at least one member selected from Group K, as shown in Table 1, where: Group I comprises polyunsaturated fatty acids, such as e.g. omega-3 fatty acids, including for example eicosapentaenoic acid (EPA), and docosahexaenoic acid (DHA); and Group K comprises superoxide dismutase (SOD) and similar antioxidant compounds.
Example 2-71. Any combination comprising or consisting of One Member of Group I and One Member of Group L (see Table 1). In alternative aspects, a therapy provided herein comprises or consists of at least one member selected from Group I, and at least one member selected from Group L, as shown in Table 1, where: Group I comprises polyunsaturated fatty acids, such as e.g. omega-3 fatty acids, including for example eicosapentaenoic acid (EPA), and docosahexaenoic acid (DHA); and Group L comprises activators of heat shock response.
Example 2-72. Any combination comprising or consisting of One Member of Group I and One Member of Group M (see Table 1). In alternative aspects, a therapy provided herein comprises or consists of at least one member selected from Group I, and at least one member selected from Group M, as shown in Table 1, where: Group I comprises polyunsaturated fatty acids, such as e.g. omega-3 fatty acids, including for example eicosapentaenoic acid (EPA), and docosahexaenoic acid (DHA); and Group M comprises drugs that reduce the inflammatory/progressive tissue damage response.
Example 2-73. Any combination comprising or consisting of One Member of Group J and One Member of Group K (see Table 1). In alternative aspects, a therapy provided herein comprises or consists of at least one member selected from Group J, and at least one member selected from Group K, as shown in Table 1, where: Group J comprises α-difluoromethylornithine; and Group K comprises superoxide dismutase (SOD) and similar antioxidant compounds.
Example 2-74. Any combination comprising or consisting of One Member of Group J and One Member of Group L (see Table 1). In alternative aspects, a therapy provided herein comprises or consists of at least one member selected from Group J, and at least one member selected from Group L, as shown in Table 1, where: Group J comprises α-difluoromethylornithine; and Group L comprises activators of heat shock response.
Example 2-75. Any combination comprising or consisting of One Member of Group J and One Member of Group M (see Table 1). In alternative aspects, a therapy provided herein comprises or consists of at least one member selected from Group J, and at least one member selected from Group M, as shown in Table 1, where: Group J comprises α-difluoromethylornithine; and Group M comprises drugs that reduce the inflammatory/progressive tissue damage response.
Example 2-76. Any combination comprising or consisting of One Member of Group K and One Member of Group L (see Table 1). In alternative aspects, a therapy provided herein comprises or consists of at least one member selected from Group K, and at least one member selected from Group L, as shown in Table 1, where: Group K comprises superoxide dismutase (SOD) and similar antioxidant compounds; and Group L comprises activators of heat shock response.
Example 2-77. Any combination comprising or consisting of One Member of Group K and One Member of Group M (see Table 1). In alternative aspects, a therapy provided herein comprises or consists of at least one member selected from Group K, and at least one member selected from Group M, as shown in Table 1, where: Group K comprises superoxide dismutase (SOD) and similar antioxidant compounds; and Group M comprises drugs that reduce the inflammatory/progressive tissue damage response.
Example 2-78. Any combination comprising or consisting of One Member of Group L and One Member of Group M (see Table 1). In alternative aspects, a therapy provided herein comprises or consists of at least one member selected from Group L, and at least one member selected from Group M, as shown in Table 1, where: Group L comprises activators of heat shock response; and Group M comprises drugs that reduce the inflammatory/progressive tissue damage response.

### Examples: "Triple Combination Therapies" (All "Combination Possibilities")

The invention provides compositions and therapies (methods) comprising use of any combination of at least one compound from at least three Groups (from Table 1). In additional embodiments, this invention comprises all possible "triple combination therapies" comprising a first member selected from a first Group as listed herein, a second member selected from a second Group as listed herein, and a third member selected from third Group as listed herein (the Groups are described in Table 1), where said first Group, said second Group, and said third Group may all be the same Group (e.g. Group A) or may be multiple Groups (e.g. Group A, Group B, and Group E). Thus, this invention also provides in separate embodiments, a therapy comprising every possible combination and permutation of three members selected from any of Group A, Group B, Group C, Group D, Group E, Group F, Group G, Group H, Group I, Group J, Group K, Group L, and/or Group M, as shown in Table 1, where: of Group A comprises or consists of, or Group A comprises use of or consists of use of, geranylgeranyl compounds, including for example, geranylgeranyl acetone (GGA) and analogs of geranylgeranyl acetone (GGA); Group B comprises use of or consists of use of Angiotensin Converting Enzyme Inhibitors (ACE Inhibitors or ACE-Is); Group C comprises use of or consists of use of Angiotensin Receptor Blockers (ARBs); Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises use of or consists of use of non-steroidal anti-inflammatory drugs (NSAIDs); Group F consists of NCCN therapies; Group G comprises use of or consists of use of TNF inhibitors, e.g. pentoxifylline; Group H comprises use of or consists of use of deferrioxamine; Group I comprises polyunsaturated fatty acids, such as e.g. omega-3 fatty acids, including for example eicosapentaenoic acid (EPA), and docosahexaenoic acid (DHA); Group J comprises α-difluoromethylornithine; Group K comprises superoxide dismutase (SOD) and similar antioxidant compounds; Group L comprises activators of heat shock response; and Group M comprises drugs that reduce the inflammatory/progressive tissue damage response, where said members selected to comprise the "triple combination therapy" may all be selected from the same Group (e.g. Group A) or may be selected from multiple Groups (e.g. Group A, Group B, and Group E).

### Examples: "Quadruple Combination Therapies" (All "Combination Possibilities")

The invention provides compositions and therapies (methods) comprising use of any combination of at least one compound from at least four Groups (from Table 1). In additional embodiments, this invention comprises all possible quadruple combinations comprising a first member selected from a first Group, a second member selected from a second Group, a third member selected from third Group, and a fourth member selected from a fourth Group (the Groups are described in Table 1). Thus, this invention also provides, in separate embodiments, a therapy comprising all combinations of four members selected from any of Group A, Group B, Group C, Group D, Group E, Group F, Group G, Group H, Group I, Group J, Group K, Group L, and/or Group M, as shown in Table 1, where: of Group A comprises or consists of, or Group A comprises use of or consists of use of, geranylgeranyl compounds, including for example, geranylgeranyl acetone (GGA) and analogs of geranylgeranyl acetone (GGA); Group B comprises use of or consists of use of Angiotensin Converting Enzyme Inhibitors (ACE Inhibitors or ACE-Is); Group C comprises use of or consists of use of Angiotensin Receptor Blockers (ARBs); Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises use of or consists of use of non-steroidal anti-inflammatory drugs (NSAIDs); Group F consists of NCCN therapies; Group G comprises use of or consists of use of TNF inhibitors, e.g. pentoxifylline; Group H comprises use of or consists of use of deferrioxamine; Group I comprises polyunsaturated fatty acids, such as e.g. omega-3 fatty acids, including for example eicosapentaenoic acid (EPA), and docosahexaenoic acid (DHA); Group J comprises α-difluoromethylornithine; Group K comprises superoxide dismutase (SOD) and similar antioxidant compounds; Group L comprises activators of heat shock response; and Group M comprises drugs that reduce the inflammatory/progressive tissue damage response, where said members selected to comprise the "quadruple combination therapy" may all be selected from the same Group (e.g. Group A) or may be selected from multiple Groups (e.g. Group A, Group B, Group D, and Group E).

### Examples" "Quintuple Combination Therapies" (All "Combination Possibilities")

The invention provides compositions and therapies (methods) comprising use of any combination of at least one compound from at least five Groups (from Table 1). In similar fashion, this invention comprises all possible quintuple combinations comprising a first member selected from a first Group, a second member selected from a second Group, a third member selected from third Group, a fourth member selected from a fourth Group, and a fifth member selected from a fifth Group (the Groups are described in Table 1). Thus, this invention also provides, in separate embodiments, a therapy comprising every possible combination and permutation of five members selected from any of Group A, Group B, Group C, Group D, Group E, Group F, Group G, Group H, Group I, Group J, Group K, Group L, and/or Group M, as shown in Table 1, where: of Group A comprises or consists of, or Group A comprises use of or consists of use of, geranylgeranyl compounds, including for example, geranylgeranyl acetone (GGA) and analogs of geranylgeranyl acetone (GGA); Group B comprises use of or consists of use of Angiotensin Converting Enzyme Inhibitors (ACE Inhibitors or ACE-Is); Group C comprises use of or consists of use of Angiotensin Receptor Blockers (ARBs); Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises use of or consists of use of non-steroidal anti-inflammatory drugs (NSAIDs); Group F consists of NCCN therapies; Group G comprises use of or consists of use of TNF inhibitors, e.g. pentoxifylline; Group H comprises use of or consists of use of deferrioxamine; Group I comprises polyunsaturated fatty acids, such as e.g. omega-3 fatty acids, including for example eicosapentaenoic acid (EPA), and docosahexaenoic acid (DHA); Group J comprises α-difluoromethylornithine; Group K comprises superoxide dismutase (SOD) and similar antioxidant compounds; Group L comprises activators of heat shock response; and Group M comprises drugs that reduce the inflammatory/progressive tissue damage response, where said members selected to comprise the "triple combination therapy" may all be selected from the same Group (e.g. Group A) or may be selected from multiple Groups (e.g. Group A, Group B, Group C, Group D, and Group E)..

Additional aspects of the invention include benefits, goals, purposes, and utilities provided herein, and include any combination and and/or permutation of the following benefits, goals, purposes, and utilities:
1) to allow or enable or facilitate the tolerance and use of higher amounts and/or doses and/or longer durations of a medical therapy (e.g. cancer therapies or radiation therapies) than could be used without this invention;
2) to allow or enable or facilitate the tolerance and use of longer durations of a medical therapy (e.g. cancer therapies or radiation therapies) than could be used without this invention;
3) to reduce occurrences of (drug) resistance to a medical therapy (e.g. cancer chemotherapy or radiation therapy; administration can be before, during and/or after the radiation therapy and/or cancer chemotherapy);
4) to enable dosage intensification of a medical therapy (e.g. cancer chemotherapy or radiation therapy; administration can be before, during and/or after the medical therapy);
5) to enable increasing the frequency of a medical therapy (e.g. cancer chemotherapy or radiation therapy; administration can be before, during and/or after the medical therapy);
6) to enhance patient response rates;
7) to increase patient survival;
8) to induce a tissue protective state;
9) to induce tissue regeneration; and
10) to induce tissue repair.

Other conditions, states and disease symptoms treated, prevented and/or ameliorated by the compositions and methods of the invention are exemplified by any combination of the following examples: intolerance to cancer therapy (e.g. chemotherapy or radiation therapy), wasting, and/or atrophy, such as muscle atrophy.

The invention provides any combination and/or permutation of the following: treating, preventing and/or ameliorating conditions, diseases, symptoms and/or disease states comprising or caused by, or as a side effect of, cancer and any conditions caused by dysfunctional cells, inflammation, excessive sympathoneural drive (sympathetic nerve activity), cachexia, anorexia, and anorexia-cachexia, and stress or anxiety related thereto for the purpose of improving one or more undesirable symptoms associated with these conditions, diseases, symptoms and/or disease states, or for slowing the progression (worsening) of one or more symptoms associated with these conditions, diseases, symptoms and/or disease states.

In one aspect, this invention provides a product (e.g. a cytoprotection product) and/or a therapy (e.g. a cytoprotection therapy), that is selective for (e.g. selectively affects) normal tissue, e.g. non-cancer tissue that is not the intended target of cancer therapy (such as chemotherapy or radiation therapy). In one embodiment, the cytoprotection provided by this invention includes the protection of tissues at the cellular level; and in another embodiment, the cytoprotection provided by this invention includes the protection of tissues at the organ system level.

In one aspect this invention provides a cytoprotection product and a cytoprotection therapy that can preferentially target tissue compartment and/or a cell type that are in a normal or unstressed state over tissue compartments and cell types that are in a stressed physiological state.

This invention provides a product (e.g. a cytoprotection therapy) and/or a therapy (e.g. a cytoprotection therapy), that will induce a protective state in normal host tissue (e.g. non-cancer tissue) compartments and cells (including, in different embodiments, mucosal tissue compartment(s), bone marrow, brain, heart, lung,, kidney, liver, GI track, auditory compartment(s), dermal/scalp compartment(s), etc.) preferentially over abnormal stressed tissue compartments and stressed cancer cells. As used herein, this invention provides that stressed cancer cells and associated tissue compartment(s) have already undergone a stress response and are not (or not as) responsive to a therapy or composition intended to provide prophylactic cytoprotection to non-cancerous and and/or normal cells.

This invention provides combination preparations and formulations, kits and other products of manufacture (e.g., blister packs, tablets, capsules, pills and the like) that can be used as cytoprotective supportive care therapies and can be furthermore used as adjunct therapies for multi-drug-resistant cases.

In alternative embodiments of this invention, a cytoprotection product or a cytoprotection therapy is exemplified by a therapeutic combination comprising at least one member of a first group and at least one member of a second group; wherein members of the first group are selected from the group consisting of angiotensin-converting enzyme (ACE) inhibitors, and angiotensin receptor blockers (ARBs). In one aspect, the therapeutic combination comprises: at least one angiotensin-converting enzyme (ACE) inhibitors; and at least one angiotensin receptor blocker (ARB).

In one aspect, the therapeutic combinations comprise at least one member of the first group and the at least one member of the second group are formulated as separate compositions. In one aspect, the therapeutic combinations comprise at least one member of the first group and the at least one member of the group are formulated in the same composition. In one aspect, each member of each selected group is formulated as a separate composition, or, all selected members are formulated in the same composition, or, a combination thereof. In one aspect, each member of each selected group is manufactured in a separate package or container (e.g., a "blister package"), or, all selected members are manufactured in the same package or container, or, any combination thereof.

The invention provides pharmaceutical compositions comprising a therapeutic combination of the invention; or, therapeutic combination of the invention can be singly or multiply formulated or packaged as one or more pharmaceutical compositions. The pharmaceutical compositions can further comprise any pharmaceutically acceptable excipient. Compositions used in the therapeutic combinations of the invention, e.g., the pharmaceutical compositions of the invention, can be formulated in or as a feed, a food, a liquid, an elixir, an aerosol, a spray, a powder, a tablet, a pill, a capsule, a gel, a geltab, a nanosuspension, a nanoparticle, a microgel or a suppository.

In one aspect of the methods, the therapeutic combination of the invention, or the pharmaceutical composition of the invention, is administered in single or multiple doses, and optionally the pharmaceutical compositions are packaged in a single or a plurality of packages or packets.

In one aspect of the methods, the therapeutic combination of the invention, or the pharmaceutical composition of the invention, is administered intravenously, topically, orally, by inhalation, by infusion, by injection, intraperitoneally, intramuscularly, subcutaneously, intra-aurally, by intra-articular administration, by intra-mammary administration, by topical administration or by absorption through epithelial or mucocutaneous linings.

The invention provides kits comprising the therapeutic combination of the invention, or the pharmaceutical composition of the invention. The kit can comprise at least one package, e.g., a blister pack, containing any one or more of a therapeutic combination of any of the invention, or the pharmaceutical composition of the invention.

In alternative aspects, the therapeutic combination of the invention, or the pharmaceutical composition of the invention, is formulated for administration intravenously, topically, orally, by inhalation, by infusion, by injection, intraperitoneally, intramuscularly, subcutaneously, intra-aurally, for intra-articular administration, for intra-mammary administration, for topical administration or for absorption through epithelial or mucocutaneous linings.

The therapeutic combination of the invention, or the pharmaceutical composition of the invention, can be packaged for administration intravenously, topically, orally, by inhalation, by infusion, by injection, intraperitoneally, intramuscularly, subcutaneously, intra-aurally, for intra-articular administration, for intra-mammary administration, for topical administration or for absorption through epithelial or mucocutaneous linings.

In one aspect, therapeutic combinations of the invention, or pharmaceutical compositions of the invention (e.g., the multi-ingredient combinations of drugs of the invention), are formulated for chrono-dosing, or the methods of the invention comprise use of chrono-dosing or escalating dosage regimens. For example, in one aspect the therapeutic combinations of the invention, or pharmaceutical compositions of the invention, are formulated for administration once a day, b.i.d. or t.i.d or more often, or for continuous administration. In one aspect, therapeutic combinations of the invention, or pharmaceutical compositions of the invention, are formulated and dosaged as set forth in any one of exemplary ingredient combinations.

The invention provides methods wherein a formulation or preparation is administered by oral means, inhalation, infusion or injection, topical application or by absorption through epithelial or mucocutaneous linings.

The invention provides liquids comprising the preparation of the invention, or formulation of the invention. The invention provides capsules, sprays, powders, lotions, tablets or pills comprising a preparation of the invention or formulation of the invention.

The invention provides foods or food supplements comprising a preparation of the invention or a formulation of the invention. The food or food supplement can comprise a flavored bar, a power bar, a diet bar, an energy bar or a nutritional bar.

The invention provides methods for maintaining the health of a tissue comprising administering an effective amount of a preparation of the invention or a formulation of the invention. The tissue can be skeletal muscle tissue, fat tissue, or more than one body tissue type, including the majority of the body. The invention provides methods for ameliorating a disease or condition in an individual comprising administering an effective amount of a preparation of the invention or a formulation of the invention. The disease or condition can affect skeletal muscle tissue or fat tissue.

In separate embodiments, the product this invention provides different products that comprise (contain at least), or methods that use, all the combinations and permutations of any ingredients provided herein by specific mention. This invention also provides different products that comprise (contain at least) all the combinations and permutations of any ingredients provided herein, if not by specific mention of said ingredients, then by knowledge in the art that said ingredients are part of one or more category or group of ingredients provided herein.

In separate embodiments, this invention provides different products that comprise (contain at least), or methods that use, all the combinations and permutations of ingredients selected from members of Group A, Group B, Group C, Group D, Group E, Group F, Group G, Group H, Group I, Group J, Group K, Group L, and/or Group M, as shown in Table 1, where: of Group A comprises or consists of, or Group A comprises use of or consists of use of, geranylgeranyl compounds, including for example, geranylgeranyl acetone (GGA) and analogs of geranylgeranyl acetone (GGA); Group B comprises use of or consists of use of Angiotensin Converting Enzyme Inhibitors (ACE Inhibitors or ACE-Is); Group C comprises use of or consists of use of Angiotensin Receptor Blockers (ARBs); Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises use of or consists of use of non-steroidal anti-inflammatory drugs (NSAIDs); Group F consists of NCCN therapies; Group G comprises use of or consists of use of TNF inhibitors, e.g. pentoxifylline; Group H comprises use of or consists of use of deferrioxamine; Group I comprises polyunsaturated fatty acids, such as e.g. omega-3 fatty acids, including for example eicosapentaenoic acid (EPA), and docosahexaenoic acid (DHA); Group J comprises α-difluoromethylornithine; Group K comprises superoxide dismutase (SOD) and similar antioxidant compounds; Group L comprises activators of heat shock response; and Group M comprises drugs that reduce the inflammatory/progressive tissue damage response.

By way of illustration, in separate embodiments, this invention provides kits and packages containing therapeutic preparations, which therapeutic preparations that comprise (contain at least) all the combinations and permutations of from any one ingredient to any 100 ingredients selected from members of Group A, Group B, Group C, Group D, Group E, Group F, Group G, Group H, Group I, Group J, Group K, Group L, and/or Group M, as shown in Table 1, where: of Group A comprises or consists of, or Group A comprises use of or consists of use of, geranylgeranyl compounds, including for example, geranylgeranyl acetone (GGA) and analogs of geranylgeranyl acetone (GGA); Group B comprises use of or consists of use of Angiotensin Converting Enzyme Inhibitors (ACE Inhibitors or ACE-Is); Group C comprises use of or consists of use of Angiotensin Receptor Blockers (ARBs); Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises use of or consists of use of non-steroidal anti-inflammatory drugs (NSAIDs); Group F consists of NCCN therapies; Group G comprises use of or consists of use of TNF inhibitors, e.g. pentoxifylline; Group H comprises use of or consists of use of deferrioxamine; Group I comprises polyunsaturated fatty acids, such as e.g. omega-3 fatty acids, including for example eicosapentaenoic acid (EPA), and docosahexaenoic acid (DHA); Group J comprises α-difluoromethylornithine; Group K comprises superoxide dismutase (SOD) and similar antioxidant compounds; Group L comprises activators of heat shock response; and Group M comprises drugs that reduce the inflammatory/progressive tissue damage response.

In one aspect, the term "at least one member" in reference to exemplary alternative embodiments comprises minimally every integer value from one to about 20 or more, inclusive; i.e. in one aspect "at least one member" in this context means at least one member, in another aspect "at least one member" in this context means at least two members, in another aspect "at least one member" in this context means at least three members, ..., etc, and in another aspect "at least one member" in this context means at least 20 members. In further alternative embodiments "at least one member" in this context can comprise more than 20 members.

In one embodiment, this invention provides every combination and permutation of ingredients exemplified in Table 1 (i.e. from members of Group A, Group B, Group C, Group D, Group E, Group F, Group G, Group H, Group I, Group J, Group K, Group L, and/or Group M, as shown in Table 1, for example). Exemplary combinations and permutations provided herein comprise ingredients selected from at least 1 group, where the at least 1 group is selected from any of Group A, Group B, Group C, Group D, Group E, Group F, Group G, Group H, Group I, Group J, Group K, Group L, and/or Group M, as shown in Table 1. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of members of Group A, Group B, Group C, Group D, Group E, Group F, Group G, Group H, Group I, Group J, Group K, Group L, and/or Group M, as shown in Table 1.

Exemplary combinations and permutations provided herein comprise ingredients selected from at least 2 groups, where the at least 2 groups are selected from any of members of Group A, Group B, Group C, Group D, Group E, Group F, Group G, Group H, Group I, Group J, Group K, Group L, and/or Group M, as shown in Table 1. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of members of Group A, Group B, Group C, Group D, Group E, Group F, Group G, Group H, Group I, Group J, Group K, Group L, and Group M, as shown in Table 1.

Exemplary combinations and permutations provided herein comprise ingredients selected from at least 3 groups, where the at least 3 groups are selected from any of members of Group A, Group B, Group C, Group D, Group E, Group F, Group G, Group H, Group I, Group J, Group K, Group L, and/or Group M, as shown in Table 1. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of Groups 1-5 members of Group A, Group B, Group C, Group D, Group E, Group F, Group G, Group H, Group I, Group J, Group K, Group L, and Group M, as shown in Table 1.

Exemplary combinations and permutations provided herein comprise ingredients selected from at least 4 groups, where the at least 4 groups are selected from any of members of Group A, Group B, Group C, Group D, Group E, Group F, Group G, Group H, Group I, Group J, Group K, Group L, and/or Group M, as shown in Table 1. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of members of Group A, Group B, Group C, Group D, Group E, Group F, Group G, Group H, Group I, Group J, Group K, Group L, and Group M, as shown in Table 1.

Exemplary combinations and permutations provided herein comprise ingredients selected from at least 5 groups, where the at least 5 groups are selected from any of members of Group A, Group B, Group C, Group D, Group E, Group F, Group G, Group H, Group I, Group J, Group K, Group L, and/or Group M, as shown in Table 1. In alternative embodiments there may be at least one, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and at least 20 different members selected from any to each of members of Group A, Group B, Group C, Group D, Group E, Group F, Group G, Group H, Group I, Group J, Group K, Group L, and/or Group M, as shown in Table 1.

The invention also provides computer program products for implementing the computer implemented methods of the invention, and computers comprising the computer program products of the invention.

The invention provides therapeutic combination of drugs for an individual in need thereof comprising or consisting of: (a) a beta adrenergic receptor antagonist (a beta blocker), e.g., a propranolol or equivalent; (b) a non-steroidal anti-inflammatory drug (a NSAID), e.g., an etodolac or equivalent; and (c) a therapeutic agent for the treatment of cancer. In one embodiment, the non-steroidal anti-inflammatory drug (a NSAID) comprises etodolac, e.g., the etodolac can be LODINE™. In one embodiment, the beta adrenergic receptor antagonist (a beta blocker) comprises propranolol, e.g., the propranolol can be INDERAL™. In one embodiment, the beta adrenergic receptor antagonist (a beta blocker) comprises propranolol and the non-steroidal anti-inflammatory drug (a NSAID) comprises etodolac.

In alternative embodiments, the therapeutic agent for the treatment of cancer can comprise or consist of a monoclonal antibody, a peptide, a synthetic polypeptide or peptidomimetic, a nucleic acid, a synthetic nucleic acid, a lipid, a carbohydrate and/or a small molecule. The therapeutic agent for the treatment of cancer can comprise or consist of sorafenib (NEXAVAR™), sunitinib (e.g., SUTENT)™, erlotinib (e.g., TARCEVA™), imatinib (e.g., GLEEVEC™), lapatinib (e.g., TYKERB™), bevacizumab (e.g., AVASTIN™), trastuzumab (e.g., HERCEPTIN™), cetuximab (e.g., ERBITUX™), bevacizumab (e.g., AVASTIN™), BIBW 2992, gefitinib (e.g., IRESSA™), ranibizumab (e.g., LUCENTIS™), pegaptanib (e.g., MACUGEN™), dasatinib (e.g., BMS-354825™), sunitinib (e.g., SUTENT)™, pazopanib, nilotinib (e.g., TASIGNA™), panitumumab (e.g., VECTIBIX™), bandetinib, brivanib, E7080™ or a combination thereof.

In alternative embodiments, the therapeutic agent for the treatment of cancer can comprise or consist of a tyrosine kinase inhibitor or a serine/threonine kinase inhibitor; or a histone deacetylase inhibitor, e.g., comprising or consisting of a vorinostat (rINN) or ZOLINZA™, or suberoylanilide hydroxamic acid (SAHA).

In alternative embodiments, the therapeutic agent for the treatment of cancer can comprise or consist of an angiogenesis inhibitor, e.g., a vascular endothelial growth factor (VEGF)-mediated angiogenesis inhibitor.

In alternative embodiments, the therapeutic agent for the treatment of cancer can comprise or consist of an inducer of apoptosis or a mitotic and anti-microtubule inhibitor (inhibition of microtubule function), e.g., a raltitrexed (e.g., TOMUDEX™), doxorubicin (e.g., ADRIAMYCIN™), fluorouracil (e.g., 5-fluorouracil), paclitaxel (e.g., TAXOL™ or ABRAXANE™) docetaxel (e.g., TAXOTERE™), vinblastin, vindesine, vinorelbine (NAVELBINE™); an epothilone (e.g., epothilone A, B, C, D, E or F), ixabepilone (also known as azaepothilone B, e.g., BMS-247550™) or a combination thereof.

In alternative embodiments, the therapeutic agent for the treatment of cancer can comprise or consist of an alkylating agent, e.g., a cisplatin, cisplatinum or *cis-*diamminedichloridoplatinum(II) (CDDP), carboplatin, oxaloplatin, cyclophosphamide (cytophosphane) (e.g., ENDOXAN™, CYTOXAN™, NEOSAR™, REVIMMUNE™), mechlorethamine (chlormethine, mustine, nitrogen mustard), chlorambucil (e.g., LEUKERAN™) or a combination thereof.

In alternative embodiments, a topoisomerase inhibitor, e.g., an etoposide (e.g., EPOSIN™, ETOPOPHOS™, VEPESID™, VP-16™), amsacrine, topotecan (e.g., HYCAMTIN™), teniposide (e.g., VUMON™, VM-26™), an epipodophyllotoxin, a camptothecin, irinotecan (e.g., CAMPTOSAR™), or a combination thereof.

In alternative embodiments, the therapeutic agent for the treatment of cancer can comprise or consist of a glycopeptide antibiotic, e.g., a bleomycin (e.g., bleomycin A₂ or 8₂.), mitomycin (e.g., mitomycin C), plicamycin (also known as mithramycin; e.g., MITHRACIN™), or a combination thereof.

In alternative embodiments, the therapeutic agent for the treatment of cancer can comprise or consist of a steroid receptor inhibitor or steroid inhibitor (an anti-steroid), e.g., an estrogen receptor modulator (a SERM), such as a tamoxifen (e.g., NOLVADEX™, ISTUBAL™, VALODEX™); or, the steroid inhibitor or an anti-steroid can comprise or consist of a finasteride (e.g., PROSCAR™, PROPECIA™, FINCAR™, FINPECIA™, FINAX™, FINAST™, FINARA™, FINALO™, PROSTERIDE™, GEFINA™, APPECIA™, FINASTERID IVAX™, FINASTERID ALTERNOVA™).

In alternative embodiments, the therapeutic agent for the treatment of cancer can comprise or consist of (a) a matrix metalloproteinase (MMP) inhibitor; (b) an mTOR (mammalian target of rapamycin) inhibitor, or (c) an mTOR inhibitor comprising or consisting of a temsirolimus or equivalent, or TORISEL™.

In alternative embodiments, the beta adrenergic receptor antagonist (a beta blocker), the non-steroidal anti-inflammatory drug (a NSAID), and the therapeutic agent for the treatment of cancer are formulated, are all formulated as separate compositions; or, the beta adrenergic receptor antagonist (a beta blocker), e.g., propranolol or equivalent; the non-steroidal anti-inflammatory drug (a NSAID), e.g., an etodolac or equivalent; and the therapeutic agent for the treatment of cancer are formulated in the same composition (they are formulated together).

In alternative embodiments, the beta adrenergic receptor antagonist (a beta blocker), e.g., a propranolol or equivalent; the non-steroidal anti-inflammatory drug (a NSAID), e.g., an etodolac or equivalent; and the therapeutic agent for the treatment of cancer are packaged individually in a single package, a plurality of packages or packettes, or a blister packet, lidded blister or blister card or packets, or a shrink wrap.

In alternative embodiments, the beta adrenergic receptor antagonist (a beta blocker), e.g., propranolol or equivalent; the non-steroidal anti-inflammatory drug (a NSAID), e.g., an etodolac or equivalent; and the therapeutic agent for the treatment of cancer are packaged together in a single package, a plurality of packages or packettes, or a blister packet, lidded blister or blister card or packets, or a shrink wrap.

In alternative embodiments, the beta adrenergic receptor antagonist (a beta blocker), e.g., a propranolol or equivalent; the non-steroidal anti-inflammatory drug (a NSAID), e.g., an etodolac or equivalent; and the therapeutic agent for the treatment of cancer are packaged together in a single package, a plurality of packages or packettes, or a blister packet, lidded blister or blister card or packets, or a shrink wrap, and with both drugs released upon opening of the single package, plurality of packages or packettes, blister packet, lidded blister, blister card or packets or shrink wrap.

In alternative embodiments, the beta adrenergic receptor antagonist (a beta blocker), e.g., a propranolol or equivalent; the non-steroidal anti-inflammatory drug (a NSAID), e.g., an etodolac or equivalent; and the therapeutic agent for the treatment of cancer are packaged together in a single package, a plurality of packages or packettes, or a blister packet, lidded blister or blister card or packets, or a shrink wrap, and with both drugs are formulated as a tablet, a pill, a lozenge, a capsule, a caplet, a patch, a spray, an inhalant, a gel, a geltab, a nanosuspension, a nanoparticle, a microgel and/or a pellet, and the tablet, pill, lozenge, capsule, gel, geltab, nanosuspension, nanoparticle, microgel and/or a pellet are released upon opening of the single package, plurality of packages or packettes, blister packet, lidded blister, blister card or packet, or shrink wrap.

In alternative embodiments, the beta adrenergic receptor antagonist (a beta blocker), e.g., propranolol or equivalent; the non-steroidal anti-inflammatory drug (a NSAID), e.g., an etodolac or equivalent; and the therapeutic agent for the treatment of cancer are formulated or manufactured as a feed, a food, a pellet, a lozenge, a liquid, an elixir, an aerosol, an inhalant, a spray, a powder, a tablet, a pill, a capsule, a gel, a geltab, a nanosuspension, a nanoparticle, a microgel or a suppository. In alternative embodiments, the beta adrenergic receptor antagonist (a beta blocker), e.g., a propranolol or equivalent; the non-steroidal anti-inflammatory drug (a NSAID), e.g., an etodolac or equivalent; and the therapeutic agent for the treatment of cancer are packaged as a blister pack or plurality of blister packettes, or lidded blister or blister card or packets, or a shrink wrap.

In alternative embodiments, the dosage of etodolac ranges from about 200 mg to 400 mg a day, or, about 10, 15, 20, 25, 30, 35, 40, 45, 50, 75, 80, 85, 90, 100, 150, 200, 250, 300, 350, 400, 450, 500, 600, 700, 800, 900 or 1000 mg or more. In alternative embodiments, the dosage of propranolol ranges from 10 to 320 mg per day based on heart rate and blood pressure of the individual, or, about 10, 15, 20, 25, 30, 35, 40, 45, 50, 75, 80, 85, 90, 100, 150, 200, 250, 300, 350, 400, 450, 500, 600, 700, 800, 900 or 1000 mg or more.

In alternative embodiments, the beta adrenergic receptor antagonist (a beta blocker), e.g., a propranolol or equivalent; the non-steroidal anti-inflammatory drug (a NSAID), e.g., an etodolac or equivalent; and the therapeutic agent for the treatment of cancer are packaged in dosages that match a chrono-dosing regimen to match an optimal dose for the time of day. In alternative embodiments, the beta adrenergic receptor antagonist (a beta blocker), e.g., a propranolol or equivalent; the non-steroidal anti-inflammatory drug (a NSAID), e.g., an etodolac or equivalent; and the therapeutic agent for the treatment of cancer are packaged in dosages that match a chrono-dosing regimen comprising: (a) in the AM, 20 mg propranolol, 200mg etodolac; in the afternoon, 10 mg propranolol, 200 mg etodolac; in the PM, 10 mg propranolol, 400 mg etodolac; (b) in the AM 40 mg propranolol, 200 mg etodolac; in the afternoon 20 mg propranolol, 200 mg etodolac; in the evening, 20 mg propranolol, 400 mg etodolac; (c) in the AM 80 mg propranolol, 200 mg etodolac; in the afternoon 40 mg propranolol, 200 mg etodolac, in the evening 40 mg, etodolac; or (d) a dose escalation comprising a regimen of (a) to (b) to (c). In alternative embodiments, the beta adrenergic receptor antagonist (a beta blocker), e.g., a propranolol or equivalent; the non-steroidal anti-inflammatory drug (a NSAID), e.g., an etodolac or equivalent; and the therapeutic agent for the treatment of cancer are packaged in dosages that match a chrono-dosing regimen comprising: Start: AM, 20 mg propranolol, 200mg etodolac; afternoon, 10 mg propranolol, 200 mg etodolac; PM 5 mg propranolol, 400 mg etodolac; Dose Escalation 1: AM 40 mg propranolol, 200 mg etodolac; afternoon 20 mg propranolol, 200 mg etodolac; evening, 10 mg propranolol, 400 mg etodolac; Dose escalation 2: AM 80 mg propranolol, 200 mg etodolac; afternoon 40 mg propranolol, 200 mg etodolac, evening 20 mg, etodolac.

In alternative embodiments, the beta adrenergic receptor antagonist (a beta blocker), e.g., a propranolol or equivalent; the non-steroidal anti-inflammatory drug (a NSAID), e.g., an etodolac or equivalent; and the therapeutic agent for the treatment of cancer are formulated for administration once a day, b.i.d. or t.i.d, or weekly, or biweekly, or monthly.

In alternative embodiments, the beta adrenergic receptor antagonist (a beta blocker), e.g., a propranolol or equivalent; the non-steroidal anti-inflammatory drug (a NSAID), e.g., an etodolac or equivalent; and the therapeutic agent for the treatment of cancer are formulated for administration intravenously, topically, orally, by inhalation, by infusion, by injection, by inhalation, intraperitoneally, intramuscularly, subcutaneously, intra-aurally, for intra-articular administration, for intra-mammary administration, for topical administration or for absorption through epithelial or mucocutaneous linings.

In alternative embodiments, the therapeutic combination of the invention further comprises instructions for use in the treatment of cancer, cachexia, cancer cachexia, mucositis and/or anorexia. The cachexia can be defined as at least two of the symptoms selected from the group consisting of: 1) a hyper-inflammatory state, 2) altered hormone levels and cytokine levels; 3) decreased heart rate variability; 4) weight loss, and 5) increased heart rate, wherein optionally the increased heart rate is having a sustained elevated heart rate of at least about 6 bpm. The cachexia can be defined by an individual having at least a sustained elevated heart rate of at least about 6 bpm and weight loss.

The invention provides pharmaceutical compositions or formulations comprising the therapeutic combination of the invention. The pharmaceutical compositions or formulations can further comprise a pharmaceutically acceptable excipient. The pharmaceutical composition or formulation can be formulated or manufactured as a feed, a food, a food or feed concentrate, a pellet, a lozenge, a liquid, a lotion, an implant, a nanoparticle, an elixir, an aerosol, a spray, an aerosol, an inhalant, a powder, a tablet, a pill, a capsule, a gel, a geltab, a nanosuspension, a nanoparticle, a patch, a microgel or a suppository.

The invention provides uses of the therapeutic combination of the invention in the manufacture of a medicament or pharmaceutical composition for treating, ameliorating or preventing a trauma, condition or disease comprising: a cancer or dysfunctional cell condition; any side effect from the treatment of cancer, chronic Systemic Inflammatory Response State (SIRS); chronic systemic inflammatory stress; burns, chronic obstructive pulmonary disease; congestive heart failure; chronic kidney disease; surgery; sepsis; ageing; acute respiratory distress syndrome; acute lung injury; infection; a CNS disorder or injury; anemia; immunosuppression; insulin resistance; anorexia; anxiety; sleep disturbances; weakness; fatigue; gastrointestinal distress; sleep disturbances; wake disturbances; pain; listlessness; shortness of breath; lethargy; depression; malaise; or, a combination thereof. The trauma, condition or disease can comprise a maladaptive nutritional state secondary to the SIRS, or the maladaptive nutritional state comprises cachexia, and optionally the cachexia comprises cachexia secondary to cancer. The cancer or dysfunctional cell condition can comprise (be) any metastatic or benign tumor, and the use is for treating (killing, eliminating, stopping the growth and/or metastasis of) cancer stem cells or cancer cells from: lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma or pituitary adenoma, and any combination thereof. The cachexia can be defined as at least two of the symptoms selected from the group consisting of: 1) a hyper-inflammatory state, 2) altered hormone levels and cytokine levels; 3) decreased heart rate variability; 4) weight loss, and 5) increased heart rate, wherein optionally the increased heart rate is having a sustained elevated heart rate of at least about 6 bpm. The cachexia can be defined by an individual having at least a sustained elevated heart rate of at least about 6 bpm and weight loss. The CNS disorder can comprise (be) Parkinson's disease or Alzheimer's disease.

The invention provides methods for treating or ameliorating a trauma, condition or disease comprising a cancer or dysfunctional cell condition mucositis, any side effect from the treatment of a cancer or dysfunctional cell condition, chronic Systemic Inflammatory Response State (SIRS), chronic systemic inflammatory stress; burns, chronic obstructive pulmonary disease; congestive heart failure; chronic kidney disease; surgery; cancer; sepsis; ageing; acute respiratory distress syndrome; acute lung injury; infection; a CNS disorder or injury; anemia; immunosuppression; insulin resistance; anorexia; anxiety; sleep disturbances; weakness; fatigue; gastrointestinal distress; sleep disturbances; wake disturbances; pain; listlessness; shortness of breath; lethargy; depression; malaise; or, a combination thereof, the method comprising the steps of:
(a) providing the therapeutic combination of the invention, or the pharmaceutical composition or formulation of the invention; and,
(b) administering a therapeutically effective amount of the therapeutic combination or the pharmaceutical composition or formulation of step (a), thereby treating or ameliorating the side effect, trauma, condition or disease.

In alternative embodiments of the methods, the condition or disease comprises a maladaptive nutritional state secondary to the SIRS; or, the maladaptive nutritional state comprises cachexia or anorexia, or a cachexia secondary to cancer. The cancer or dysfunctional cell condition can comprises (be) any metastatic or benign tumor, and the use is for treating (killing, eliminating, stopping the growth and/or metastasis of) cancer stem cells or cancer cells from: lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma or pituitary adenoma, and any combination thereof.

In alternative embodiments of the methods, the cachexia is defined as at least two of the symptoms selected from the group consisting of: 1) a hyper-inflammatory state, 2) altered hormone levels and cytokine levels; 3) decreased heart rate variability; 4) weight loss, and 5) increased heart rate, wherein optionally the increased heart rate is having a sustained elevated heart rate of at least about 6 bpm; or, the cachexia is defined by an individual having at least a sustained elevated heart rate of at least about 6 bpm and weight loss.

The invention provides compositions or products of manufacture comprising the therapeutic combination of the invention, or the pharmaceutical composition or formulation of the invention. The composition or product of manufacture can comprise (can be formulated, manufactured or fabricated as) a blister pack, clamshell or tray, wherein the therapeutic combination is formulated for unit dosage administration to an individual in need thereof at the same time, and each unit dosage is contained within one blister in the blister pack, or compartment in the clamshell or tray; or, can comprise (can be formulated, manufactured or fabricated as) a pill, capsule, tablet, tab, geltab or implant, wherein the therapeutic combination is formulated for unit dosage administration to an individual in need thereof at the same time, and each unit dosage is contained within one pill, capsule, tablet, tab, geltab or implant.

The invention provides nanoparticles, microparticles or liposomes comprising the therapeutic combination of the invention, or the pharmaceutical composition or formulation of the invention.

The invention provides kits comprising the therapeutic combination of the invention, or the pharmaceutical composition or formulation of the invention. In alternative embodiments, the kits comprise at least one blister pack, lidded blister or blister card or packets, or a shrink wrap, comprising the therapeutic combination or the pharmaceutical composition. The invention provides kits for the treatment, amelioration or prevention of a cancer or any dysfunctional cell condition, a side effect from the treatment of a cancer or dysfunctional cell condition, a chronic Systemic Inflammatory Response State (SIRS) or a maladaptive nutritional state in a patient population, the kit comprising the therapeutic combination of the invention, or the pharmaceutical composition or formulation of the invention, and instructions for use of the therapeutic combination or pharmaceutical composition. The maladaptive nutritional state can comprise cachexia, and optionally the cachexia comprises cachexia secondary to cancer. The cachexia can be defined: (a) as having (being associated with) at least two of the symptoms selected from the group consisting of: 1) a hyper-inflammatory state, 2) altered hormone levels and/or cytokine levels; 3) decreased heart rate variability; 4) weight loss, and 5) sustained increased heart rate, wherein optionally the sustained increased heart rate is having a sustained elevated heart rate of at least about 6 bpm; or (b) by an individual having at least a sustained elevated heart rate of at least about 6 bpm and weight loss. The cancer or dysfunctional cell condition can comprise (be) any metastatic or benign tumor, and the use is for treating (killing, eliminating, stopping the growth and/or metastasis of) cancer stem cells or cancer cells from: lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma or pituitary adenoma, and any combination thereof.

The invention provides products of manufacture comprising a blister package, a lidded blister or a blister card or packet, a clamshell, a tray or a shrink wrap comprising the therapeutic combination of the invention, or the pharmaceutical composition or formulation of the invention. The invention provides products of manufacture comprising a blister package, a lidded blister or a blister card or packet, a clamshell, a tray or a shrink wrap comprising the therapeutic combination of the invention, or the pharmaceutical composition or formulation of the invention, wherein the therapeutic combination or pharmaceutical composition or formulation are manufactured and/or formulated for at least two dosage administrations. The therapeutic combination or pharmaceutical composition or formulation can be formulated as one dosage administration in the morning and one dosage administration in the evening. The dosage schedule can provide a relatively higher dose of one drug in the morning (the AM) than in the evening, and a relatively higher dose of another medication in the evening than in the morning.

The invention provides products of manufacture or formulations comprising the therapeutic combination of the invention, or the pharmaceutical composition or formulation of the invention, and a nutritional supplement.

The invention provides methods for treating, ameliorating or preventing a cachexia and/or a chronic Systemic Inflammatory Response State (SIRS), wherein the therapeutic combination of the invention, or the pharmaceutical composition or formulation of the invention, is administered to an individual in need thereof, and the therapeutic combination or the pharmaceutical composition are formulated for at least two administrations, one in the morning and one in the evening, wherein the dosage schedule provides a relatively higher dose of beta blocker in the morning (the AM) than in the evening, and a relatively higher dose of an anti-inflammatory medication in the evening than in the morning. The administration regimen can comprise at least two dosages of beta adrenergic receptor antagonist (a beta blocker) drug and at least two dosages of non-steroidal anti-inflammatory drug (a NSAID), and further comprising administration of an anti-anxiety drug, and the drugs are organized or labeled in a blister package, a lidded blister or a blister card or packet, a clamshell, a tray or a shrink wrap for usage by an individual for at least two administrations, one in the morning and one in the evening, wherein the dosage schedule provides a relatively higher dose of beta blocker in the morning (the AM) than in the evening, and a relatively higher dose of an anti-anxiety and/or an anti-inflammatory medication in the evening than in the morning. The administration regimen can comprise doses of beta adrenergic receptor antagonist (e.g., propranolol) given in 20 or 40 mg tablets immediate release on a bid basis, and in the first dose week the doses for beta adrenergic receptor antagonist are 20 mg in the morning and 20 mg at bedtime, and after 1 week the dosage is adjusted to 20 mg of the immediate release product in the morning and 60 mg of the extended release at bedtime, and optionally if after an additional week the subject shows no improvement or has not obtained a 20% reduction in heart rate, without decreasing heart rate below 60 bpm or blood pressure below 90/60, the dose is adjusted to 40 mg of the immediate release propranolol in the morning and 120 mg of the extended release beta adrenergic receptor antagonist at bedtime. The administration regimen can comprise doses of non-steroidal anti-inflammatory drug (a NSAID) (e.g., etodolac) given in 200 mg capsules or 500 mg tablets on a bid basis, and doses for the NSAID (e.g., etodolac) are started at 200 mg in the morning and at bedtime, and after 1 week the dosage are adjusted to 200 mg in the morning and 500 mg at bedtime.

The invention provides blister packs or a plurality of blister packettes, a blister package, a lidded blister or a blister card or packet, a clamshell, a tray or a shrink wrap, comprising the therapeutic combination of the invention, or the pharmaceutical composition or formulation of the invention, wherein the beta adrenergic receptor antagonist (e.g., propranolol or equivalent), NSAID (e.g., etodolac or equivalent) and the therapeutic agent for the treatment of cancer are arranged or clustered in the blister pack or a plurality of blister packettes: (a) in a chrono-dosing arrangement or pattern; or (b) individually.

The invention provides papers, plastics or cellophane packages or a plurality of packettes comprising the therapeutic combination of the invention, or the pharmaceutical composition or formulation of the invention, wherein the beta adrenergic receptor antagonist (e.g., propranolol or equivalent), NSAID (e.g., etodolac or equivalent) and the therapeutic agent for the treatment of cancer are arranged or clustered in the paper, plastic or cellophane package or a plurality of packettes: (a) in a chrono-dosing arrangement or pattern; or (b) individually.

In alternative embodiments, the blister pack or a plurality of blister packettes, a blister packages, a lidded blisters or blister cards or packets, clamshells, trays or shrink wraps of the invention, or the paper, plastic or cellophane package or a plurality of packettes of the invention, can be formulated or designed for drug regimen compliance of a cancer patient population, a pediatric or geriatric population, or a mentally compromised patient population, which in various embodiments includes patients having mild or severe mental retardation, slow cognition, dementia, senility, Alzheimer's disease, traumatic brain injury, chemical brain damage, mental diseases (e.g., dissociative disorder, obsessive-compulsive disorder, delusional disorder, schizophrenia, mania, panic disorder, depression, dyslexia, any learning disability and the like) post-traumatic stress disorder, traumatic war neurosis, post-traumatic stress syndrome (PTSS), physical disability (e.g., blindness).

The invention provides foods or food supplements comprising the therapeutic combination of the invention, or the pharmaceutical composition or formulation of the invention.

The invention provides uses of the therapeutic combination of the invention; the pharmaceutical composition or formulation of the invention; the composition or product of manufacture of the invention; the nanoparticle, microparticle, nanoliposome or liposome of the invention; at least one composition or product of manufacture of the invention; or a blister pack or a plurality of blister packettes, a blister package, a lidded blister or a blister card or packet, a clamshell, a tray or a shrink wrap of the invention, or the paper, plastic or cellophane package or a plurality of packettes of the invention, the kit of the invention; for making a deliverable package for increasing drug regimen compliance of a stressed, challenged or non-compliant patient population. In one embodiment of the uses, the stressed, challenged or non-compliant patient population comprises a cancer patient population; or a patient population having mild or severe mental retardation, slow cognition, dementia, senility, Alzheimer's disease, traumatic brain injury, chemical brain damage or a mental disease; or post-traumatic stress disorder, traumatic war neurosis, post-traumatic stress syndrome (PTSS) or a physical disability or blindness. In one embodiment of the uses, the mental disease comprises a dissociative disorder, an obsessive-compulsive disorder, a delusional disorder, a schizophrenia, a mania, a panic disorder, depression, dyslexia or a learning disability.

The details of one or more aspects of the invention are set forth in the description below. Other features, objects, and advantages of the invention will be apparent from the description and from the claims.

All publications, patents and patent applications cited herein are hereby expressly incorporated by reference for all purposes.

### DETAILED DESCRIPTION

In alternative embodiments, this invention provides cancer and mucositis therapies, and cytoprotection products and methods, for use either alone or in combination with other medical therapies, such as cancer chemotherapies and radiation therapies for e.g., cancer. The invention provides compositions and methods for treating, ameliorating (e.g., delaying the onset of or diminishing the severity of) or preventing oral mucositis; digestive mucositis; esophageal mucositis; intestinal mucositis; or any side effect of a cancer chemotherapy or radiation therapy for e.g., cancer.

In alternative embodiments the invention provides compositions, e.g., formulations and products of manufacture, formulated or designed for drug regimen compliance of a "challenged" patient population, e.g., a population of patients where drug regimen compliance is difficult, e.g., a stressed population such as a cancer patient population, a pediatric or geriatric population, or a mentally compromised patient population; or a patient population having mild or severe mental retardation, slow cognition, dementia, senility, Alzheimer's disease, traumatic brain injury, chemical brain damage, a mental disease, e.g., a dissociative disorder, an obsessive-compulsive disorder, delusional disorder, schizophrenia, mania, panic disorder, depression, dyslexia, any learning disability and the like; or a patient population having post-traumatic stress disorder, traumatic war neurosis, post-traumatic stress syndrome (PTSS), physical disability, e.g., blindness and the like.

In one embodiment, the invention provides compositions and methods for using them as a cytoprotection product, e.g., as a product that provides cytoprotection. In one embodiment, the invention provides compositions and methods that are cytoprotection therapies, e.g., as compositions and therapies that provide cytoprotection.

In alternative embodiments, the invention provides compositions and methods that provide cytoprotection, includes providing or aiding in providing an acquired cellular tolerance to a normally lethal stress or toxicant by pre-exposure to a sub-lethal amount of the same or a different stress or toxicant (cross-tolerance). In one exemplification the successful manifestation of cytoprotection may be detected in a targeted cell or tissue by the appearance of molecules called "heat shock proteins" (HSPs) and/or other related molecules.

In one embodiment, the invention provides compositions for use with medical treatments associated with or causing one or more detrimental or unwanted side effects, e.g., cancer chemotherapy or radiation therapy, or any drug therapy.

An exemplary therapy of this invention is a combination of ingredients that may be administered (a) prior to a cancer therapy, a radiation therapy and/or a drug therapy, (b) at about the same time or during a cancer therapy, a radiation therapy and/or a drug therapy, (c) after a cancer therapy, a radiation therapy and/or a drug therapy, or (d) any combination thereof, for treating, preventing, ameliorating or abating an unwanted side effect; wherein the cancer therapy, a radiation therapy and/or a drug therapy causes the unwanted side effect(s); for example, the compositions and/or therapies (methods) of this invention are used for treating, preventing, ameliorating or abating a drug-induced, a chemotherapy-induced, and/or a radiation therapy-induced mucositis, including oral or a digestive mucositis.

In one embodiment, compositions and therapies (methods) of this invention also provide cytoprotection, e.g., as a myelo-protection therapy. An exemplary therapy of this invention can be used with a cancer therapy that causes unwanted side effects, such as a drug therapy, a chemotherapy and/or a radiation therapy, e.g., that causes mucositis, including an oral mucositis, digestive mucositis, esophageal mucositis and/or intestinal mucositis. In alternative embodiments, compositions and therapies (methods) of this invention are used for treating, preventing, ameliorating or abating any mucositis, including an oral mucositis, digestive mucositis, esophageal mucositis and/or intestinal mucositis.

The invention provides compositions and methods for treating, preventing or ameliorating an unwanted side effect or disease state symptom, e.g., the invention provides compositions and methods to abolish, to ameliorate, to diminish, to improve, and/or to inhibit, the unwanted side effects of a drug therapy, a chemotherapy and/or a radiation therapy, e.g., wherein the unwanted side effect is a mucositis, including an oral mucositis, digestive mucositis, esophageal mucositis and/or intestinal mucositis.

In alternative embodiments, the invention provides combinations of active agents (ingredients) comprising one or a plurality of (multiple) member, including only one member, or two members, three members, four members, five members, six members, or more; e.g., a composition comprising a combination of between about two and twenty or more members, i.e., active agents, such as a drug or a treatment composition.

In alternative embodiments, the invention provides therapies, which can comprise us of a composition, a method, a product, or any tool that is serviceable for that particular therapy of the invention, e.g. a therapy that is serviceable for treating ("to treat") a patient.

In alternative embodiments, the invention provides therapies for use in any individual, which can include the animals (e.g., farm animals, zoo or research animals, or domestics, e.g., dogs, cats).

While the invention not directed to any particular mechanism of action, compositions and methods of the invention for treating a mucositis, e.g., an oral or a digestive mucositis and related maladies and conditions, are designed to treat key stages of the mucositis by identifying pathways activated in the disease process. Imbalances of flux that are outside adaptive ranges of the tissue are addressed by compositions and methods of this invention; thus, the invention provides the appropriate therapy to restore flux to within the cells' adaptive ranges to treat the disease or condition, for example, to delay or prevent mucositis (e.g., an oral mucositis, digestive mucositis, esophageal mucositis and/or intestinal mucositis) onset and reduce its severity.

While the invention not directed to any particular mechanism of action, compositions and methods of the invention manipulate and/or augment or enhance the normal cycle of wound healing for a cell and/or tissue compartment that suffers an injury or an unwanted side effect, e.g., as a mucositis, e.g., an oral or a digestive mucositis. In one embodiment, the invention identifies when, where and how to intervene to treat a disease or condition, e.g., a mucositis (e.g., an oral mucositis, digestive mucositis, esophageal mucositis and/or intestinal mucositis); for example, the invention identifies what the specific macro fluxes (tissue or cellular) and micro fluxes (protein/DNA/other molecules) are to place a cell or tissue in an adaptive, or healing, range - particularly when it is outside an adaptive or healing range, e.g., because of the injury, disease or condition. This invention provides drug combinations to attenuate the signals and cellular environmental conditions that are outside the cells' or the tissues' adaptive range to lead a benign range of signals and cellular environmental conditions needed for a healing, remodeling response.

The compositions (e.g., drug combinations) and methods of this invention treat and ameliorate disease and injury by understanding and directly addressing the clinical manifestations of disease pathology and when the molecular signals of damage and inflammation exceed the capacity of a cell or a tissue. While the invention not directed to any particular mechanism of action, compositions and methods of the invention are a therapeutic intervention that does not interfere with the normal wound healing process, but rather prevents or reduces damage.

Initiation and Ulcer Formation. Compositions and methods of the invention can treat, ameliorate (which includes delaying the onset or severity of) or prevent adverse side effects of a medical therapy, e.g., a cancer therapy, that are commonly observed in the oral cavity, e.g., changes in the epithelium as a result of damage to proliferating and differentiating cells and signals associated with the insult. While the invention not directed to any particular mechanism of action, in one embodiment compositions and methods of the invention act to treat, ameliorate (which includes delaying the onset or severity of) or prevent pathology in the more rapidly proliferating tissues where atrophy and ulceration can represent a dose-limiting and potentially serious complication of treatment; e.g., damage to normally rapidly proliferating tissues by a drug, e.g., a cancer drug, many times requires limitation on the dosage of that drug, e.g., the cancer drug.

Compositions and methods of the invention can treat, ameliorate (which includes delaying the onset or severity of) or prevent the lesions first observed on the soft palate, tongue, and cheeks caused by a drug or a medical treatment, e.g., a chemotherapy or radiotherapy, e.g., for a cancer. Compositions and methods of the invention can treat, ameliorate or prevent these lesions and prevent or delay their forming or their enlargement, and thus decrease associated pain and dysphagia (e.g. difficulty with swallowing or inability to swallow, often associated with pain), and coincident dehydration, poor nutritional status (because of painful chewing), and a decreased quality of life.

Compositions and methods of the invention can be administered with, or for a prophylactic effect, before, or after, any medical therapy, e.g., a chemotherapy and/or a radiation therapy, e.g., a medical therapy which can lower cell proliferation and turnover in connective tissue. Compositions and methods of the invention can be used to ameliorate or prevent the harmful effects of ionizing radiation (from radiation therapy), including tissue matrix damage, which causes increased vascular permeability, tissue edema, and an infiltration of inflammatory cells; the invention can be practiced before, during and/or after the therapy. Compositions and methods of the invention can be used to ameliorate damage to fibroblasts, which can result in cell loss and fibrosis, and ameliorate damage to blood vessels, which can lead to hypovascularity and tissue ischemia. Together, these changes will reduce the ability of the tissue to heal and resist infection; thus, practicing the compositions and methods of the invention will increase the ability of tissue to heal and resist infection. Indirect effects of radiation therapy, such as damage to the salivary glands, which will reduce salivary production and impair barrier efficiency, resulting in a reduction in immunocompetence (especially when associated with a myeloablative therapy, e.g. a therapy that severely damages a patient's bone marrow function and ability to make blood cells), also can be ameliorated using the compositions and methods of the invention. Because this damage will increase the risk of local infection from oral organisms; thus, practicing the compositions and methods of the invention will increase the ability to resist or recover from local, pathological infection from oral organisms, including viruses, yeast, fungi and/or bacteria.

Compositions and methods of the invention can be used to decrease both DNA and non-DNA (e.g., cellular or tissue) damage, which occur almost immediately after exposure to a medical therapy, e.g., radiation or chemotherapy, or exposure to a poison or radiation (e.g., as an accident, a workplace exposure or in warfare or terrorist attacks). Compositions and methods of the invention can be used to decrease DNA strand breaks in the epithelium as well as in the cells of the submucosa, thus preventing cells from dying, or from dying quickly, and preventing or ameliorating a cascade of biological events that occurs as a sequelae to radiation exposure or to chemotherapy.

Compositions and methods of the invention can be used to decrease events mediated by the generation of reactive oxygen species (ROS), which occurs shortly after radiation exposure or chemotherapy. ROS have a far-ranging and broad biological downstream impact; thus, practicing the compositions and methods of the invention will decrease or eliminate these negative effects. Compositions and methods of the invention can be used to decrease the harmful effects of ROS, which can effectively activate a number of central biological control mechanisms, including a select group of transcription factors, pro-inflammatory cytokines and adhesion molecules. Compositions and methods of the invention can be used to decrease or dampen the self-amplification of this process that results in induction of other effector proteins and tissue injury. Compositions and methods of the invention can be used to decrease the tissue damage that leads to activation of additional transcription factors, each being associated with an expression of genes and their biologically active proteins that have a harmful effect.

Thus, in some embodiment, the compositions and methods of the invention augment, mimic or reproduce "normal" responses to injury, allowing downstream elements in the injury response (e.g., wound healing) process to proceed normally, or closer to normal. Therefore, in alternative embodiments, compositions and methods of the invention provide an effective therapy for reducing the impact of an injury, whether that injury be from drug administration (including chemotherapy), toxin exposure (including allergens or poisons), radiation administration or radiation exposure (including workplace exposure), or trauma; thus attenuating the response to that injury and not negatively impacting the wound healing process.

Wound Healing. Compositions and methods of the invention are used to treat wounds, whether that wound be from drug administration (including chemotherapy), toxin exposure (including allergens or poisons), radiation administration or radiation exposure, workplace exposure or trauma. In alternative embodiments, compositions and methods of the invention are used to mimic or reproduce an acute wound healing process, e.g., reproduce the specific manner or approximate a wound healing process; which can be characterized by four distinct, but overlapping phases: 1/ hemostasis; 2/ inflammation; 3/ proliferation (3a/ granulation and 3b/ tissue restitution); and 4/ remodeling, as described below. Compositions and methods of the invention can augment, trigger, or replace, the specific biological markers that characterize the healing of acute wounds, or wounds or injuries from drug administration (including chemotherapy), toxin exposure (including allergens or poisons), radiation administration or radiation exposure, or trauma. In one embodiment, compositions and methods of the invention can ameliorate or decrease the biologic markers that characterize pathologic responses that result in fibrosis and chronic non-healing ulcers.

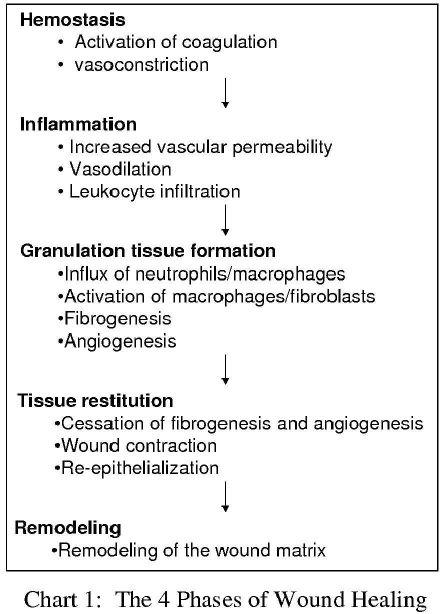

In one embodiment, compositions and methods of the invention are used to augment or initiate, or mimic, a normal healing response, which can begin the moment the tissue is injured. For example, compositions and methods of the invention are used to augment or initiate, or mimic the injury healing response where blood components enter the site of injury, and platelets come into contact with exposed collagen and other elements of the extracellular matrix. The platelet contact triggers the platelets to release clotting factors (that promote hemostasis) as well as essential growth factors and cytokines such as platelet-derived growth factor (PDGF) and transforming growth factor beta (TNF-β). Following *hemostasis*, the neutrophils then enter the wound site and begin the task of phagocytosis to remove foreign materials, bacteria and damaged tissue, and these processes promote an inflammation phase. As part of this *inflammatory* phase, the macrophages appear and continue the process of phagocytosis as well as releasing more PDGF and TGF-ß. Once the wound site is cleaned out, fibroblasts migrate in to begin a *proliferative* phase and deposit new extracellular matrix. The new collagen matrix then becomes cross-linked and organized during the final *remodeling* phase. Compositions and methods of the invention are used to augment or initiate, or mimic, this efficient and highly controlled repair process.

Although the oral mucosa heals faster than skin does and is more resistant to initial damage, compositions and methods of the invention can be used to augment this process and to ameliorate the tissue injury caused by radiotherapy, e.g., fractionated radiotherapy, and many types of chemotherapy, most of which are sufficient to cause wound formation. Compositions and methods of the invention can be used to decrease the injury seen in a mucositis, e.g., an oral or a digestive mucositis, and/or to augment or initiate the healing process, e.g., as illustrated in the five steps of the traditional pathobiology model of mucositis, see Chart 2, below.

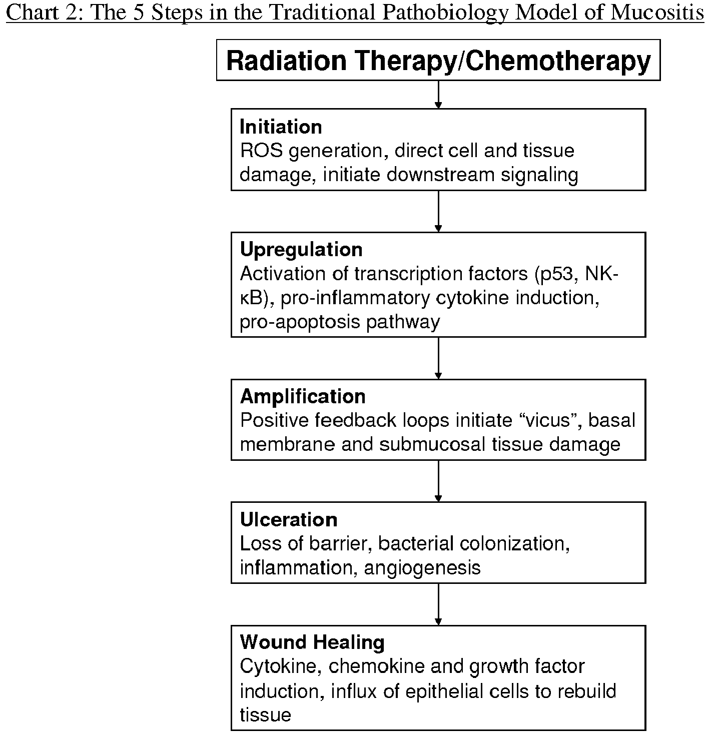

Compositions and methods of the invention can be used with or in place of existing mucositis treatment products, e.g., to augment a product or a method of treatment, including e.g. any products already approved and available for mucositis, such as an oral or a digestive mucositis, treatment or prevention, including e.g. the following.
1. Palifermin (e.g., KEPIVANCE™), a recombinant protein mimic of Keratinocyte Growth Factor (KGF), may impact prevention, upregulation/amplification and facilitate the start of normal healing.
2. Amifostine (commonly known as WR-1065 in its active form) (e.g., ETHYOL™), which scavenges oxygen free-radicals that are produced by ionizing radiation.
3. Products that target the mucositis and are administered locally, thereby eliminating side-effects from systemic delivery. These products include the tetracycline class of antibiotics which reduce inflammation and promote wound healing (similar to KGF), and food additives, antioxidants and gels/barrier to reduce mechanical trauma and infiltration of bacteria. These interventions administered alone can be less effective because they are typically applied after the initiating event and amplification stage.

Compositions and methods of the invention can be used with or in place of other known pharmacological treatments for mucositis, such as an oral or a digestive mucositis; including non steroidal anti-inflammatory drugs (NSAIDs), angiotensin converting enzyme inhibitors (ACE inhibitors), angiotensin receptor blockers (ARBs), pentoxifylline, deferrioxamine, polyunsaturated fatty acids, α-difluoromethylornithine, and superoxide dismutase.

For example, compositions and methods of the invention can be used with NSAIDS to augment their anti-inflammatory effect in the prophylaxis or treatment of radiation-induced normal tissue injury. In some aspects, use of compositions and methods of the invention can mitigate the injury caused by some anti-inflammatory agents in some organs.

Compositions and methods of the invention also can be used with ACE inhibitors or ARBs for the effective treatment and prophylaxis of radiation nephropathy. Compositions and methods of the invention also can be used with ACE inhibitors or ARBs for a prophylaxis effect to reduce pulmonary fibrosis and block radiation-induced rises in pulmonary arterial pressure. Compositions and methods of the invention also can be used with ACE inhibitors or ARBs for prophylaxis of radiation pneumonitis.

Compositions and methods of the invention also can be used with pentoxifylline for all types of radiation injuries, including for the treatment of radiation-induced fibrosis and radiation-induced soft-tissue necrosis.

Compositions and methods of the invention also can be used with deferrioxamine: the iron chelator, deferrioxamine, has shown efficacy against experimental radiation induced spinal cord injury when combined with a low-iron diet.

Compositions and methods of the invention also can be used with polyunsaturated fatty acids to reduce radiation- induced skin and spinal cord injury in animals and humans.

Compositions and methods of the invention also can be used with α-difluoromethylornithine, a polyamine-synthesis inhibitor for, e.g., reducing brain injury after cranial irradiation.

Compositions and methods of the invention also can be used with superoxide dismutase (SOD) to treat radiation-induced fibrosis.

Compositions and methods of the invention can be used in wound generation and healing, and as an adjunctive treatment to minimize ulcer formation.

While the invention is not limited by any particular mechanism of action, compositions and methods of the invention can be used to treat mucositis, such as an oral or a digestive mucositis, by affecting at least five relevant points of intervention:
1. Compositions and methods of the invention can be used to prevent initiation of the damage response state - by e.g., increasing the tissue compartment's capacity to tolerate radiation and chemotherapy-induced damage, (includes the endothelial, fibroblast, epithelial as well as tissue resident macrophages, stem cells etc.).
2. Compositions and methods of the invention can be used attenuate the inflammatory response and minimize damage response state of adjacent tissues - by reducing the influx of inflammatory cells and the degranulation and creation of reactive oxygen species to within the tissue compartment's ability to clear. This protective step attenuates the buildup of damaging breakdown products and inflammatory signaling that may lead to further inflammation outside the adaptive range.
3. Compositions and methods of the invention can be used provide appropriate signaling during the damage response state to facilitate healthy wound healing - once the cycle has been initiated, appropriate normal processes need to be activated and extrinsic damaging signaling needs to be offset. For example, use of corticosteroids alone might undermine normal wound healing process and would be contraindicated.
4. Compositions and methods of the invention can be used to prevent or decrease tertiary damage or interference with wound healing - to prevent or treat any secondary infection or further trauma to the tissue compartment that may increase damage or block healthy healing response.
5. Compositions and methods of the invention can be used to provide or augment an appropriate nutrient and healthy body response - including providing a nutrient and an oxygen rich environment, and circulating and tissue resident stem cells, immune cells, systemic hormones that sustain a healthy wound healing cycle.

Compositions and methods of the invention can be used to treat and/or ameliorate other diseases that may be caused by different assaults but which cause the same imbalances of flux and therefore result in similar pathology as a mucositis, such as an oral or a digestive mucositis. Compositions and methods of the invention can be used to lesson or abrogate the primary drivers of an eventual lesion, are signals generated directly or indirectly by DNA and cell damage. For example, compositions and methods of the invention can be used to treat or ameliorate ischemia/reperfusion (I/R) injury. Although the cause of tissue injury in I/R is very different from mucositis caused by chemo- or radiotherapy, the underlying disease progression is highly similar. Therefore, compositions and methods of the invention effective for mucositis also can be used in I/R protection, which is a complex pathophysiological event associated with significant impairment of multiple vascular and cellular responses. Compositions and methods of the invention can be used to lesson or abrogate oxidative damage due to the presence of radical oxygen species, which is an essential step that initiates a wide range of intracellular stress signaling processes that culminate in excessive cytokine and chemokine response, adhesion molecule upregulation and nitric oxide overproduction. Compositions and methods of the invention can be used to lesson or abrogate gut ischemia, which is frequently encountered in trauma, shock, cardiovascular surgery, and organ transplantation has the same pathophysiology.

In one embodiment, the invention provides a treatment for mucositis, such as an oral or a digestive mucositis, comprising a combination of geranyl geranyl acetone (GGA) and analogs of GGA, or teprenone (CAS Registry Number 6809-52-5) (SELBEX™), and captopril (e.g., CAPOTIN™), e.g., an exemplary combination in the form of Vicus Therapeutics (Morristown NJ) product VT-211™.

In one embodiment, the invention provides a treatment for mucositis, such as an oral or a digestive mucositis, comprising a combination of: a drug approved for GI indications, such asteprenone or teprenone (SELBEX™) or geranyl geranyl acetone (GGA) and analogs of GGA; and, the second drug approved for use to treat inflammation, e.g., the NSAID etodolac (e.g., LODINE™). In one aspect, this drug combination is provided in the form of Vicus Therapeutics (Morristown NJ) product VT-212™ (i.e., a formulation comprising active agents consisting of NSAID etodolac and geranyl geranyl acetone (GGA)).

In one aspect, the drug combinations of this invention, e.g., VT-212™ and/or VT-211™, are used to target the initial stage of a maladaptive response to prevent, delay, and/or reduce the severity of ulceration that is often seen about 7 to 10 days after mucotoxic therapies.

In alternative embodiments, compositions (the drug combinations) and methods of the invention:
1. Are administered orally.
2. Are administered such that they will not interfere with the patient's ongoing oncologic therapy and will not adversely affect the treatment outcome. Thus, in this embodiment, the ongoing therapy is not compromised, and this invention's treatment for mucositis should not compromise the safety of the patient. In one aspect, the drug combination of this invention also has anti-cancer activity.

Accordingly, alternative embodiments of the invention, including the compositions (the drug combinations) and methods of the invention for treating mucositis, such as an oral or a digestive mucositis, provide a safe oral regimen of pre-approved drugs that are able to act synergistically to:
1. Provide prophylactic protection to the oral cavity.
2. Reduce signals that could lead to ulceration (runaway inflammation).
3. Facilitate wound healing/repair.

Alternative embodiments of the invention have two aspects: First is to induce a protective response in the normal tissue by, e.g., inducing the tissue's natural "heat shock" protective response. Second is to attenuate excess inflammatory reaction by, e.g., inhibiting lymphocyte influx so that rate of debridement and reactive oxygen species (ROS) generation is within the adaptive range of the tissue. In one embodiment, the compositions and methods of this invention prevent accumulation of toxic products during the initial beneficial inflammatory response and do not cause excess damaging (runaway) inflammation.

In one embodiment, the compositions and methods of this invention induce a protective Heat Shock Response. In response to stress, mammalian cells produce stress or heat shock proteins (HSPs). Stress or heat shock proteins are among the most conserved proteins present in both prokaryotes and eukaryotes. HSPs comprise molecular chaperones that are essential for the quality control of intracellular proteins. Intracellular HSP have been shown to have an anti-inflammatory role in various conditions. HSP induction by a composition or method of this invention before a pro-inflammatory stimulus can be beneficial; however, HSP induction after a pro-inflammatory stimulus is cytotoxic.

In one embodiment, the compositions (the drug combinations) and methods of this invention comprise the acyclic isoprenoid, geranylgeranylacetone (teprenone), which is a non-toxic Hsp 70 inducer. Teprenone-comprising compositions (the drug combinations) and methods of this invention can block insult-induced apoptosis at multiple levels; for example, they can inhibit activation of c-Jun N-terminal kinases, decline of mitochondrial membrane potential, and formation of apoptosome by binding with Apaf-1.

In one embodiment, the compositions (the drug combinations) and methods of this invention comprising geranylgeranylacetone (teprenone) have a cytoprotective function by altering the membrane signaling cascade that leads to DNA damage. The prophylactic administration of compositions (the drug combinations) and methods of this invention comprising geranylgeranylacetone (teprenone) before radiation can promote an accumulation of protective HSPs in non-cancer cells in advance of radiation treatment and potential damage.

Compositions (the drug combinations) and methods of this invention can comprise, or be used in conjunction with, other heat shock protein inducers such as zinc, salicylates, nicotine, alcohol, α-adrenergic agonists, PPAR-γ agonists, bimoclomol, geldanamycin, teprenone, and cyclopentenone prostanoids.

In one embodiment, the compositions (the drug combinations) and methods of this invention comprise Angiotensin Converting Enzyme (ACE) inhibitors. The renin-angiotensin system (RAS) is an important determinant of vascular and fluid homeostasis as well as blood pressure regulation. Thus, compositions (the drug combinations) and methods of this invention can be used to regulate cellular growth in a variety of tissues through the principal effector of an ACE inhibitor, angiotensin II, and its two receptors, AT1 and AT2.

In one embodiment, the compositions (the drug combinations) and methods of this invention comprise N-Acetyl-Seryl-Aspartyl-Lysyl-Proline (Ac-SDKP or AcSDKP), a tetrapeptide that has been reported to inhibit endothelin 1 (ET-1)-induced cell proliferation and collagen synthesis in cultured rat cardiac fibroblasts (CFs) and to reduce left ventricle collagen deposition in rats with aldosterone-salt- and angiotensin II (Ang II)-induced hypertension. By comprising ACE inhibitors, the compositions (the drug combinations) and methods of this invention can increase plasma Ac-SDKP, which in turn inhibits LV inflammatory cell infiltration, interstitial cell proliferation (most likely fibroblasts), TGF-β, Smad2 activation, and collagen deposition. By comprising ACE inhibitors, the compositions (the drug combinations) and methods of this invention can increase plasma and tissue Ac-SDKP and decrease cardiac and renal fibrosis. By comprising Ac-SDKP , the compositions (the drug combinations) and methods of this invention can mediate the anti-inflammatory and antifibrotic effects of ACE inhibitors.

In one embodiment, the compositions (the drug combinations) and methods of this invention comprise angiotensin II (ang II). By comprising Ang II, the compositions (the drug combinations) and methods of this invention can cause neutrophil recruitment, which can be mediated through the release of CXC chemokines such as CINC/KC and MIP-2 in rats and IL-8 in humans, and may contribute to the infiltration of neutrophils.

In one aspect of this invention, a mucositis therapy (e.g., for oral or digestive mucositis) will begin at least about three days in advance of a chemotherapy dose and, in one aspect, will end the evening before the chemotherapy or radiation therapy. In one embodiment of this invention, dosing with a composition of this invention (a drug combination) comprising an ACE inhibitor will start at the same time as the teprenone but will continue for two days after chemotherapy treatment. In one aspect of this invention, to address any safety concerns the first dose of ACE inhibitor can be administered in the physician office so blood pressure can be monitored during the first few hours when a hypotensive event would be most likely. In one embodiment of this invention, the dosing of the teprenone is tid (three times daily) using the highest safe dose (current label is for 150mg per day total). In an alternative embodiment of this invention, the dosing for captopril is the lowest recommended dose (6.25 mg/dose) administered tid (three times daily).

In alternative embodiments, further benefits of compositions of this invention comprising teprenone and ARBs/ACE inhibitors of this invention are: a number of additional potential benefits of both teprenone and ARBs/ACE inhibitors beyond the mucosal (oral and intestinal) tissue, especially in the hematopoietic tissue compartment, auditory compartment, eye, lung and central nervous system that suggest these products may have much broader protection. In addition, the systemic cytoprotection may have a positive impact on the underlying cause of cancer fatigue and impairment of cognitive and physical function.

While the invention is not limited by any particular mechanism of action, additional modes of actions by which a product and methods of this invention, e.g., using VT-212™ and/or VT-211™, may provide benefit include:
1. Teprenone may systemically damp tissue turnover via inhibition of prenylation through other means that in turn block Rab, Ras and Rho signaling. This can have both system anti-inflammatory effects as well as causing a greater percentage of cells to be in Go or stationary phase. Tissues are more protected in Go than in other phases of the cell cycle. In addition, teprenone modulates fluidity of membranes and there is evidence that this increases the ability of the tissue to heal or deal with trauma to the mucosal membranes.
2. ACE inhibitors increase the flux of bradykinins as well as AcSDKP, both of which have anti-inflammatory and cytoprotective impact. In particular, AcSDKP has a direct impact on inducing the Go phase of the hematopoietic tissue (and possibly other tissue compartments) that further protects those compartments from radiotherapy or chemotherapy induced toxicity.
3. ARBs increase flux through the AT receptor 2 signaling pathway that has anti-inflammatory effects. The increase in Ang II also increases the creation of Angiotensin 1-7 which has additional hematopoietic protective as well as growth stimulating effects.

Impact of Teprenone, ARBs/ACE Inhibitors, and NSAIDs. There are many years of experience with respect to use and safety for all these classes of drugs, and no evidence of significant adverse effects on chemotherapy or radiation therapy has been shown to date. An area of possible concern is with hematological cancers with myeloablative therapy because ACE inhibitors in particular may prevent the virtually total ablation of the bone marrow when this virtually total ablation is in fact desired. Therefore, in a particular non-limiting embodiment of this invention, a mucositis therapy (e.g. comprising ACE inhibitors), as provided herein, is to be used for solid tumors (rather than to protect against mucositis from myeloablation therapy).

As noted above, cancer cells often have an already highly activated stress response (including, e.g. a near maximal or maximal heat shock response); in particular in cancer tissue environments characterized by the typical hypoxic, acidic and non-physiological conditions of a cancer tissue. Therefore adding an HSP inducer should have little or no effect on the tumor whereas it will increase the level of protection of the healthy tissue.

Therapies Provided and Intended for Protection. The instant invention provides therapies comprising one or more members selected from the following Groups: as shown in Table 1, where: of Group A comprises or consists of, or Group A comprises use of or consists of use of, geranylgeranyl compounds, including for example, geranylgeranyl acetone (GGA) and analogs of geranylgeranyl acetone (GGA); Group B comprises use of or consists of use of Angiotensin Converting Enzyme Inhibitors (ACE Inhibitors or ACE-Is); Group C comprises use of or consists of use of Angiotensin Receptor Blockers (ARBs); Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises use of or consists of use of non-steroidal anti-inflammatory drugs (NSAIDs); Group F consists of NCCN therapies; Group G comprises use of or consists of use of TNF inhibitors, e.g. pentoxifylline; Group H comprises use of or consists of use of deferrioxamine; Group I comprises polyunsaturated fatty acids, such as e.g. omega-3 fatty acids, including for example eicosapentaenoic acid (EPA), and docosahexaenoic acid (DHA); Group J comprises α-difluoromethylornithine; Group K comprises superoxide dismutase (SOD) and similar antioxidant compounds; Group L comprises activators of heat shock response; and Group M comprises drugs that reduce the inflammatory/progressive tissue damage response.

This invention provides compositions, e.g., pharmaceutical compounds, preparations, formulations, kits and products of manufacture (e.g., blister packages, shrink wraps, etc.), comprising combinations of beneficial ingredients for the primary treatment of cancer and any conditions caused by dysfunctional cells. The invention provides compositions, e.g., pharmaceutical compounds, preparations, formulations, kits and products of manufacture (e.g., blister packages, shrink wraps, etc.), comprising combinations of beneficial ingredients that are serviceable as therapies for improving, treating, ameliorating and/or preventing conditions, states and/or disease symptoms involving mucositis, such as oral mucositis. Relevant symptoms improved, treated, ameliorated and/or prevented by the compositions and methods of the invention further include, e.g., inflammation, excessive sympathoneural drive, cachexia, anorexia, anorexia-cachexia, stress, anxiety related thereto, wasting and/or atrophy, such as muscle atrophy, or anorexia-cachexia, e.g., as cachexia related to chronic and/or wasting diseases, such as cancer, and method of using them.

Table 1 provides exemplary combinations of drugs and/or pharmaceuticals of the invention. These components (e.g., exemplary combinations of drugs) are presented as members of groups with non-limiting examples provided that can be used in the compositions (e.g., pharmaceutical compounds, preparations, formulations, kits and products of manufacture) and methods of the invention.

Table 1 sets forth ingredients and combinations of active ingredients, e.g., drugs, of this invention. By way of non-limiting exemplification, any heat shock protein inducer can be used, including all possible permutations and combinations of ingredients as provided herein.

The following Table 1 describes exemplary ingredients of compositions of this invention, and compositions used in methods of this invention:

| **Group** | **Group Members** |
|---|---|
| A | Geranyl compounds: acyclic polyisoprenoids: |
| | Including, e.g., geranyl geranyl acetone (GGA) and analogs of geranyl geranyl acetone (GGA). |
| | Geranyl geranoic acid; and 4,5-didehydro geranyl geranoic acid. |
| | Additional compounds that can be used to practice this invention are described in: U.S. Patent No. (USPN) 7,1258,52; U.S. Patent Application No. 2006/0135623. |
| | Teprenone, e.g., SELBEX™ (CAS Registry Number 6809-52-5 |
| B | Angiotensin Converting Enzyme Inhibitors (ACE Inhibitors, ACE-I's, ACEi): Including, e.g., benazepril (e.g., LOTENSIN™); captopril (e.g., CAPOTIN™); cilazapril; enalapril (e.g., RENITEC™, VASOTEC™); enalaprilat; fosinopril (e.g., MONOPRIL™), fosinoprilat, imidapril (or imidaprilum), lisinopril; moexipril (e.g., UNIVASC™, PERDIX™); perindopril (or coversyl); quinapril (e.g., ACCUPRIL™); ramipril (e.g., TRITACE™, RAMACE™, ALTACE™); trandolapril (e.g., MAVIK™). |
| C | Angiotensin Receptor Blockers (ARBs) |
| | Including, e.g., Candesartan; Eprosartan; Irbesartan; Losartan; Olmesartan; Telmisartan; Valsartan. |
| D | Peroxisome Proliferator-Activated Receptor (PPAR) ligands. e.g. agonists |
| | Including, e.g., PPAR alpha agonists, PPAR gamma agonists; PPAR alpha/gamma dual agonists, and other PPAR ligands (e.g. PPAR delta ligands). Examples include rosiglitazone (e.g., AVANDIA™), pioglitazone (e.g., ACTOS™), troglitazone (e.g., REZULIN™), Muraglitzar, naveglitazar, netoglitazone, rivoglitazone, and tesaglitazar.. |
| | Additional compounds that can be used to practice this invention are described in: U.S. Patent No. (USPN) 6,756,399. Additional examples are provided in US Patent No. 6,869,967. |
| E | Non-Steroidal Anti-Inflammatory Drugs (NSAIDs) |
| | Including, e.g., NSAIDs, e.g. aspirin, diclofenac (e.g., VOLTAREN™, CATAFLAM™, VOLTAREN-XR™); diflunisal (e.g., DOLOBID™); etodolac (e.g., LODINE™); fenoprofen (e.g., NALFON™); flurbiprofen (e.g., ANSAID™, FROBEN™); ibuprofen (e.g., MOTRIN™); indomethacin (e.g., INDOCIN™, INDOCIN -SR™); ketoprofen, or (RS)2-(3-benzoylphenyl)-propionic acid (e.g., ORUDIS™, ORUVAIL™); ketorolac or ketorolac tromethamine (e.g., TORADOL™); Meclofenamate (e.g., MECLOMEN™); mefenamic acid, or 2(2,3-dimethylphenyl)aminobenzoic acid (e.g., PONSTEL™); meloxicam (e.g., MOBICOX™, MOBIC™); nabumetone (e.g., RELIFEX™, RELAFEN™); Naproxen (e.g., ALEVE™, ANAPROX™, MIRANNAX™, NAPROGESIC™, NAPROSYN™, NAPRELAN™, SYNFLEX™); oxaprozinum or oxaprozin (e.g., DAYPRO™ or DURAPROX™) ; piroxicam (e.g., FELDENE™); salsalate (e.g., DISALCID™, SALFLEX™), sulindac (e.g., CLINORIL™); tolmetin (e.g., TOLECTIN™); and, COX-2-selective NSAIDs, e.g. celecoxib (e.g., CELEBRA™, CELEBREX™); rofecoxib (e.g., VIOXX™, CEOXX™); etoricoxib (e.g., ARCOXIA™); valdecoxib (e.g., BEXTRA™); parecoxib (e.g., DYNASTAT™); meloxicam (e.g., MOBIC™); Lumiracoxib (e.g., PREXIGE™). |

**Table 1-Section 2: "Cidal/Static" Ingredients**

| **Group** | **Group Members** |
|---|---|
| F | Any Chemotherapy or Radiation Therapy |
| | Including, e.g., any chemotherapy or radiation therapy, that is listed in the current NCCN Clinical Practice Guidelines in Oncology™. |

**Table 1-Section 3: "Complementary" Ingredients**

| **Group** | **Group Members** |
|---|---|
| G | TNF inhibitors |
| | Including, e.g., anti-TNF agents, anti-TNF mAbs, pentoxifylline, or 1-(5-oxohexyl)-3, 7-dimethylxanthine (e.g., TRENTAL™), thalidomide or 2-(2,6-dioxopiperidin-3-yl) isoindoline-1,3-dione (e.g., THALOMID™). NOTE: Pentoxifylline is a PDE4 inhibitor that increases intracellular cAMP and stimulates PKA activity. Pentoxifylline is also an inhibitor of tumor necrosis factor-alpha. |
| H | Deferrioxamine or deferoxamine |
| | synonyms: deferoxamine mesylate; deferoxaminum; deferrioxamine; deferoxamin; DFO; N-benzoylferrioxamine B; DF B; DFOA; DFOM; desferrioxamine; desferrioxamine B; desferrioxamine B; deferoxamide B; deferoxamine B; brand names containing deferoxamine: DESFERAL™, DESFERAN™, DESFERAL™, DESFEREX™, DESFERIN™, DESFERRIN™. |
| | NOTE: Deferrioxamine is a chelator that has been used for than 20 years in treatment of severe ion poisoning, of disturbances in iron storage and of diseases which lead to increased iron values. It may have other activities. |
| I | Polyunsaturated fatty acids |
| | Including, e.g., polyunsaturated fatty acid; e.g., a ω-3 fatty acid or omega-3 fatty acid, such as α-linolenic acid (ALA), eicosapentaenoic acid (EPA), and docosahexaenoic acid (DHA); or a ω-6 fatty acid or omega-6 fatty acid, such as linoleic acid (LA); or a as ω-9 fatty acid or omega-9 fatty acid, such as oleic acid and erucic acid. In non-limiting examples, these fatty acids may be administered orally or intravenously. |
| J | eflornithine (α-difluoromethylornithine or DFMO) (e.g., ORNIDYL™) Note: α-Difluoromethylornithine is an inhibitor of ornithine decarboxylase (an enzyme involved in polyamine synthesis), by reducing the polyamine content of mucosa. |
| K | Superoxide dismutase (SOD) (EC 1.15.1.1) |
| | Including, e.g., Group K comprises superoxide dismutase (SOD), catalase, superoxide dismutase (SOD)-catalase conjugates or complexes, peroxiredoxins, and peroxidases (e.g. glutathione peroxidase), and enzymes of similar activity. |
| | Superoxide dismutase (SOD), and similar antioxidant compounds, can include: |
| | a/ enzymes with the ability to catalyze the conversion of hydrogen peroxide into other compounds; |
| | b/ enzymes with the ability to catalyze the decomposition of peroxides; |
| | c/ compounds that have antioxidant activity; and |
| | d/ compounds that promote antioxidant activity, such as co-factors, precursors and/or subunits and other enzymes in a shared biochemical reaction pathway, including, e.g. selenium, cysteine, glutamine, tryptophan, and glutathione reductase. |
| L | Activators of heat shock response |
| | Including, e.g. geranylgeranylacetone or gernate, zinc, tin, salicylates, dexamethasone, cocaine, nicotine, alpha-adrenergic agonist, ppar-gamma agonist, a geldanamycin (which is a benzoquinone ansamycin antibiotic that binds to Hsp90, or Heat Shock Protein 90), biomolecular-geldanamycin, cyclopentanone, a prostanoid (a subclass of eicosanoid consisting of: the prostaglandins, thromboxanes, prostacyclins), enprostil (a synthetic prostaglandin), paracetamol or acetaminophen (*N*-(4-hydroxyphenyl)-acetamide), ketotiphen, levamisole, diazepam (e.g., VALIUM™), bromocriptine (e.g., PARLODEL™), dopamine (4-(2-aminoethyl)benzene-1,2-diol). |
| M | Drugs that reduce the inflammatory/progressive tissue damage response including |
| | Including, e.g. ACE inhibitors, ARBs, phenylbutryrate or PPAR gamma (insulin sensitizer) and/or probucol, calcitriol as alternative combinations to further stimulate protective vascular response, NSAIDs to reduce inflammation, peptide-containing compounds, inhibitors of macrophage migration inhibitory factor (MIF), inhibitors of lipoxin biosynthesis, natural alternatives to the synthetic COX inhibitors and LOX inhibitors, and other natural products (including existing, novel and yet to be discovered products) such as lipids, oils (e.g. mussel oil), peptides, and small molecules, and products derived and/or extracted from lipids or oils or peptides or small molecules. |

In alternative aspects, subgroups (categories within each Group) in Table 1 are not necessarily mutually exclusive.

The following Table 2 describes exemplary amounts of ingredients used to formulate and/or administer compositions of this invention:

| Group (with example of a group member) | Example 1 Dose | Exemplary amounts of(both individually and collectively as a group) |
|---|---|---|
| Group A. | 30 mg to 3 | From about 0.10 mg to about 20.00000 gm per day inclusively, including for example each daily amount within this range separated by an increment of about 0.01 mg (e.g. 0.10 mg, 0.11 mg, 0.12 mg, 0.13 mg, etc.). |
| Gerantylgeranylacetones | gm per day | |
| ▪ E.g. GGA | | |
| Group B. | 10 mg to | From about 0.10 mg to about 10.00000 gm per day inclusively, including for example each daily amount within this range separated by an increment of about 0.01 mg (e.g. 0.10 mg, 0.11 mg, 0.12 mg, 0.13 mg, etc.). |
| ACE Inhibitors | 450mg per | |
| ▪ E.g. Captopril | day | |
| Group C. | 10 to 100 mg | From about 0.10 mg to about 10.00000 gm per day inclusively, including for example each daily amount within this range separated by an increment of about 0.01 mg (e.g. 0.10 mg, 0.11 mg, 0.12 mg, 0.13 mg, etc.). |
| Angiotensin II receptor | per day | |
| Blockers | | |
| ▪ E.g. Lovartab | | |
| Group D. | 1mg to 8mg | From about 0.10 mg to about 10.00000 gm per day inclusively, including for example each daily amount within this range separated by an increment of about 0.01 mg (e.g. 0.10 mg, 0.11 mg, 0.12 mg, 0.13 mg, etc.). |
| PPAR gamma (and alpha) | per day | |
| agonists | | |
| ▪ E.g. | | |
| Rosiglitazone | | |
| Group E. | Various | From about 0.10 mg to about 10.00000 gm per day inclusively, including for example each daily amount within this range separated by an increment of about 0.01 mg (e.g. 0.10 mg, 0.11 mg, 0.12 mg, 0.13 mg, etc.). |
| Non-steroidal anti- | | |
| inflammatorv drugs | | |
| (NSAIDs) E.g. etodolac (e.g., LODINE™) | | |
| Group F. | Various | Various (including the ranges recommended by NCCN, but also including up to 10x higher limits). |
| NCCN therapies | | |

Group A: The invention provides compositions comprising geranylgeranyl compounds, including for example geranylgeranyl acetone (GGA) and analogs of geranylgeranyl acetone (GGA).

GGA: The ingredients serviceable for the current invention include the GGA analogues named in U.S. Patent Application Pub. No. 20060135623; specifically they include members that are categorized by U.S. Patent Application Pub. No. 20060135623 into categories as exemplified by categories termed Class I, Class II, Class IIIa, Class IIIb, Class IIIc, Class IIId, IVa, IVb, IVc, IVd, V, VIa, VIb, VIc, VId, VIe, VIf, VIg, VIIa, VIIb, VIIc, VIId, VIIIa, VIIIb, VIIIc, VIIId, IXa, IXb, Xa, Xb, Xc, XIa, XIb, XII, XIIIa, XIIIb, XIV, XVa, XVb, XVc, XVIa, XVIb, XVIc, XVIIa, XVIIb, and XVIIc, as follows:
The invention provides compositions comprising GGA analogues, including GGA-like molecules with GGA antioxidant activity. Compositions and methods of this invention comprising geranylgeranyl acetone can have anti-ulcer activity and can induce heat-shock protein upregulation. Compositions and methods of this invention comprising GGA can be used as an antioxidant, or to induce thioredoxin, a protein which plays an important protective role against oxidative stress. Compositions and methods of this invention can comprise derivatives of GGA with varying numbers and configurations of double bonds and differing attached functional groups, and these compositions can have antioxidant activity. While the invention is not limited by any particular mechanism of action, such analogues may derive their antioxidant activity from increasing the expression of thioredoxin or other genes involved in the protection of cells and tissues from oxidative stress. Non-limiting Exemplary GGA analogues are shown below.

Class I: One class of GGA analogues (Class I) used to practice this invention, e.g., in the methods, formulations, dosages, combinations, and routes of administration of this invention, comprises compounds of the formula:
In alternative embodiments, Class I compounds used to practice this invention can have n=8 or 9 and R1 can be any of the following functional groups: 1) acetone, 2) ethyl, 3) hydroxyalkyl 4) amine 5) hydroxyl 6) pivaloyl 7)formyl 8) propionyl 9) butryl) 10) valeryl 11) isovaleryl 12) stearoyl 13) myristoyl 14) palmitoyl 15) oleoyl 16) benzoyl 17) lauroyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class II: Compositions and methods of this invention can comprise another class of GGA analogues (Class II) including compounds of formula (I) in which n=8 or 9 and R1 can be any of the following functional groups: 1) 2-oxopropyl 2) 2-oxopentyl 3) 3-methyl-2-oxobutyl 4) amino 5) carbethoxyl amino 6) 2-hydroxypropyl 7) retinol acetate.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class IIIa: Compositions and methods of this invention can comprise another class of GGA analogues (Class IIIa), which can include derivatives of the geranyl geranyl family that have 5 instead of 4 double bonds with an additional double bond as illustrated below:
Compositions and methods of this invention can comprise use of Class IIIa compounds, which can be in all-trans or a 5-cis form or can be a mixture of these two isomers. R₂ can be any one of the following functional groups: 1) acetone, 2) ethyl, 3) hydroxyalkyl 4) amine 5) hydroxyl 6)formyl 7) propionyl 8) butryl) 9) 2-oxopropyl 10) 2-oxopentyl 11) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino; 23) 3-methyl-2-oxobutyl; 24) 2-hydroxypropyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class IIIb: Compositions and methods of this invention can comprise another class of GGA analogues (Class IIIb), which can include derivatives of the geranyl geranyl family that have 6 instead of 4 double bonds with additional double bonds as illustrated below:

In alternative embodiments, Class IIIb compounds can be in all-trans or a 5-cis form or can be a mixture of these two isomers. R₃ can be any one of the following functional groups: 1) acetone, 2) ethyl, 3) hydroxyalkyl 4) amine 5) hydroxyl 6)formyl 7) propionyl 8) butryl) 9) 2-oxopropyl 10) 2-oxopentyl 11) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino 23) 3-methyl-2-oxobutyl 24) 2-hydroxypropyl.

These compounds may be synthesized using chemical synthesis methods familiar to those of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class IIIc: Compositions and methods of this invention can comprise another class of GGA analogues (Class IIIc) includes derivatives of the geranyl geranyl family that have 7 instead of 4 double bonds with additional double bonds as illustrated below:
In alternative embodiments, Class IIIc compounds can be in all-trans or a 5-cis form or can be a mixture of these two isomers. R₄ can be any one of the following functional groups: 1) acetone, 2) ethyl, 3) hydroxyalkyl 4) amine 5) hydroxyl 6)formyl 7) propionyl 8) butryl) 9) 2-oxopropyl 10) 2-oxopentyl 11) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino 23) 3-methyl-2-oxobutyl 24) 2-hydroxypropyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class IIId: Compositions and methods of this invention can comprise another class of GGA analogues (Class IIId) which includes derivatives of the geranyl geranyl family that have 8 instead of 4 double bonds with additional double bonds as illustrated below:
In alternative embodiments, Class IIId compounds can be in all-trans or a 5-cis form or can be a mixture of these two isomers. R₅ can be any one of the following functional groups: 1) acetone, 2) ethyl, 3) hydroalkyl, 4) amine, 5) hydroxyl 6)formyl, 7) propionyl, 8) butryl,) 9) 2-oxopropyl, 10) 2-oxopentyl, 11) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino 23) 3-methyl-2-oxobutyl 24) 2-hydroxypropyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class IVa: Compositions and methods of this invention can comprise another class of GGA analogues (Class IVa) which includes derivatives of the geranyl geranyl family that have 3 instead of 4 double bonds, with the double bonds at positions C13 and C17 being saturated and with an additional double bond at the C I position as illustrated below:
In alternative embodiments, Class IVa compounds can be in all-trans or a 5-cis form or can be a mixture of these two isomers. R₆ can be any one of the following functional groups: 1) acetone, 2) ethyl, 3) hydroxyalkyl 4) amine 5) hydroxyl 6)formyl 7) propionyl 8) butryl) 9) 2-oxopropyl 10) 2-oxopentyl 11) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino 23) 3-methyl-2-oxobutyl 24) 2-hydroxypropyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class IVb: Compositions and methods of this invention can comprise another class of GGA analogues (Class IVb) which includes derivatives of the geranyl geranyl family that have 2 instead of 4 double bonds with some double bonds being saturated and with a new double bond as illustrated below:
In alternative embodiments, Class IVb compounds can be in all-trans or a 5-cis form or can be a mixture of these two isomers. R₇ can be any one of the following functional groups: 1) acetone, 2) ethyl, 3) hydroxyalkyl 4) amine 5) hydroxyl 6)formyl 7) propionyl 8) butryl) 9) 2-oxopropyl 10) 2-oxopentyl 11) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino 23) 3-methyl-2-oxobutyl 24) 2-hydroxypropyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class IVc: Compositions and methods of this invention can comprise another class of GGA analogues (Class IVc) which includes derivatives of the geranyl geranyl family that have 1 instead of 4 double bonds with some double bonds saturated and with a single double bond as illustrated below:
In alternative embodiments, Class IVc compounds can be in all-trans or a 5-cis form or can be a mixture of these two isomers. R₈ can be any one of the following functional groups: 1) acetone, 2) ethyl, 3) hydroxyalkyl 4) amine 5) hydroxyl 6)formyl 7) propionyl 8) butryl) 9) 2-oxopropyl 10) 2-oxopentyl 11) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino 23) 3-methyl-2-oxobutyl 24) 2-hydroxypropyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class IVd: Compositions and methods of this invention can comprise another class of GGA analogues (Class IVd) which includes derivatives of the geranyl geranyl family that have no double bonds as illustrated below:
In alternative embodiments, R₉ can be any one of the following functional groups: 1) acetone, 2) ethyl, 3) hydroxyalkyl 4) amine 5) hydroxyl 6)formyl 7) propionyl 8) butryl) 9) 2-oxopropyl 10) 2-oxopentyl 11) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino 23) 3-methyl-2-oxobutyl 24) 2-hydroxypropyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class V: Compositions and methods of this invention can comprise another class of GGA analogues (Class V) which includes a 5-cis-geranylgeranyl compound as shown below:
In alternative embodiments, R.sub.10 can be any one of the following functional groups: 1) acetone, 2) ethyl, 3) hydroxyalkyl 4) amine 5) hydroxyl 6)formyl 7) propionyl 8) butryl) 9) 2-oxopropyl 10) 2-oxopentyl 11) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino 23) 3-methyl-2-oxobutyl 24) 2-hydroxypropyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class VIa: Compositions and methods of this invention can comprise another class of GGA analogues (Class VIa) which includes derivatives of .alpha.-carotene, as illustrated below, where n=2-4 and the isoprenoid units can exist in any reduced or form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class VIb: Compositions and methods of this invention can comprise another class of GGA analogues (Class VIb) which includes derivatives of beta-carotene, as illustrated below, where n=2-4 and the isoprenoid units can exist in any reduced or oxidized form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001

Class VIc: Another class of GGA analogues (Class VIc) includes derivatives of gamma-carotene, as illustrated below, where n=2-4 and the isoprenoid units can exist in any reduced or oxidized form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001

Class VId: Compositions and methods of this invention can comprise another class of GGA analogues (Class VId) which includes derivatives of lycopene, as illustrated below, where n=24 and the isoprenoid units can exist in any reduced or oxidized form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class VIe: Compositions and methods of this invention can comprise another class of GGA analogues (Class VIe) which includes derivatives of phytoene, as illustrated below, where n=2-6, 8, or 9 and the compound is either in a reduced or oxidized form

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class VIf: Compositions and methods of this invention can comprise another class of GGA analogues (Class VIf) which includes derivatives of lutein, as illustrated below, where n=2-4 and the isoprenoid units can exist in any reduced or oxidized form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class VIg: Compositions and methods of this invention can comprise another class of GGA analogues (Class VIg) which includes derivatives of zeaxanthin, as illustrated below, where n=24 and the isoprenoid units can exist in any reduced or oxidized form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class VIIa: Compositions and methods of this invention can comprise another class of GGA analogues (Class VIIa) which includes derivatives of alpha-tocopherol (Vitamin E), as illustrated below, where n=24 and the isoprenoid units can exist in any reduced or oxidized form

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class VIIb: Compositions and methods of this invention can comprise another class of GGA analogues (Class VIIb) which includes derivatives of tocopherol, as illustrated below, where n=2-4 and the isoprenoid units can exist in any reduced or oxidized form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class VIIc: Compositions and methods of this invention can comprise another class of GGA analogues (Class VIc) which includes derivatives of delta-tocopherol, as illustrated below, where n=2-4 and the isoprenoid units can exist in any reduced or oxidized form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class VIId: Compositions and methods of this invention can comprise another class of GGA analogues (Class VIId) which includes derivatives of .gamma.-tocopherol, as illustrated below, where n=2-4 and the isoprenoid units can exist in any reduced or oxidized form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class VIIIa: Compositions and methods of this invention can comprise another class of GGA analogues (Class VIIIa) which includes derivatives of alpha-tocotrienol, as illustrated below, where n=1, 2, 4, 5, 6, 8, or 9, and the isoprenoid chain existing in any reduced or oxidized form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class VIIIb: Compositions and methods of this invention can comprise another class of GGA analogues (Class VIIIb) which includes derivatives of beta-tocotrienol, as illustrated below, where n=1, 2, 4, 5, 6, 8, or 9, and the isoprenoid chain existing in any reduced or oxidized form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001

Class VIIIc: Compositions and methods of this invention can comprise another class of GGA analogues (Class VIIIc) which includes derivatives of delta-tocotrienol, as illustrated below, where n=1, 2, 4, 5, 6, 8, or 9, and the isoprenoid chain existing in any reduced or oxidized form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001

Class VIIId: Compositions and methods of this invention can comprise another class of GGA analogues (Class VIIId) which includes derivatives of gamma-tocotrienol, as illustrated below, where n=1, 2, 4, 5, 6, 8, or 9, and the isoprenoid chain existing in any reduced or oxidized form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class IXa: Compositions and methods of this invention can comprise another class of GGA analogues (Class IXa) which includes derivatives of phylloquinone (Vitamin K₁), as illustrated below, where n=2-4, and the isoprenoid chain exists in any reduced or oxidized form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class IXb: Compositions and methods of this invention can comprise another class of GGA analogues (Class IXb) which includes derivatives of menaquinone (Vitamin K₂), as illustrated below, where n=2-4, and the isoprenoid chain exists in any reduced or oxidized form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001

Class Xa: Compositions and methods of this invention can comprise another class of GGA analogues (Class Xa) which includes derivatives of semiquinone (Coenzyme Q10) radical, as illustrated below, where n=2-6, 8, or 9

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001

Class Xb: Compositions and methods of this invention can comprise another class of GGA analogues (Class Xb) which includes derivatives of the reduced form of Coenzyme Q10, as illustrated below, where n=2-6, 8, or 9.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class Xc: Compositions and methods of this invention can comprise another class of GGA analogues (Class Xc) which includes derivatives of the oxidized form of Coenzyme Q10, as illustrated below, where n=2-6, 8, or 9.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class XIa: Compositions and methods of this invention can comprise another class of GGA analogues (Class XIa) which includes derivatives of plaunotol, as illustrated below.

Class XIa compounds can be in all-trans or a 5-cis form or can be a mixture of these two isomers. R₁₁ can be any one of the following functional groups: 1) acetone, 2) ethyl, 3) hydroxyalkyl 4) amine 5) hydroxyl 6)formyl 7) propionyl 8) butryl) 9) 2-oxopropyl 10) 2-oxopentyl 11) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino 23) 3-methyl-2-oxobutyl 24) 2-hydroxypropyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class XIb: Compositions and methods of this invention can comprise another class of GGA analogues (Class XIb) which includes derivatives of gefarnate, as illustrated below, where n=2-4, and the isoprenoid units can exist in any reduced or oxidized form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class XII: Compositions and methods of this invention can comprise another class of GGA analogues (Class XII) which includes derivatives of cholesterol glucoside, as illustrated below, where n=1-4, and the isoprenoid units can exist in any reduced or oxidized form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class XIIIa: Compositions and methods of this invention can comprise another class of GGA analogues (Class XIIIa) which includes derivatives of diferuloylmethane (curcumin), as illustrated below, where n=1-4, and the isoprenoid units can exist in any reduced or oxidized form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class XIIIb: Compositions and methods of this invention can comprise another class of GGA analogues (Class XIIIb) which includes derivatives of sulforaphane, as illustrated below, where n=1-4, and the isoprenoid units can exist in any reduced or oxidized form.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class XIV: Compositions and methods of this invention can comprise another class of GGA analogues (Class XIV) which includes derivatives of triterpenoid electrophiles, also known as avicins, as illustrated below. The R**_{12 to 15}** groups of the Class XIV compositions shown below can be either hydrogen or a hydroxyl group.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class XVa: Compositions and methods of this invention can comprise another class of analogues (Class XVa) which includes derivatives of docosahexaenoic acid, as illustrated below, where the structure may contain between 0-8 methyl groups.

R.sub.16 can be any one of the following functional groups: 1) acetone, 2) ethyl, 3) hydroxyalkyl 4) amine 5) hydroxyl 6)formyl 7) propionyl 8) butryl) 9) 2-oxopropyl 10) 2-oxopentyl 11) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino 23) 3-methyl-2-oxobutyl 24) 2-hydroxypropyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class XVb: Compositions and methods of this invention can comprise another class of analogues (Class XVb) which includes derivatives of eicosapentaenoic acid, as illustrated below, where the structure may contain between 0-8 methyl groups.

R₁₇ can be any one of the following functional groups: 1) acetone, 2) ethyl, 3) hydroxyalkyl 4) amine 5) hydroxyl 6)formyl 7) propionyl 8) butryl) 9) 2-oxopropyl 10) 2-oxopentyl 11) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino 23) 3-methyl-2-oxobutyl 24) 2-hydroxypropyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class XVc: Compositions and methods of this invention can comprise another class of analogues (Class XVc) which includes derivatives of alpha linolenic acid, as illustrated below, where the structure may contain between 0-8 methyl groups.

R₁₈ can be any one of the following functional groups: 1) acetone, 2) ethyl, 3) hydroxyalkyl 4) amine 5) hydroxyl 6)formyl 7) propionyl 8) butryl) 9) 2-oxopropyl 10) 2-oxopentyl 11) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino 23) 3-methyl-2-oxobutyl 24) 2-hydroxypropyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class XVIa: Compositions and methods of this invention can comprise another class of analogues (Class XVIa) which includes derivatives of arachidonic acid, as illustrated below, where the structure may contain between 0-8 methyl groups.

R₁₉ can be any one of the following functional groups: 1) acetone, 2) ethyl, 3) hydroxyalkyl 4) amine 5) hydroxyl 6)formyl 7) propionyl 8) butryl) 9) 2-oxopropyl 10) 2-oxopentyl 11) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino 23) 3-methyl-2-oxobutyl 24) 2-hydroxypropyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class XVIb: Compositions and methods of this invention can comprise another class of analogues (Class XVIb) which includes derivatives of linoleic acid, as illustrated below, where the structure may contain between 0-8 methyl groups.

R₂₀ can be any one of the following functional groups: 1) acetone, 2) ethyl, 3) hydroxyalkyl 4) amine 5) hydroxyl 6)formyl 7) propionyl 8) butryl) 9) 2-oxopropyl 10) 2-oxopentyl 1) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino 23) 3-methyl-2-oxobutyl 24) 2-hydroxypropyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class XVIc: Compositions and methods of this invention can comprise another class of analogues (Class XVIc) which includes derivatives of gamma linolenic acid, as illustrated below, where the structure may contain between 0-8 methyl groups.

R₂₁ can be any one of the following functional groups: 1) acetone, 2) ethyl, 3) hydroxyalkyl 4) amine 5) hydroxyl 6)formyl 7) propionyl 8) butryl) 9) 2-oxopropyl 10) 2-oxopentyl 11) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino 23) 3-methyl-2-oxobutyl 24) 2-hydroxypropyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class XVIIa: Compositions and methods of this invention can comprise another class of analogues (Class XVIIa) which includes derivatives of erucic acid, as illustrated below, where the structure may contain between 0-8 methyl groups.

R₂₂ can be any one of the following functional groups: 1) acetone, 2) ethyl, 3) hydroxyalkyl 4) amine 5) hydroxyl 6)formyl 7) propionyl 8) butryl) 9) 2-oxopropyl 10) 2-oxopentyl 11) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino 23) 3-methyl-2-oxobutyl 24) 2-hydroxypropyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class XVIIb: Compositions and methods of this invention can comprise another class of analogues (Class XVIIb) which includes derivatives of 11-docosenoic acid, as illustrated below, where the structure may contain between 0-8 methyl groups.

R₂₃ can be any one of the following functional groups: 1) acetone, 2) ethyl, 3) hydroxyalkyl 4) amine 5) hydroxyl 6)formyl 7) propionyl 8) butryl) 9) 2-oxopropyl 10) 2-oxopentyl 11) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino 23) 3-methyl-2-oxobutyl 24) 2-hydroxypropyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

Class XVIIc: Compositions and methods of this invention can comprise another class of analogues (Class XVIIc) which includes derivatives of 5-docosenoic acid, as illustrated below, where the structure may contain between 0-8 methyl groups.

R₂₄ can be any one of the following functional groups: 1) acetone, 2) ethyl, 3) hydroxyalkyl 4) amine 5) hydroxyl 6)formyl 7) propionyl 8) butryl) 9) 2-oxopropyl 10) 2-oxopentyl 11) retinol acetate 12) stearoyl 12) myristoyl 13) valeryl 14) isovaleryl 15) pivaloyl 16) palmitoyl 17) oleoyl 18) benzoyl 19) lauroyl 20) stearoyl 21) amino 22) carbethoxyl amino 23) 3-methyl-2-oxobutyl 24) 2-hydroxypropyl.

These compounds may be synthesized using chemical synthesis methods familiar to one of ordinary skill in the art. General methods for chemical synthesis may be found in, among other sources, "Comprehensive Organic Transformations: A Guide to Functional Group Preparations", Richard C. Larock, Wiley-VCH: 1999 and in "March's Advanced Organic Chemistry: Reactions, Mechanisms and Structure", Jerry March and Michael Smith, John Wiley and Sons Inc.: 2001.

In the classes of compounds described herein, if a GGA analogue or related analogue contains stereochemistry, all enantiomeric and diastereomeric forms of the GGA analogues or related analogue are intended. Pure stereoisomers, mixtures of enantiomers and/or diastereomers, and mixtures of different GGA analogues or related analogue are described. Thus, the GGA analogues or related analogues may occur as racemates, racemic mixtures and as individual diastereomers, or enantiomers with all isomeric forms being included. A racemate or racemic mixture does not necessarily imply a 50:50 mixture of stereoisomers.

Where a given structural formula or chemical name is presented for a GGA analogue or related analogues, it is intended that all possible solvates, pharmaceutically acceptable salts, esters, amides, complexes, chelates, stereoisomers, geometric isomers, crystalline or amorphous forms, metabolites, metabolic precursors or prodrugs of the compound are also separately described by the chemical structural formula or chemical name."

### Group B: Angiotensin Converting Enzyme Inhibitors (ACE Inhibitors or ACE-Is). Group C: Angiotensin Receptor Blockers (ARBs).

Compositions and methods of this invention can comprise ACE inhibitor drugs that effect the renin-angiotensin system, which has a large degree of overlap with the effects of beta blockers. Compositions and methods of this invention can comprise compounds that inhibit the enzyme that converts angiotensin I to angiotensin II for controlling blood pressure, including ACE inhibitors e.g., angiotensin converting enzyme inhibitors), e.g., structurally related compounds.

Group D: Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands), or equivalents thereof.

The invention provides compositions and methods using rosiglitazone (e.g., AVANDIA™), pioglitazone (e.g., ACTOS™), troglitazone (e.g., REZULIN™), muraglitazar, naveglitazar, netoglitazone, rivoglitazone, and/or tesaglitazar, or equivalents thereof. Additional examples are provided in U.S. Patent No. 6,756,399, and U.S. Patent No. 6,869,967.

### Group E: non-steroidal anti-inflammatory drugs (NSAIDs).

The invention provides compositions and methods using NSAIDs. Exemplary non-steroidal anti-inflammatory drugs (NSAIDs) that can be used to practice this invention include, e.g., comprising aspirin, diclofenac (e.g., VOLTAREN™, CATAFLAM™, VOLTAREN-XR™); diflunisal (e.g., DOLOBID™); etodolac (e.g., LODINE™); fenoprofen (e.g., NALFON™); flurbiprofen (e.g., ANSAID™, FROBEN™); ibuprofen (e.g., MOTRIN™); indomethacin (e.g., INDOCIN™, INDOCIN -SR™); ketoprofen, or (RS)2-(3-benzoylphenyl)-propionic acid (e.g., ORUDIS™, ORUVAIL™); ketorolac or ketorolac tromethamine (e.g., TORADOL™); Meclofenamate (e.g., MECLOMEN™); mefenamic acid, or ₂-(2,3-dimethylphenyl) aminobenzoic acid (e.g., PONSTEL™); meloxicam (e.g., MOBICOX™, MOBIC™); nabumetone (e.g., RELIFEX™, RELAFEN™); Naproxen (e.g., ALEVE™, ANAPROX™, MIRANNAX™, NAPROGESIC™, NAPROSYN™, NAPRELAN™, SYNFLEX™); oxaprozinum or oxaprozin (e.g., DAYPRO™ or DURAPROX™); piroxicam (e.g., FELDENE™); salsalate (e.g., DISALCID™, SALFLEX™), sulindac (e.g., CLINORIL™); tolmetin (e.g., TOLECTIN™); and, COX-2-selective NSAIDs, e.g. celecoxib (e.g., CELEBRA™, CELEBREX™); rofecoxib (e.g., VIOXX™, CEOXX™); etoricoxib (e.g., ARCOXIA™); valdecoxib (e.g., BEXTRA™); parecoxib (e.g., DYNASTAT™); meloxicam (e.g., MOBIC™); or Lumiracoxib (e.g., PREXIGE™).

The invention provides compositions and methods using steroids. In additional aspects, invention also provides therapeutic combinations, e.g., pharmaceutical compositions of the invention, comprising steroids (including those shown in Table 1, above), e.g., anabolic steroids, such as andarine, ethylestrenol, mesterolone, methandrostenolone, methenolone, methyltestosterone, oxandrolone, oxymetholone stanozolol, boldenone, hexoxymestrolum, methandrostenolone, methenolone enanthate, nandrolone decanoate, nandrolone phenproprionate, stanozolol, stenbolone, testosterone cypionate, testosterone enanthate, testosterone, testosterone nicotinate, therobolin, trenbolone, trenbolone, trophobolene or a combination thereof. Alternative embodiments comprise all possible combinations of ingredients provided herein (e.g. members of Group A, Group B, Group C, Group D, Group E, Group F, Group G, Group H, Group I, Group J, Group K, Group L, and Group M in Table 1), including those in Table 1, and these ingredients include steroids and steroid combinations as provided herein.

### Group F: NCCN therapies.

The invention provides compositions and methods using NCCN Therapies: Exemplary chemotherapy or radiation therapies that can be used to practice this invention include therapies that are listed in the current NCCN Clinical Practice Guidelines in Oncology™ (NCCN therapies), and are exemplified by NCCN therapies in the category of A) NCCN "Treatment Of Cancer By Site" and in the category of B) NCCN "Supportive Care". The current NCCN therapies are published and available online, in print, and by request.

Exemplary NCCN therapies that can be used to practice this invention can be categorized under "Treatment by Site" include treatments for: 1) Acute Myeloid Leukemia; 2) Bladder Cancer (incl. Bladder Cancer, Upper Tract Tumors, Urothelial Carcinoma of the Prostate); 3) Bone Cancer (including Chondrosarcoma, Ewing's Sarcoma, Osteosarcoma); 4) Breast Cancer (incl. Noninvasive, Invasive, Phyllodes Tumor, Paget's Disease, Breast Cancer During Pregnancy); 5) Central Nervous System Cancers (incl. Adult Low-Grade Infiltrative Supratentorial Astrocytoma/ Oligo-dendroglioma, Adult Intracranial Ependymoma, Anaplastic Astrocytoma/Anaplastic Oligodendroglioma/Glioblastoma Multiforme, Limited (1-3) Metastatic Lesions, Multiple (>3) Metastatic Lesions, Carcinomatous Lymphomatous Meningitis, Non-immunosuppressed Primary CNS Lymphoma, Metastatic Spine Tumors, Principles of Brain Tumor Therapy); 6) Cervical Cancer; 7) Chronic Myelogenous Leukemia; 8) Colon/Rectal Cancer (incl. Colon Cancer, Rectal Cancer, Anal Carcinoma); 9) Esophageal Cancer; 10) Gastric Cancer; 11) Head and Neck Cancers (incl. Ethmoid Sinus Tumors, Maxillary Sinus Tumors, Salivary Gland Tumors, Cancer of the Lip, Cancer of the Oral Cavity, Cancer of the Oropharynx, Cancer of the Hypopharynx, Occult Primary, Cancer of the Glottic Larynx, Cancer of the Supraglottic Larynx, Cancer of the Nasopharynx, Advanced Head and Neck Cancer); 12) Hepatobiliary Cancers (incl. Hepatocellular Carcinoma, Gallbladder Cancer, Intrahepatic Cholangiocarcinoma, Extrahepatic Cholangiocarcinoma); 13) Hodgkin Disease/Lymphoma; 14) Kidney Cancer; 15) Melanoma; 16) Multiple Myeloma (incl. Multiple Myeloma, Waldenström's Macroglobulinemia); 17) Myelodysplastic Syndromes; 18) Neuroendocrine Tumors (incl. Multiple Endocrine Neoplasia: Type 1, Multiple Endocrine Neoplasia: Type 2, Carcinoid Tumors, Islet Cell Tumors, Pheochromocytoma, Poorly Differentiated/Small Cell/ Atypical Lung Carcinoids); 19) Non-Hodgkin's Lymphoma (incl. Chronic Lymphocytic Leukemia/Small Lymphocytic Lymphoma, Follicular Lymphoma, Marginal Zone Lymphoma, Mantle Cell Lymphoma, Diffuse Large B-Cell Lymphoma, Burkitt's Lymphoma, Lymphoblastic Lymphoma, AIDS-Related B-Cell Lymphoma); 20) Non-Melanoma Skin Cancers(incl. Basal and Squamous Cell Skin Cancers, Dermatofibrosarcoma Protuberans, Merkel Cell Carcinoma); 21) Non-Small Cell Lung Cancer; 22) Occult Primary; 23) Ovarian Cancer (incl. Epithelial Ovarian Cancer, Borderline Epithelial Ovarian Cancer (Low Malignant Potential), Less Common Ovarian Histologies); 24) Pancreatic Adenocarcinoma; 25) Prostate Cancer; 26) Small Cell Lung Cancer (incl. Small Cell Lung Cancer, Lung Neuroendocrine Tumors); 27) Soft Tissue Sarcoma (incl. Soft-tissue Extremity, Retroperitoneal, Intra-abdominal Sarcoma, Desmoid); 28) Testicular Cancer; 29) Thyroid Carcinoma (incl. Nodule Evaluation, Papillary Carcinoma, Follicular Carcinoma, Hürthle Cell Neoplasm, Medullary Carcinoma, Anaplastic Carcinoma); and 30) Uterine Cancers (incl. Endometrial Cancer, Uterine Sarcoma); and all these therapies are provided herein for protection as part of this invention.

Exemplary NCCN therapies that can be used to practice this invention are categorized under "Supportive Care" include treatments for: 1) Adult Cancer Pain, 2) anti-emesis; 3) Cancer- and Treatment-Related Anemia; 4) Cancer-Related Fatigue; 5) Distress Management; 6) Fever and Neutropenia; 7) Myeloid Growth Factors; 8) Palliative Care; 9) Pediatric Cancer Pain; 10) Senior Adult Oncology; and 11) Venous Thromboembolic Disease; and all these therapies are provided herein for protection as part of this invention. Supportive care, as provided herein for protection in this invention, includes G-CSF therapy, and erythropoietin therapy.

### Group F: comprises use of or consists of use of TNF inhibitors, e.g. pentoxifylline.

Group G: comprises use of or consists of use of TNF inhibitors, e.g. anti-TNF agents, anti-TNF mAbs, pentoxifylline, thalidomide. Pentoxifylline is a PDE4 inhibitor that increases intracellular cAMP and stimulates PKA activity. Pentoxifylline is also an inhibitor of tumor necrosis factor-alpha (TNF-alpha or TNF-α). Group G also includes (comprises or consists of) all phosphodiesterase inhibitors and TNF inhibitors.

### Group H: comprises use of or consists of use of deferrioxamine.

Deferrioxamine is a chelator that has been used for than 20 years in treatment of severe ion poisoning, of disturbances in iron storage and of diseases which lead to increased iron values. Group H also includes all chelators, such as DMSA, EDTA, and EDTA.

### Group I: comprises or consists of polyunsaturated fatty acids, such as e.g. omega-3 fatty acids, including for example eicosapentaenoic acid (EPA), and docosahexaenoic acid (DHA).

In one aspect, invention provides therapeutic combinations, e.g., pharmaceutical compositions of the invention, comprising a natural oil or fatty acid, e.g., comprising an omega-3 fatty acid, a fish oil, a long-chain polyunsaturated fatty acid, an n-3 and/or n-6 highly unsaturated fatty acid, eicosapentaenoic acid (EPA), docosahexaenoic acid (DHA) or a combination thereof. In non-limiting examples, these fatty acids may be administered orally or intravenously.

### Group J: comprises or consists of α-difluoromethylornithine.

α-Difluoromethylornithine is an inhibitor of ornithine decarboxylase (an enzyme involved in polyamine synthesis), by reducing the polyamine content of mucosa. Group J also includes (comprises or consists of) all inhibitors of ornithine decarboxylase.

### Group K: comprises or consists of superoxide dismutase (SOD) and similar antioxidant compounds.

Group K comprises or consists of superoxide dismutase (SOD), catalase, superoxide dismutase (SOD)-catalase conjugates or complexes, peroxiredoxins, and peroxidases (e.g. glutathione peroxidase).

Superoxide dismutase (SOD), and similar antioxidant compounds, includes specifically:
a/ enzymes with the ability to catalyze the conversion of hydrogen peroxide into other compounds;
b/ enzymes with the ability to catalyze the decomposition of peroxides;
c/ compounds that have antioxidant activity; and
d/ compounds that promote antioxidant activity, such as co-factors, precursors and/or subunits and other enzymes in a shared biochemical reaction pathway, including, e.g. selenium, cysteine, glutamine, tryptophan, and glutathione reductase.

### Group L: comprises or consists of activators of heat shock response.

Group L comprises or consists of activators of heat shock response, e.g. Geranylgeranylacetone or gernate, zinc, tin, salicylates, dexamethasone, cocaine, nicotine, alpha-adrenergic agonist, ppar-gamma agonist, biomolecular-geldanamycin, cyclopentanone, prostanoids enprostil, paracetamol, ketotiphen, levamisole, diazepam, bromocriptine, dopamine.

### Group M: comprises or consists of drugs that reduce the inflammatory/progressive tissue damage response.

Group M comprises or consists of ACE inhibitors, ARBs, phenylbutryrate or PPAR gamma (insulin sensitizer) and/or probucol, calcitriol as alternative combinations to further stimulate protective vascular response, NSAIDs to reduce inflammation, peptide-containing compounds, inhibitors of macrophage migration inhibitory factor (MIF), inhibitors of lipoxin biosynthesis, natural alternatives to the synthetic COX inhibitors and LOX inhibitors, and other natural products (including existing, novel and yet to be discovered products) such as lipids, oils (e.g. mussel oil), peptides, and small molecules, and products derived and/or extracted from lipids or oils or peptides or small molecules.

Treating, Ameliorating, Reducing, and/or Improving Conditions, States and Disease Symptoms. The instant invention may be used to treat all possible combination and permutations of conditions, states and disease symptoms as provided herein for protection, including by way of non-limiting exemplification, those shown in Table 3, below. In alternative embodiments, the products and compositions of this invention can be used to treat, ameliorate, reduce, and/or improve, by way of non-limiting exemplification, the conditions, states and symptoms listed in Table 3, below. In alternative embodiments of this invention, the products and compositions of this invention may be used to treat ameliorate, reduce, and/or improve, all possible combinations and permutations of the conditions, states and symptoms listed in Table 3, below.

**Table 3. Exemplary conditions, states and disease symptoms ameliorate, reduce, and/or improved by compositions and methods of this invention.**

| | |
|---|---|
| 1 | anger |
| 2 | anemia |
| 3 | anorexia |
| 4 | anorexia-cachexia |
| 5 | anxiety |
| 6 | atrophy (e.g. muscle atrophy) |
| 7 | cachexia |
| 8 | cancer cachexia |
| 9 | cardiotoxicity |
| 10 | cognitive impairment |
| 11 | cytoprotection deficiency |
| 12 | depression |
| 13 | despair |
| 14 | delayed emesis |
| 15 | diarrhea |
| 16 | difficulties with activities of daily living |
| 17 | discomfort |
| 18 | emesis |
| 19 | erectile or sexual dysfunction or sexual disinterest |
| 20 | excessive sympathoneural drive |
| 21 | fatigue |
| 22 | febrile neutropenia |
| 23 | frustration |
| 24 | hair loss |
| 25 | heart failure |
| 26 | infection (in different aspects, infection types provided herein include bacterial, viral, mycobacterium, yeast and protozoan infections, and any combination thereof) |
| 27 | inflammation |
| 28 | intolerance to a medical therapy |
| 29 | lack of appetite |
| 30 | lack of energy |
| 31 | lack or motivation |
| 32 | mucositis (e.g. oral or digestive, including esophageal or intestinal mucositis) |
| 33 | myeloprotection deficiency |
| 34 | myelosupression (including neutropenia) |
| 35 | nausea |
| 36 | nephrotoxicity |
| 37 | neutropenia |
| 38 | oral mucositis |
| 39 | ototoxicity |
| 40 | pain |
| 41 | peripheral neuropathy |
| 42 | reduced physical activity |
| 43 | toxicity (including cyto-toxicity) from any chemotherapy or radiation or surgical trauma |
| 44 | wasting |
| 45 | worrying |
| 46 | stress or anxiety related to any of the above |

Additional Benefits in the Context of the Adverse Effects of Chemotherapy and Radiation. This invention provides compositions (e.g., products of manufacture) and methods (e.g., therapies), in non-limiting exemplifications: 1) to allow or enable or facilitate the tolerance and use of higher amounts or doses and/or longer durations of a medical therapy (e.g. cancer therapies or radiation therapies) than could be used without this invention; 2) to allow or enable or facilitate the tolerance and use of longer durations of a medical therapy (e.g. cancer therapies or radiation therapies) than could be used without this invention; 3) to reduce occurrences of (drug) resistance to a medical therapy (e.g. cancer chemotherapy or radiation therapy); 4) to enable dosage intensification of a medical therapy (e.g. cancer chemotherapy or radiation therapy); 5) to enable increasing the frequency of a medical therapy (e.g. cancer chemotherapy or radiation therapy); 6) to enhance patient response rates; 7) to increase patient survival; 8) to induce a tissue protective state; 9) to induce tissue regeneration; and/or 10) to induce tissue repair.

**Table 4. Exemplary uses of compositions and/or methods of the invention.**

| | |
|---|---|
| 1 | to allow or enable or facilitate the tolerance and use of higher amounts and/or doses and/or longer durations of a medical therapy (e.g. cancer therapies or radiation therapies) than could be used without this invention |
| 2 | to allow or enable or facilitate the tolerance and use of longer durations of a medical therapy (e.g. cancer therapies or radiation therapies) than could be used without this invention |
| 3 | to reduce occurrences of (drug) resistance to a medical therapy (e.g. cancer chemotherapy or radiation therapy) |
| 4 | to enable dosage intensification of a medical therapy (e.g. cancer chemotherapy or radiation therapy) |
| 5 | to enable increasing the frequency of a medical therapy (e.g. cancer chemotherapy or radiation therapy) |
| 6 | to enhance patient response rates |
| 7 | to increase patient survival |
| 8 | to induce a tissue protective state |
| 9 | to induce tissue regeneration |
| 10 | to induce tissue repair |

In alternative aspects, some ingredients of the invention (e.g., exemplary combinations of drugs) provided herein can be administered by more than one route.

By non-limiting exemplification, in alternative aspects a number of steroids can be administered by more than one route, and their mention herein under one list of examples with a common route of administration (e.g. oral administration) does not preclude the understanding that said ingredient may also be administered by a different route; this invention provides that, for each ingredient provided herein, that all possible routes of administration are provided herein; the invention also provides kits comprising the combinations of compounds (e.g., drugs, ingredients) of the invention, including ingredient forms suitable for different modes of administration (e.g., a form of a compound that can be administered orally, a form of a compound that can be administered topically, etc.). In separate aspects, this invention provides protection for each subgroup, and each subgroup may include members of another subgroup.

The invention provides alternative embodiments wherein the preparations of the invention comprise ingredients of the invention (e.g., exemplary combinations of drugs of the invention) as exemplified in Table 1, and, in one aspect, are orally ingestible. In a non-limiting exemplification these preparations can be liquids (e.g. that can be orally ingested with the help of a spoon), or powders, capsules, tablets, and pills. In non-limiting exemplifications, they can also be formed into flavored bars (e.g. similar to what bars that are marketed as "power bars", "diet bars", "energy bars", and "nutritional bars").

This invention provides that the ingredients of the invention (e.g., exemplary combinations of drugs of the invention), such as the ingredients exemplified in Table 1 (for making the preparations of the invention) are ingredients that are available commercially (and thus can be purchased or manufactured).

In alternative aspects, ingredients used to practice this invention (e.g., for use in the exemplary combinations of drugs of the invention), and in one aspect, methods and compositions of the invention used to treat, prevent and/or ameliorate wasting and/or atrophy, such as muscle atrophy, or methods and compositions of the invention used for protection against oxidants (e.g., used as an antioxidant), as discussed herein, include any single known or groups of ingredients that have a measurable positive ORAC value (or similar value using another comparable test, see below for explanation; ORAC assays and ORAC values discussed in detail, below) of at least about 1 ORAC unit/gram, or, at least about 5 ORAC units/gram, or, at least about 10 ORAC units/gram, or, at least about 20 ORAC units/gram, or, at least about 30 ORAC units/gram, or, at least about 40 ORAC units/gram, or, at least about 50 ORAC units/gram, or, at least about 60 ORAC units/gram, or, at least about 70 ORAC units/gram, or, at least about 80 ORAC units/gram, or, at least about 90 ORAC units/gram, or, at least about 100 ORAC units/gram, or, at least about 200 ORAC units/gram, or, at least about 300 ORAC units/gram, or, at least about 400 ORAC units/gram, or, at least about 500 ORAC units/gram, or, at least about 600 ORAC units/gram, or, at least about 700 ORAC units/gram, or, at least about 800 ORAC units/gram, or, at least about 900 ORAC units/gram, or, at least about 1000 ORAC units/gram, or, at least about 1000 ORAC units/gram, or, at least about 1500 ORAC units/gram, or, at least about 2000 ORAC units/gram, or, at least about 2500 ORAC units/gram, or, at least about 3000 ORAC units/gram, or, at least about 3500 ORAC units/gram, or, at least about 4000 ORAC units/gram, or, at least about 4500 ORAC units/gram, and or, at least about 5000 ORAC units/gram.

Oxygen Radical Absorbance Capacity (ORAC) is a method of measuring antioxidant capacities of different foods; correlation between the high antioxidant capacity of fruits and vegetables, and the positive impact of diets high in fruits and vegetables, is believed to play an important role in the free-radical theory of aging. An exemplary ORAC assay is: measure the oxidative degradation of a fluorescent molecule (e.g., a beta-phycoerythrin or fluorescein) after being mixed with free radical generators such as azo-initiator compounds. Azo-initiators are considered to produce peroxyl free radical by heating, which damages the fluorescent molecule, resulting in the loss of fluorescence. Antioxidant is able to protect the fluorescent molecule from the oxidative degeneration. The degree of protection can be quantified using a fluorometer. Equipment that can automatically measure and calculate the capacity is commercially available; see e.g., Ou (2001) Development and validation of an improved oxygen radical absorbance capacity assay using fluorescein as the fluorescent probe. J Agric Food Chem 49(10):4619-4626.

### Propranolol, etodolac and a cancer chemotherapy agent

This invention provides compositions (e.g., drugs, pharmaceutical compositions, formulations, preparations, kits and the like) comprising a propranolol, an etodolac (e.g., VT-122™, Vicus Therapeutics, Morristown, NJ), and a cancer chemotherapy agent or compositions; chemotherapy agents or compositions include e.g., biologics such as proteins (e.g., peptides, antibodies, cytokines and the like) and small molecules such as alkylating agents. An exemplary propranolol-etodolac-chemo drug combination of the invention comprises use of a tyrosine kinase inhibitor (TKI) as the chemotherapeutic agent. Another exemplary propranolol-etodolac-chemo drug combination of the invention comprises use of any compound that inhibits and/or prevents vascular endothelial growth factor (VEGF)-mediated angiogenesis.

For example, in one embodiment the invention provides a composition comprising (1) a propranolol (e.g., INDERAL™) or equivalents, a non selective antagonist that inhibits beta1, beta2 and beta3 subclasses of beta adrenergic receptors; (2) etodolac (e.g., LODINE™) or equivalents, an NSAID that inhibits both Cox-1 and Cox-2 enzymes, with some preference towards Cox-2 causing less gastrointestinal problems; and (3) a cancer chemotherapy agent. An exemplary embodiment is VT-122™ and a cancer chemotherapy agent. As with any composition of the invention, this embodiment can be administered by several routes, including intravenous, topical and oral, and the like. This embodiment can be used in the treatment of a cachexia, for example, a cancer cachexia, including administration to patients before, during and/or after chemotherapy, radiation therapy or a combination thereof. The compositions of the invention can also be administered to treat, ameliorate or prevent inflammation, excessive sympathoneural drive, cachexia, anorexia, anorexia-cachexia or stress or anxiety related to any of these.

In alternative embodiments, this combination is used in defined regimens with approved chemotherapies, and can increase tolerability, increase efficacy and/or reduce cost of a cancer treatment.

While the invention is not limited by any particular mechanism of action: a composition of the invention (e.g., comprising the propranolol-etodolac-chemo drug combination) can work by directly by reducing systemic inflammation and autonomic dysfunction the patient feels better and is able and willing to tolerate more cycles of a drug, e.g., a chemotherapy drug. In one embodiment, it is possible to reduce the dose of the chemotherapy and maintain high efficacy of the chemotherapy because the composition of the invention (e.g., the propranolol-etodolac-chemo drug combination of exemplary pharmaceuticals of the invention) has multiple mechanisms of action; this can allow reduction of the amount of the chemotherapy drug administered to the patient in need thereof. This can also result in reduced cost of the treatment for the patient.

### Tyrosine Kinase Inhibitors

A drug combination of the invention (e.g., the exemplary propranolol-etodolac-chemo drug combination) comprises use of a tyrosine kinase inhibitor (TKI) as the chemotherapeutic agent. In one embodiment, a drug combination of the invention of the invention comprises VT-122™ with targeted therapy such as a protein kinase inhibitor, e.g., a tyrosine kinase inhibitor (TKI). In alternative embodiment, TKIs that are specific for certain kinases are used, or, TKIs that are broad protein kinase inhibitors, e.g., are multi-kinase inhibitors, are used. In alternative embodiments, TKIs that can interfere epidermal growth factor receptor (EGFR) activity are used; e.g., monoclonal antibodies and small-molecule inhibitors of protein kinases, including EGFR.

Use of a drug combination of the invention will allow a decreased amount of EGFR inhibitor be used; this will decrease, ameliorate or eliminate the rash that occurs upon use of protein kinase inhibitors (it is estimated that rash occurs in 60% to 80% of patients, with the majority experiencing a mild to moderate rash; severe symptoms can necessitate dose alterations occur in up to 20% of the patients with rash). Use of a drug combination of the invention, e.g., an exemplary propranolol-etodolac-chemo drug combination, will allow high clinical benefit with reduced toxicity.

In alternative embodiments, because expression of EGFR is detected in many human cancers including those of the head and neck, colon, and rectum, compositions of the invention (e.g., comprising the propranolol-etodolac-chemo drug combination) are used to treat head and neck, colon, and rectum cancers.

In alternative embodiments, because vascular endothelial growth factor (VEGF)-mediated angiogenesis is thought to play a critical role in tumor growth and metastasis, compositions of the invention (e.g., comprising the propranolol-etodolac-chemo drug combination) are used in conjunction with anti-VEGF therapies, e.g., either as an alternative or as an adjunct to conventional chemo or radiation therapy. Compositions of the invention can be used with any technique that can block the VEGF pathway, including neutralizing monoclonal antibodies against VEGF or its receptor; small molecule tyrosine kinase inhibitors of VEGF receptors; or soluble VEGF receptors which act as decoy receptors for VEGF. Compositions of the invention can be used with any tyrosine kinase inhibitor to treat cancer, for example:

| | | | |
|---|---|---|---|
| Name | Target | Company | Class |
| bevacizumab (e.g., AVASTIN™) | VEGF | Genentech | Monoclonal antibody |
| BIBW 2992 | EGFR and Erb2 | Boehringer Ingelheim | Small molecule |
| cetuximab (e.g., ERBITUX™) | Erb1 | Imclone/BMS | Monoclonal antibody |
| imatinib (e.g., GLEEVEC™) | Bcr-Abl | Novartis | Small molecule |
| trastuzumab (e.g., HERCEPTIN™) | Erb2 | Genentech/Roche | Monoclonal antibody |
| gefitinib (e.g., IRESSA™) | EGFR | AstraZeneca | Small molecule |
| ranibizumab (e.g., LUCENTIS™) | VEGF | Genentech | Monoclonal antibody |
| pegaptanib (e.g., MACUGEN™) | VEGF | OSI/Pfizer | Small molecule |
| sorafenib (e.g., NEXAVAR™) | TKI | Onyx/Bayer | Small molecule |
| dasatinib (e.g., BMS-354825™) | TKI | BMS | Small molecule |
| sunitinib (e.g., SUTENT)™ | TKI | Pfizer | Small molecule |
| erlotinib (e.g., TARCEVA™) | Erb1 | Genentech/Roche | Small molecule |
| pazopanib | TKI | GSK | Small molecule |
| nilotinib (e.g., TASIGNA™) | Bcl-Abr | Novartis | Small molecule |
| lapatinib (e.g., TYKERB™) | Erb1/Erb2 | GSK | Small molecule |
| panitumumab (e.g., VECTIBIX™) | EGFR | Amgen | Monoclonal antibody |
| bandetinib | RET/VEGFR | AstraZeneca | Small molecule |
| brivanib | VEGF/FGF | BMS | Small Molecule |
| E7080™ | Multiple targets: RET/VEGFR | Esai | Small molecule |

**Compositions of the invention can be used with any protein kinase inhibitor, for example:**

| **Drug (*Trade name***) | **Class** | **Target** | **Indication** | **Dose** |
|---|---|---|---|---|
| cetuximab (e.g., ERBITUX™) ImClone, Bristol-Myers Squibb | mAb | EGFR | In combination with radiation therapy for locally or regionally advanced HNSCC | Intravenous 400 mg/m² initial dose then 250 mg/m² weekly |
| | | | Recurrent or metastatic HNSCC progressing after platinum-based therapy | |
| | | | Single agent in metastatic CRC (EGFR-expressing) after failure of irinotecan- and oxaliplatin-based regimens | |
| | | | Metastatic CRC (EGFR-expressing) in combination with irinotecan for irinotecan-refractory patients | |
| erlotinib (e.g., TARCEVA™) Genentech, OSI Pharmaceuticals | TKI | EGFR | Second-line therapy in locally advanced or metastatic non-small cell lung cancer (NSCLC) | Oral 150 mg daily |
| | | | First line, in combination with gemcitabine in locally advanced or metastatic pancreatic cancer | |
| gefitinib (e.g., IRESSA™) AstraZeneca | TKI | EGFR | Monotherapy for the treatment of patients with advanced or metastatic NSCLC who are benefiting or have benefited from gefitinib | Oral 250 mg daily |
| lapatinib (e.g., TYKERB™) GSK | TKI | EGFR/HER2 | In combination with capecitabine for the treatment of patients with advanced or metastatic HER2-overexpressing breast cancer who have received prior treatment with an anthracycline, a taxane, and trastuzumab | Oral 1250 mg daily for 21 days then 1 week off |
| panitumumab (e.g., VECTIBIX™) Amgen | mAb | EGFR | Metastatic colorectal carcinoma (EGFR-expressing) after treatment with fluoropyrimidine-, oxaliplatin-, and irinotecan-containing chemotherapy regimens | Intravenous 6 mg/kg every 14 days |

### Effect on Cancer

In alternative embodiments, compositions, uses and methods of the invention (including e.g., compositions comprising the propranolol-etodolac-chemo drug combination), are used in the treatment or amelioration of (including the primary treatment or amelioration of) any cancer or disease or conditions caused by dysfunctional cells. In alternative embodiments, compositions, uses and methods of the invention are used prophylactically (as a preventative treatment).

While the invention is not limited by any particular mechanism of action: a composition of the invention (e.g., comprising the propranolol-etodolac-chemo drug combination) can work by: switching the tumor from a state of auto-promotion (malignancy) to a state of wound healing, or switching the tumor from a developmental state to a normal tissue regulated state. A composition or method of the invention also can ameliorate a cachexia, thereby allowing extension of the duration of treatment. While the invention is not limited by any particular mechanism of action: a composition or method of the invention also can switch tumor-associated cells from wound healing and developmental states to infection and tissue maintenance states, this "switching" leading to further eradication of the tumor by the patient's immune system.

While the invention is not limited by any particular mechanism of action: a composition or method of the invention (e.g., comprising the propranolol-etodolac-chemo drug combination) also can reduce catecholamine levels; an increase in catecholamine levels can increase VEGF secretion by human cancer cells and enhance tumor cell invasion. Catecholamine levels are reduced by beta adrenergic blockade.

While the invention is not limited by any particular mechanism of action: a composition of the invention (e.g., comprising the propranolol-etodolac-chemo drug combination) also can have a direct impact on the cancer through its NSAID, e.g., etodolac, component by regulating angiogenesis, inducing apoptosis, prevention of metastases, reprioritization of energy utilization, reactivation of the immune system, cancer chemoprevention, radiosensitivity and/or repression of catenin function (catenin is central to the invasive cellular phenotype). In one embodiment, the specific NSAID etodolac may induce apoptosis, prevent metastases or act as a cytotoxic anticancer agent.

While the invention is not limited by any particular mechanism of action: a composition of the invention (e.g., comprising the propranolol-etodolac-chemo drug combination), by combined use of beta-blockers and NSAIDs, can modulate a dysregulated neuroendocrine-immune system and its associated clinical manifestations of cachexia, thus being beneficial for cancer patients.

In alternative embodiments, compositions of the invention (including e.g., compositions comprising the propranolol-etodolac-chemo drug combination), are used in the treatment or amelioration of (including the primary treatment or amelioration of): 1) Acute Myeloid Leukemia; 2) Bladder Cancer (incl. Bladder Cancer, Upper Tract Tumors, Urothelial Carcinoma of the Prostate); 3) Bone Cancer (including Chondrosarcoma, Ewing's Sarcoma, Osteosarcoma); 4) Breast Cancer (incl. Noninvasive, Invasive, Phyllodes Tumor, Paget's Disease, Breast Cancer During Pregnancy); 5) Central Nervous System Cancers (incl. Adult Low-Grade Infiltrative Supratentorial Astrocytoma/Oligodendroglioma, Adult Intracranial Ependymoma, Anaplastic Astrocytoma/Anaplastic Oligodendroglioma/Glioblastoma Multiforme, Limited (1-3) Metastatic Lesions, Multiple (>3) Metastatic Lesions, Carcinomatous Lymphomatous Meningitis, Non-immunosuppressed Primary CNS Lymphoma, Metastatic Spine Tumors, Principles of Brain Tumor Therapy); 6) Cervical Cancer; 7) Chronic Myelogenous Leukemia; 8) Colon/Rectal Cancer (incl. Colon Cancer, Rectal Cancer, Anal Carcinoma); 9) Esophageal Cancer; 10) Gastric Cancer; 11) Head and Neck Cancers (incl. Ethmoid Sinus Tumors, Maxillary Sinus Tumors, Salivary Gland Tumors, Cancer of the Lip, Cancer of the Oral Cavity, Cancer of the Oropharynx, Cancer of the Hypopharynx, Occult Primary, Cancer of the Glottic Larynx, Cancer of the Supraglottic Larynx, Cancer of the Nasopharynx, Advanced Head and Neck Cancer); 12) Hepatobiliary Cancers (incl. Hepatocellular Carcinoma, Gallbladder Cancer, Intrahepatic Cholangiocarcinoma, Extrahepatic Cholangiocarcinoma); 13) Hodgkin Disease/Lymphoma; 14) Kidney Cancer; 15) Melanoma; 16) Multiple Myeloma (incl. Multiple Myeloma, Waldenström's Macroglobulinemia); 17) Myelodysplastic Syndromes; 18) Neuroendocrine Tumors (incl. Multiple Endocrine Neoplasia: Type 1, Multiple Endocrine Neoplasia: Type 2, Carcinoid Tumors, Islet Cell Tumors, Pheochromocytoma, Poorly Differentiated/Small Cell/Atypical Lung Carcinoids); 19) Non-Hodgkin's Lymphoma (incl. Chronic Lymphocytic Leukemia/Small Lymphocytic Lymphoma, Follicular Lymphoma, Marginal Zone Lymphoma, Mantle Cell Lymphoma, Diffuse Large B-Cell Lymphoma, Burkitt's Lymphoma, Lymphoblastic Lymphoma, AIDS-Related B-Cell Lymphoma); 20) Non-Melanoma Skin Cancers(incl. Basal and Squamous Cell Skin Cancers, Dermatofibrosarcoma Protuberans, Merkel Cell Carcinoma); 21) Non-Small Cell Lung Cancer; 22) Occult Primary; 23) Ovarian Cancer (incl. Epithelial Ovarian Cancer, Borderline Epithelial Ovarian Cancer (Low Malignant Potential), Less Common Ovarian Histologies); 24) Pancreatic Adenocarcinoma; 25) Prostate Cancer; 26) Small Cell Lung Cancer (incl. Small Cell Lung Cancer, Lung Neuroendocrine Tumors); 27) Soft Tissue Sarcoma (incl. Soft-tissue Extremity, Retroperitoneal, Intra-abdominal Sarcoma, Desmoid); 28) Testicular Cancer; 29) Thyroid Carcinoma (incl. Nodule Evaluation, Papillary Carcinoma, Follicular Carcinoma, Hürthle Cell Neoplasm, Medullary Carcinoma, Anaplastic Carcinoma); and 30) Uterine Cancers (incl. Endometrial Cancer, Uterine Sarcoma).

### Methods of administration

This invention provides compositions (e.g., the exemplary combinations of drugs of the invention), e.g., pharmaceutical compositions, formulations, products of manufacture, preparations and kits, that can be administered by several routes, including intravenous, topical and oral, and the methods for administering them. In separate embodiments, this invention provides forms of compositions, preparations and kits that can be administered by inhalation, infusion or injection, (e.g., intraperitoneal, intramuscular, subcutaneous, intra-aural, intra-articular, intra-mammary, etc.), topical application (e.g., on areas, such as eyes, ears, skin or on afflictions such as wounds, burns, etc.), and by absorption through epithelial or mucocutaneous linings (e.g. vaginal and other epithelial linings, gastrointestinal mucosa, etc.). Methods are known for making the compositions of the invention (e.g., the pharmaceutical compositions, formulations, products of manufacture, preparations, kits and the like comprising the therapeutic combinations of drugs of the invention) that are suitable for each of these methods of administration as well as other methods of administration that are know in the art.

For example, in alternative embodiments, this invention provides compositions, preparations and kits in liquid forms that can be administered orally. The compositions, preparations and kits can be also prepared as capsules, gels, geltabs, tablets, powders, sprays, aerosols, pellets (e.g. for animal consumption), suppositories, or creams and ointments. The compositions, preparations and kits can be also prepared as physiological solutions suitable for I.V. administration or other parenteral administration.

In alternative embodiments, this invention also provides all the possible combinations of component quantities that are possible (e.g. the total of all the components does not surpass 100% of the relevant total dosage compositions, preparations and kits, and admixing or solubility limitations are not exceeded).

In alternative embodiments, a multi-ingredient kit, as provided herein (see e.g., section on packaging, below) comprises two, three, four or five or more ingredients in approximately equal amounts. An amount may be determined, e.g. by mass or by weight or by molar amount. In alternative embodiments, a multi-ingredient kit comprises two, three, four or five or more ingredients in unequal amounts. In alternative embodiments, a multi-ingredient kit comprises two, three, four or five or more ingredients in approximately equal amounts as well as one or more ingredients that are not in unequal amounts.

In alternative embodiments, a multi-ingredient kit of the invention may contain comprises two, three, four or five or more ingredients in approximately equimolar amounts. In alternative embodiments, a multi-ingredient kit comprises two, three, four or five or more ingredients that are not in equimolar amounts. In alternative embodiments, a multi-ingredient kit comprises two, three, four or five or more ingredients that are in approximately equimolar amounts as well as one or more ingredients that are not in equimolar amounts.

In alternative embodiments, a multi-ingredient kit comprises two, three, four or five or more ingredients that are admixed. In alternative embodiments, a multi-ingredient kit comprises two, three, four or five or more ingredients that are not admixed. In alternative embodiments, a multi-ingredient kit comprises two, three, four or five or more ingredients that are partially admixed. In alternative embodiments, a multi-ingredient kit comprises two, three, four or five or more ingredients that are at least partially admixed, as well as one or more ingredients that are not admixed. In alternative embodiments, an ingredient in a multi-ingredient kit of the invention can be formulated or manufactured in a liquid form, e.g., a form that can be administered orally or parenterally.

An ingredient or ingredients (e.g., a drug or a therapeutic combination of drugs of this invention) in a multi-ingredient kit of the invention can be formulated or manufactured in any delivery form, e.g., as capsules, tablets, powders, sprays, aerosols, pellets (e.g. for animal consumption), suppositories, or creams and ointments. An ingredient or ingredients also can be formulated or manufactured in delivery forms such as physiological (e.g., saline) solutions suitable for I.V. administration or other parenteral administration.

An ingredient or ingredients (e.g., a drug or a therapeutic combination of drugs of this invention) in a multi-ingredient kit of the invention can be separated by physical compartmentalization; e.g. in separate compartments that are part of said kit, where said kit is a multi-compartment kit. Alternatively, separate compartments, e.g., as found in a "blister pack" type of packaging, may contain different ingredients (e.g., a therapeutic combination of drugs of this invention), as discussed below. In one embodiment, the composition or product of manufacture is contained in a multiparticulate and/or a solid dispersion formulation, e.g., as described in, e.g., U.S. Patent App. Pub. No. 20080118560, e.g., comprising a hydrophobic matrix former which is a water-insoluble, non-swelling amphiphilic lipid; and a hydrophilic matrix former which is a meltable, water-soluble excipient.

In one embodiment, the composition or product of manufacture is contained in tablets, pills, capsules, troches, and the like comprising any combination of a binder, e.g., as a starch, polyvinyl pyrrolidone, gum tragacanth or gelatin; a filler, such as microcrystalline cellulose or lactose; a disintegrating agent, such as crospovidone, sodium starch glycolate, corn starch, and the like; a lubricant, such as magnesium stearate, stearic acid, glyceryl behenate; a glidant, such as colloidal silicon dioxide and talc; a sweetening agent, such as sucrose or saccharin, aspartame, acesulfame-K; and/or flavoring agent, such as peppermint, methyl salicylate, or orange flavoring. When the dosage unit form is a capsule, it also can comprise a liquid carrier, such as a fatty oil.

In one aspect, a composition or product of manufacture of the invention comprises (or is contained or packaged in) unit dosage formulations having a coating, e.g., a coat comprising a sugar, shellac, sustained and/or other enteric coating agents, or any pharmaceutically pure and/or nontoxic agents.

In one aspect, a composition or product of manufacture of the invention comprises (or is contained or packaged in) unit dosage formulations, wherein each different compound of the composition or product of manufacture is contained in a different layer of a pill, tablet or capsule, e.g., as described in USPN 7,384,653, e.g., having an outer base-soluble layer and an inner acid-soluble layer.

In one aspect, a composition or product of manufacture of the invention comprises (or is contained or packaged in) unit dosage formulations, wherein each different compound of the composition or product of manufacture is contained in a liquid or a gel of different viscosity, e.g., described in U.S. Patent App. Pub. No. 20050214223.

In one aspect, a composition or product of manufacture of the invention comprises (or is contained or packaged in) unit dosage formulations having reduced abuse potential, e.g., as described in U.S. Patent App. Pub. No. 20040228802, e.g., comprising a bittering agent, a bright deterrent/indicator dye, or a fine insoluble particulate matter.

In alternative embodiments, the invention provides methods and compositions to treat, ameliorate, reduce, and/or improve a conditions, state and/or disease symptom. This invention further provides myelo-protective and/or cyto-protective therapies that are serviceable for treating, ameliorating, reducing, and/or improving a condition, a state and/or a disease symptom coincident with mucositis and/or present in patient who experiences mucositis or in a patient who is expected to experience mucositis.

### Dosaging

The compositions (e.g., pharmaceuticals, formulations) of the invention can be administered for prophylactic and/or therapeutic treatments. In therapeutic applications, compositions are administered to a subject already suffering from a condition, infection or disease (e.g., cancer) in an amount sufficient to cure, alleviate or partially arrest the clinical manifestations of the condition (e.g., cachexia), infection or disease and its complications (a "therapeutically effective amount"). For example, in alternative embodiments, compositions of the invention (e.g., pharmaceuticals) are administered in an amount sufficient to treat, prevent and/or ameliorate normal, dysfunction (e.g., abnormally proliferating) blood vessels, including endothelial and/or capillary cell growth; including neovasculature related to (within, providing a blood supply to) hyperplastic tissue, a granuloma or a tumor. In alternative embodiments, compositions of the invention (e.g., pharmaceuticals) are administered in an amount sufficient to treat, prevent and/or ameliorate mucositis and/or cachexia. The amount of composition, e.g., as a pharmaceutical, adequate to accomplish this is defined as a "therapeutically effective dose." The dosage schedule and amounts effective for this use, i.e., the "dosing regimen," will depend upon a variety of factors, including the stage of the disease or condition, the severity of the disease or condition, the general state of the patient's health, the patient's physical status, age and the like. In calculating the dosage regimen for a patient, the mode of administration also is taken into consideration.

The dosage regimen also takes into consideration pharmacokinetics parameters well known in the art, i.e., the active agents' rate of absorption, bioavailability, metabolism, clearance, and the like (see, e.g., Hidalgo-Aragones (1996) J. Steroid Biochem. Mol. Biol. 58:611-617; Groning (1996) Pharmazie 51:337-341; Fotherby (1996) Contraception 54:59-69; Johnson (1995) J. Pharm. Sci. 84:1144-1146; Rohatagi (1995) Pharmazie 50:610-613; Brophy (1983) Eur. J. Clin. Pharmacol. 24:103-108. Details on techniques for formulation and administration are well described in the scientific and patent literature, see, e.g., the latest edition of Remington's Pharmaceutical Sciences, Maack Publishing Co, Easton PA ("Remington's"). The state of the art allows the clinician to determine the dosage regimen for each individual patient, active agent and disease or condition treated. Guidelines provided for similar compositions used as pharmaceuticals can be used as guidance to determine the dosage regiment, i.e., dose schedule and dosage levels, administered practicing the methods of the invention are correct and appropriate.

Single or multiple administrations of formulations can be given depending on the dosage and frequency as required and tolerated by the patient. The formulations should provide a sufficient quantity of active agent to effectively treat, prevent or ameliorate a conditions, diseases or symptoms as described herein. For example, an exemplary pharmaceutical formulation for oral administration is in a daily amount of between about 0.1 to 0.5 to about 20, 50, 100 or 1000 or more µg per kilogram of body weight per day. In an alternative embodiment, dosages are from about 1 mg to about 4 mg per kg of body weight per patient per day are used. Lower dosages can be used, in contrast to administration orally, into the blood stream, into a body cavity or into a lumen of an organ. Substantially higher dosages can be used in topical or oral administration or administering by powders, spray or inhalation. Actual methods for preparing parenterally or non-parenterally administrable formulations will be known or apparent to those skilled.

In one aspect the therapeutic combinations of the invention comprise formulations having the dosage of etodolac ranges from about 200 mg to 400 mg a day, or, about 10, 15, 20, 25, 30, 35, 40, 45, 50, 75, 80, 85, 90, 100, 150, 200, 250, 300, 350, 400, 450, 500, 600, 700, 800, 900 or 1000 mg or more. In alternative embodiments, the dosage of propranolol can range from 10 to 320 mg per day based on heart rate and blood pressure of the individual; or, can be about 10, 15, 20, 25, 30, 35, 40, 45, 50, 75, 80, 85, 90, 100, 150, 200, 250, 300, 350, 400, 450, 500, 600, 700, 800, 900 or 1000 mg or more; wherein the invention includes all combinations of these exemplary dosages. In one aspect, the methods of the invention comprise administration of these therapeutic combinations of the invention; wherein practicing the methods of the invention can include use of any or all combinations of these exemplary dosages.

For example, therapeutic combinations of the invention can be packaged in dosages that match a chrono-dosing regimen to match an optimal dose for the time of day, or the day or month (as any therapeutic drug combination of the invention can be applied once, twice, three or four times, or more, a day or a week or a month, depending on the patient and the indication the for which the drugs are being administered).

For example, in one aspect the therapeutic combinations of the invention are packaged in dosages that match a chrono-dosing regimen comprising: (a) in the AM, 20 mg propranolol, 200mg etodolac; in the afternoon, 10 mg propranolol, 200 mg etodolac; in the PM, 10 mg propranolol, 400 mg etodolac; (b) in the AM 40 mg propranolol, 200 mg etodolac; in the afternoon 20 mg propranolol, 200 mg etodolac; in the evening, 20 mg propranolol, 400 mg etodolac; (c) in the AM 80 mg propranolol, 200 mg etodolac; in the afternoon 40 mg propranolol, 200 mg etodolac, in the evening 40 mg, etodolac; or (d) a dose escalation comprising a regimen of (a) to (b) to (c); or any equivalents.

For example, in one aspect the drugs are packaged in dosages that match a chrono-dosing regimen comprising:
Start: AM, 20 mg propranolol, 200mg etodolac; afternoon, 10 mg propranolol, 200 mg etodolac; PM 5 mg propranolol, 400 mg etodolac;
Dose Escalation 1: AM 40 mg propranolol, 200 mg etodolac; afternoon 20 mg propranolol, 200 mg etodolac; evening, 10 mg propranolol, 400 mg etodolac;
Dose escalation 2: AM 80 mg propranolol, 200 mg etodolac; afternoon 40 mg propranolol, 200 mg etodolac, evening 20 mg, etodolac.

For example, in one aspect of the invention the drugs are formulated for administration once a day, b.i.d. or t.i.d, or four times a day, or more, or for weekly, or biweekly, or monthly administration (in any combination, as described herein, particularly to accommodate chronodosaging).

In one aspect of the invention the drugs are dosaged as set forth in any one of exemplary ingredient combinations 1 to 90.

In one aspect of the therapeutic combination of the invention the drugs are formulated for administration intravenously, topically, orally, by inhalation, by infusion, by injection, by inhalation, intraperitoneally, intramuscularly, subcutaneously, intra-aurally, for intra-articular administration, for intra-mammary administration, for topical administration or for absorption through epithelial or mucocutaneous linings.

In one aspect the therapeutic combinations of the invention, including the compositions and products of manufacture of the invention, further comprise instructions for use, e.g., in the treatment of cachexia, the treatment of anorexia or anorexia-cachexia, and stress or anxiety related thereto. In one aspect the cachexia is defined as at least two of the symptoms selected from the group consisting of: 1) a hyper-inflammatory state, 2) altered hormone levels and cytokine levels; 3) decreased heart rate variability; 4) weight loss, and 5) increased heart rate, wherein optionally the increased heart rate is having a sustained elevated heart rate of at least about 6 bpm; or, the cachexia is defined by an individual having at least a sustained elevated heart rate of at least about 6 bpm and weight loss.

In one embodiment, an extended release formulation (e.g., of propranolol and/or etodolac) is used (e.g., both extended release etodolac and propranolol can be used). In one embodiment, a simple dosage regimen is used: an extended release formulation used once a day; e.g., an extended release propranolol and extended release etodolac given once/day. In one embodiment, a more complex dosage regimen is used: for example, high dosage of one drug in the combination in the morning with progressively lower dosages administered during the day; for example, an exemplary dosage regimen comprises administration of high propranolol in the morning and lower at night, but with steady levels of etodolac throughout the day. In one embodiment, high dosages of propranolol are given in the morning and lower dosages of propranolol are given at night; with low dosages of etodolac in the morning and higher levels of etodolac at night. For each of the above exemplary embodiments, there would be multiple dose levels; for example:

**Example 1 -Level dose propranolol and etodolac**

| | Propranolol | Etodolac |
|---|---|---|
| AM | 60 mg LA | 1200 mg XR |
| Noon | | |
| PM | | |
| Bed | | |

| | | |
|---|---|---|
| IR = immediate release LA=extended release formulation (LA, XL, XR, etc...) | | |

**Example 2 -Level dose propranolol and etodolac**

| | Propranolol | Etodolac |
|---|---|---|
| AM | 20 mg IR | 200 mg IR |
| Noon | 20 mg IR | 200 mg IR |
| PM | 20 mg IR | 200 mg IR |
| Bed | 20 mg IR | 200 mg IR |

| | | |
|---|---|---|
| IR = immediate release LA=extended release formulation (LA, XL, XR, etc...) | | |

**Example 3 - High propranolol AM, level etodolac concentrations**

| | Propranolol | Etodolac |
|---|---|---|
| AM | 20 mg IR+60 mg LA | 1200 mg XR |
| Noon | | |
| PM | | |
| Bed | | |

**Example 4 - High propranolol AM, increasing etodolac concentrations**

| | Propranolol | Etodolac |
|---|---|---|
| AM | 40 mg IR | 200 mg IR |
| Noon | 20 mg IR | 200 mg IR |
| PM | 10 mg IR | 200 mg IR |
| Bed | 10 mg IR | 200 mg IR |

**Example 5 - High propranolol AM, increasing etodolac concentrations**

| | Propranolol | Etodolac |
|---|---|---|
| AM | 20 mg IR+60 mg LA | 1200 mg XR |
| Noon | | |
| PM | | 200 mg IR |
| Bed | | |

All of the above can have dose increased 50% and 100%. Alternatively, the combinations can be used at a lower (10 % less, 20 % less, 30 % less, 40 % less, 50 % less, or 60 % or more less) dosage.

For example, exemplary dosages that can be used when practicing the compositions and/or methods of this invention include:

| Oncology Drug | Standard Dose | Low Dose |
|---|---|---|
| sorafenib (NEXAVAR™) | 800 mg bid | 400 mg qd |

| | | |
|---|---|---|
| sunitinib (e.g., SUTENT)™ | 50 mg qd | 37.5 mg or 25 mg qd |
| erlotinib (e.g., TARCEVA™) | 100 or 150 mg qd | 75 or 100 mg qd |
| imatinib (e.g., GLEEVEC™) | 400 mg qd or 800 mg bid | 200 mg to 400 mg qd |
| lapatinib (e.g., TYKERB™) | 1250 mg qd | 750 mg qd |
| bevacizumab (e.g., AVASTIN™) | 5 - 15 mg/kg IV | 5 - 10 mg/kg IV |
| trastuzumab (e.g., HERCEPTIN™) | 4 - 8 mg/kg IV | 4 mg/kg IV |
| cetuximab (e.g., ERBITUX™) | 400 mg/m2 IV | 200 mg/m2 IV |

### Packaging combinations of compounds of the invention

The invention provides compositions, including preparations, formulations and/or kits, comprising combinations of ingredients (e.g., the combinations of drugs of the invention), for use, e.g., as therapies for treating, preventing, ameliorating or improving conditions, states, disease symptoms, and unwanted side effects including e.g.,: 1/ anger; 2/ anemia; 3/ anorexia; 4/ anorexia-cachexia; 5/ anxiety; 6/ atrophy (e.g. muscle atrophy); 7/ cachexia, including cancer cachexia; 8/ cancer and any conditions caused by dysfunctional cells; 9/ cardiotoxicity; 10/ cognitive impairment; 11/ cytoprotection deficiency; 12/ depression; 13/ despair; 14/ delayed emesis; 15/ diarrhea; 16/ difficulties with activities of daily living; 17/ discomfort; 18/ emesis; 19/ erectile or sexual dysfunction or sexual disinterest; 20/ excessive sympathoneural drive; 21/ fatigue; 22/ febrile neutropenia; 23/ frustration; 24/ hair loss; 25/ heart failure; 26/ infection (in different aspects, infection types provided herein include bacterial, viral, mycobacterium, yeast and protozoan infections, and any combination thereof); 27/ inflammation; 28/ intolerance to a medical therapy; 29/ lack of appetite; 30/ lack of energy; 31/ lack or motivation; 32/ mucositis (e.g. esophageal or intestinal mucositis); 33/ myeloprotection deficiency; 34/ myelosupression (including neutropenia); 35/ nausea; 36/ nephrotoxicity; 37/ neutropenia; 38/ oral mucositis; 39/ ototoxicity; 40/ pain; 41/ peripheral neuropathy; 42/ reduced physical activity; 43/ toxicity (including cyto-toxicity) from any chemotherapy or radiation or surgical trauma; 44/ wasting; 45/ worrying; 46/ stress or anxiety related to any of the above.

In alternative embodiment, the packaging is a critical component for the success of the drug treatment because the therapeutic combination of drugs of the invention cannot be successfully administered to a stressed, challenged or non-compliant patient population in a conventional formal or formulation combination. However, in alternative embodiments a stressed, challenged or non-compliant patient population can be properly administered a combination of drugs by using compositions of this invention, e.g., therapeutic combination of drugs of the invention packaged for usage compliance by a stressed, challenged or non-compliant patient population.

In alternative embodiments, the stressed, challenged or non-compliant patient population comprises a cancer patient population; or a patient population having mild or severe mental retardation, slow cognition, dementia, senility, Alzheimer's disease, traumatic brain injury, chemical brain damage or a mental disease; or post-traumatic stress disorder, traumatic war neurosis, post-traumatic stress syndrome (PTSS) or a physical disability or blindness. The mental disease can be a dissociative disorder, an obsessive-compulsive disorder, a delusional disorder, a schizophrenia, a mania, a panic disorder, depression, dyslexia or a learning disability.

In alternative embodiments, each member of the combination of ingredients is manufactured in a separate package, kit or container; or, all or a subset of the combinations of ingredients are manufactured in a separate package or container. In alternative aspects, the package, kit or container comprises a blister package, a clamshell, a tray, a shrink wrap and the like.

In alternative embodiments, the invention provides a blister package, a clamshell, a tray or a shrink wrap; and in separate exemplifications, said blister package, clamshell, tray or shrink wrap may comprise every possible combination and permutation of two members, three members, four members, five members, etc and up to and including at least 100 (one hundred) members selected from any of Group A, Group B, Group C, and Group D (as shown in Table 1), where: of Group A comprises or consists of, or Group A comprises use of or consists of use of, geranylgeranyl compounds, including for example, geranylgeranyl acetone (GGA) and analogs of geranylgeranyl acetone (GGA); Group B comprises use of or consists of use of Angiotensin Converting Enzyme Inhibitors (ACE Inhibitors or ACE-Is); Group C comprises use of or consists of use of Angiotensin Receptor Blockers (ARBs); Group D comprises use of or consists of use of Peroxisome Proliferator-Activated Receptor (PPAR) ligands, e.g. agonists, such as PPAR alpha agonists, PPAR gamma agonists, PPAR alpha/gamma dual agonists, and other PPAR ligands (e.g. PPAR delta ligands); Group E comprises use of or consists of use of non-steroidal anti-inflammatory drugs (NSAIDs); Group F consists of NCCN therapies; Group G comprises use of or consists of use of TNF inhibitors, e.g. pentoxifylline; Group H comprises use of or consists of use of deferrioxamine; Group I comprises polyunsaturated fatty acids, such as e.g. omega-3 fatty acids, including for example eicosapentaenoic acid (EPA), and docosahexaenoic acid (DHA); Group J comprises α-difluoromethylornithine; Group K comprises superoxide dismutase (SOD) and similar antioxidant compounds; Group L comprises activators of heat shock response; and Group M comprises drugs that reduce the inflammatory/progressive tissue damage response.

In alternative embodiments, the package, kit or container comprises a "blister package" (also called a blister pack, or bubble pack). In one aspect, the blister package is made up of two separate elements: a transparent plastic cavity shaped to the product and its blister board backing. These two elements are then joined together with a heat sealing process which allows the product to be hung or displayed. Exemplary types of "blister packages" include: Face seal blister packages, gang run blister packages, mock blister packages, interactive blister packages, slide blister packages.

In alternative embodiments, blister packs, clamshells or trays or any equivalent form of packaging used for goods is used to practice this invention, e.g., to package and deliver the therapeutic combination of drugs of this invention. In alternative embodiments, the invention provides for blister packs, clamshells or trays comprising a composition; e.g., a multi-ingredient combination of drugs of the invention, or a combination of active ingredients of the invention. In alternative embodiments, blister packs, clamshells or trays used to practice this invention are designed to be non-reclosable, so consumers can tell if a package has already opened. In alternative aspects, these embodiments are used to package drugs where product tampering is a consideration, such as the public sale of pharmaceuticals, including the therapeutic combinations of this invention. In alternative aspects, these embodiments are used to package drugs (e.g., therapeutic combinations of this invention) where child-resistant tampering is desired or required. In one aspect, a blister pack of the invention comprises a moulded PVC base, with raised areas (the "blisters") to contain the tablets, pills, etc. comprising the combinations of the invention, covered by a foil laminate. Tablets, pills, etc. can be removed from the pack either by peeling the foil back or by pushing the blister to force the tablet to break the foil. In one aspect, a specialized form of a blister pack that is a strip pack is used. In one aspect, in the United Kingdom, blister packs adhere to British Standard 8404.

In one aspect, a blister pack of the invention also comprises a method of packaging where the compositions comprising combinations of ingredients of the invention are contained in-between a card and a clear PVC. The PVC can be transparent so the item (pill, tablet, geltab, etc.) can be seen and examined easily; and in one aspect, can be vacuum-formed around a mould so it can contain the item snugly and have room to be opened upon purchase. In one aspect, the card is brightly colored and designed depending on the item (pill, tablet, geltab, etc.) inside, and the PVC is affixed to the card using pre-formed tabs where the adhesive is placed. The adhesive can be strong enough so that the pack may hang on a peg, but weak enough so that this way one can tear open the join and access the item. Sometimes with large items or multiple enclosed pills, tablets, geltabs, etc., the card has a perforated window for access. In one aspect, more secure blister packs, e.g., for items such as pills, tablets, geltabs, etc. of the invention are used, and they can comprise of two vacuum-formed PVC sheets meshed together at the edges, with the informative card inside. These can be hard to open by hand, so a pair of scissors or a sharp knife may be required to open.

In one aspect, blister packaging comprises at least two, three, four or five or more components (e.g., is a multi-ingredient combination of drugs of the invention): a thermoformed "blister" which houses the product (e.g., a pharmaceutical combination of the invention), and then a "blister card" that is a printed card with an adhesive coating on the front surface. During the assembly process, the blister component, which is most commonly made out of PVC, can be attached to the blister card using a blister machine. This machine introduces heat to the flange area of the blister which activates the glue on the card in that specific area and ultimately secures the PVG blister to the printed blister card. The thermoformed PVG blister and the printed blister card can be as small or as large as you would like, but there are limitations and cost considerations in going to an oversized blister card. Conventional blister packs can also be sealed (e.g., using an AERGO 8 DUO™, SCA Consumer Packaging, Inc., DeKalb IL) using regular heat seal tooling. This alternative aspect, using heat seal tooling, can seal common types of thermoformed packaging.

In one aspect, a composition or product of manufacture of the invention comprises (or is contained or packaged in) a blister pack, wherein the combination of drugs is formulated for unit dosage administration to an individual in need thereof at the same time, and each unit dosage is contained within one blister in the blister pack. In one embodiment, the composition or product of manufacture is contained in a child-resistant blister card package, e.g., as in U.S. Patent No. (USPN) 7,395,928, e.g., having a plurality of unit package regions for enclosing one unit dosage form, each comprising a cavity and a closure sheet to seal the cavity. In one embodiment, once a unit package region is detached, the corner defined by lines of weakening with perforations can be detached to expose an unsealed area and easily access the content of each unit package region.

In one embodiment, a composition or product of manufacture of the invention comprises (or is contained or packaged in) a packages, e.g., as in U.S. Patent App. Pub. No. 20060138016, e.g., having a base web comprising a polymeric film or sheet having at least one recess containing a packaged item, i.e., the composition or product of manufacture of the invention, and a sealing web, which can be sealed to a base web and covering the recess. The sealing web can have a strength which substantially prevents the packaged item from being pushed through it, unless on applying sufficient force to the recess in the base web. A portion of the package where the base web is sealed to the sealing web can have multiple lines of weakness positioned so that the portion can be folded towards a portion of the sealing web, and a corner of the package can be used to puncture the sealing web so that the packaged item can then be pushed through the sealing web.

In one embodiment, the composition or product of manufacture is contained in a child-resistant blister card package, e.g., as in U.S. Patent App. Pub. No. 20020008046, e.g., a package having a non-through score line in an exposed surface of a blister sheet of the blister package. The non-through score line can extend from one edge of an individual blister unit to an opposite or adjacent edge, e.g., across the corner of the blister unit. When the blister unit is angulated or flexed back at the non-through score line, the blister sheet can fracture. The smaller portion of the fractured blister sheet, still bonded to a backing sheet, can act as a tab for peeling the backing sheet from the blister sheet exposing the blister contents. Camouflage lines on the blister sheet can help hide the score line, thereby rendering the package highly child-resistant.

In one embodiment, the composition or product of manufacture is contained in a child-resistant blister card package, e.g., as in USPN 6,830,153, e.g., a child-resistant blister pack for unit dosage forms having a blister film sheet with depressions therein, where unit dosage forms are contained within the depressions and a lidding sheet overlies the depressions, which is secured to a film sheet so as to seal the unit dosage forms within the depressions. A network of lines of weakness in the pack can define a plurality of dosage units. Each dosage unit can include one of said dosage forms and a peel region where part of the lidding sheet is not secured to the blister film sheet. Each peel region can be disposed adjacent a respective one of the lines of weakness

In one embodiment, the composition or product of manufacture is contained in a package as described in, e.g., USPN 5,046,618, e.g., a child-resistant blister package wherein each individual package is defined by lines of weakening terminating short of the edge of the blister package and is provided with a tear strip defined by an additional line of weakening. After removal of the tear strip an unsealed corner region is exposed, which can be grasped and pulled allowing the separation of the closure sheet from the container sheet and the access to the formulation dosage.

In one embodiment, the composition or product of manufacture is contained in a package as described in, e.g., USPN 5,557,505, e.g., a blister card package with cut out areas exposing an area of the closure sheet at the intersection of the lines of weakening. After detachment of one individual dosage blister the area of exposed closure sheet forms a finger tab that when pulled separates the closure sheet from the container sheet allowing access to the content of the blister cavity.

In one embodiment, the compositions and methods of the invention are formulated for, packaged for use by, and/or are directed to a patient population where drug regimen compliance can be problematic, e.g., a stressed, challenged or non-compliant patient population, e.g., a cancer patient population, a pediatric or geriatric population, or a mentally compromised patient population, which in various embodiments includes patients having mild or severe mental retardation, slow cognition, dementia, senility, Alzheimer's disease, traumatic brain injury, chemical brain damage, mental diseases (e.g., dissociative disorder, obsessive-compulsive disorder, delusional disorder, schizophrenia, mania, panic disorder, depression, dyslexia, any learning disability and the like) post-traumatic stress disorder, traumatic war neurosis, post-traumatic stress syndrome (PTSS), physical disability (e.g., blindness) and the like. It is important to minimize the frequency and number of medications a patient must take. Patients who are required to take a medication only once or twice a day will have much higher compliance compared to a those who require medication of multiple pills three or more times a day. Thus, in one embodiment, the invention provides therapeutic combinations, formulations, packaging and the like to increase patient compliance with their needed drug regimen (the frequency and number of medications an individual must take); for example, in one aspect, a therapeutic combination of the invention is formulated and/or packaged for accurate and timely patient compliance, e.g., including once a day dosaging, multiple dosaging, or chrono-dosing as described herein.

The following examples are offered to illustrate, but not to limit the claimed invention.

### EXAMPLES

### Example: Therapy for Cellular Self-Defense Enhancement.

An exemplary therapy of this invention comprises use of or consists of use of a combination of compounds (ingredients); e.g., as in one embodiment, comprising or consisting of drugs, that will enhance the ability of a cell to defend against stress-induced damage and trauma, and this "stress-defense enhancement" is achieved through the activation of a "heat shock response" (including the production of heat shock proteins) and through increasing a cells' ability to process (and thus defend against and/or tolerate) reactive oxygen species; this "stress-defense enhancement" may also be achieved using other defense systems prior to or during, or after, chemotherapy.

Upon start of chemotherapy or radiation therapy, further tissue protection (and an aid for recovery) may be achieved by administering a combination of compounds of this invention, thereby reducing excessive or unwanted lymphocyte tracking that can lead to excess inflammation, as well as progressive and self-propagating tissue damage.

### Example: Pre-chemotherapy and Pre-radiation Therapy.

An exemplary therapy of this invention comprises use of or consists of use of a combination of compounds (ingredients); e.g., as in one embodiment, comprising or consisting of drugs, that may be administered prior to, or at about the same time as, or at the same time as, or after, or a combination thereof a therapy (such as a drug therapy, chemotherapy and/or radiation therapy that causes mucositis) that causes an unwanted side effect, such as an oral or a digestive mucositis. In one aspect, the combination of compounds (ingredients), such as drugs, of the invention activates a heat shock and/or other protective response in a cell and/or in an individual (e.g., a patient). In one aspect, a therapy of the invention can be administered in a manner that is timed optimally and/or timed to achieve a prophylactic benefit in cell and/or in an individual (e.g., a patient).

An exemplary therapy of this invention is administered at least two days prior to start of mucositis-causing drug or cancer therapy (such as chemotherapy and/or radiation therapy that causes mucositis); in an alternative embodiment, a therapy of this invention may be discontinued at about 6 hours prior to a mucositis-causing chemotherapy or a mucositis-causing radiation therapy.

### Example: Exemplary combinations of ingredients in a therapy of this invention are exemplified by ingredients that can activate a cell's heat shock response.

An exemplary therapy of this invention comprises is a combination of ingredients that can activate a heat shock response in a cell including: geranylgeranylacetone or gernate, zinc, tin, salicylates, dexamethasone, cocaine, nicotine, alpha-adrenergic agonist, ppar-gamma agonist, biomolecular-geldanamycin, cyclopentanone, prostanoids enprostil, paracetamol, ketotiphen, levamisole, diazepam, bromocriptine, and dopamine.

### Example: Exemplary combinations of ingredients in a therapy of this invention are exemplified by ingredients that can reduce a cell's inflammatory/progressive tissue damage response.

An exemplary therapy of this invention comprises is a combination of ingredients that can reduce an inflammatory/progressive tissue damage response in a cell, including: an ACE inhibitor, an ARB, a phenylbutyrate or a PPAR gamma (insulin sensitizer), and/or probucol, calcitriol an NSAID.

### Example: Exemplary therapy of this invention using VT-212™

In one embodiment, the invention provides a treatment for mucositis, such as an oral or a digestive mucositis, comprising or consisting of a combination of: Vicus Therapeutics (Morristown NJ) product VT-212™, which is GGA and etodolac. In one aspect, the drug combination comprises or consists of VT-212™ and/or VT-211™, which are used to target the initial stage of a maladaptive response to prevent, delay, and/or reduce the severity of ulceration that is often seen about 7 to 10 days after mucotoxic therapies.

VT-212, *i.e.* GGA and etodolac, was administered to 40 patients who had colorectal cancer and were scheduled to receive a chemotherapy (5-FU or 5-fluorouracil). Investigators observed a reduction in mucositis.

### Example: Exemplary therapy of this invention using VT-122™

A randomized, open label, controlled, multi dose study was conducted using a propranolol-etodolac drug combination (specifically, VT-122™ an oral, multi-targeted, chrono-modulated, fixed dose combination of propranolol and etodolac) for stage IV non-small cell lung cancer (NSCLC) subjects with cachexia who had failed prior chemotherapy, and were not receiving active therapy. Subjects meeting protocol defined eligibility criteria received a chrono-dosed administration of propranolol and etodolac in divided daily doses for one week to assess their ability to tolerate these two drugs administered simultaneously. Those subjects who tolerated this simultaneous administration of propranolol and etodolac were randomized in parallel into one of the following three groups: nutritional control (arm A) or propranolol with low (arm B) or high (arm C) doses of etodolac.

A statistically significant difference was observed in the "intention to treat" ("ITT"; including all subjects who received at least one dose of VT-122™) patient population, (Last Observation Carried Forward) in the proportion of subjects who responded with a clinically meaningful improvement of ≥ 5% in lean body mass at week 6, Week 9 and Week 12 (Group A, n= 0/12; Group B, n = 7/12, p=0.0191; Group C, n=5/12, p=0.0174; combined treatment group, n=12/24, p=0.0061). A favorable trend in response rates between Group B and Group C vs. Group A was also seen at week 4; however, this difference did not achieve statistical significance.

A statistically significant difference was observed in the Efficacy Evaluable population (all subjects who were randomized into the study, remained in the study and were at least 80% compliant with study medication for a minimum of 4 weeks, and who do not have any major protocol deviations) in the proportion of subjects who responded with a clinically meaningful improvement of ≥ 5% in lean body mass at Week 6, Week 9, and Week 12 (Group A, n= 0/6; Group B, n = 6/7, p=0.0048; Group C, n=4/5, p=0.1161; combined, n=10/12, p=0.0058). At 4 weeks there was a clinically significant difference between Group A and Group C, and strong trend but not statistical significance between Group A and Group C or the combined treatment groups.

In the ITT population the change in LBM at Week 12 in Group B was a gain of 3.6 ± 3.56 kg and Group C had a gain of 3.4 ± 6.25. In contrast Group A showed a loss of 4.4 ± 4.03 kg (p=0.0313).

Similar positive trends in both body weight and functional measures (as assessed by grip strength) were also seen in the treatment Groups (B and C) but not in the control Group (A). However, these improvements did not reach statistical significance. Also, the results from the exploratory analyses including QoL (CMSAS) assessments, while being inconclusive, primarily due to the variability of the data, are suggestive of positive efficacy trends.

Taken together, it appears that the VT-122 regimen is effective in treating cachexia (as assessed by increase in LBM and supported by changes in other parameters including weight loss and functional measures) in patients with Stage IV NSCLC.

In addition, it was reported by the investigators that disease progression was stopped in some patients, in 2 patients there was reduction of liver metastases and in 1 patient there was complete disappearance of brain metastases.

A number of aspects of the invention have been described. Nevertheless, it will be understood that various modifications may be made without departing from the spirit and scope of the invention. Accordingly, other aspects are within the scope of the following claims.

### ASPECTS OF THE INVENTION:

1. A therapeutic combination of drugs for an individual in need thereof comprising or consisting of:
   (i) (a) a beta adrenergic receptor antagonist; (b) a non-steroidal anti-inflammatory drug (a NSAID); and (c) a therapeutic agent for the treatment of cancer;
   (ii) (a) a macrolide or a composition comprising a macrolide ring, and (b) a therapeutic agent for the treatment of cancer;
   (iii) (a) an immunosuppressant composition or pharmaceutical, and (b) a therapeutic agent for the treatment of cancer;
   (iv) (a) a proton pump inhibitor (a PPI), and (b) a therapeutic agent for the treatment of cancer;
   (v) any combination of the therapeutic combination of drugs of (i), (ii), (iii) and/or (iv); or
   (vi) the combination of any of (i) to (v) further comprising a proton pump inhibitor (a PPI); a synthetic prostaglandin E₁ (PGE₁) analogue misoprostol; *N*-(4-hydroxyphenyl)acetamide (paracetamol or acetaminophen); 2-[4-(2-methylpropyl)phenyl]propanoic acid (ibuprofen); an anticonvulsant or antiseizure drug; an neuropathic pain analgesic; an opioid (opiate) painkiller (analgesic); an antibiotic; an antidepressant or antipsychotic; an antisense or siRNA nucleic acid; or, further comprising any combination thereof.
2. The therapeutic combination of aspect 1, wherein (i) the non-steroidal anti-inflammatory drug (a NSAID) comprises (a) a cyclooxygenase (COX) (or prostaglandin synthase) inhibitor; or, (b) the COX inhibitor of (a), wherein the COX inhibitor comprises or consists of an etodolac or equivalent; a naproxen or equivalent; a celecoxib or equivalent; a rofecoxib or equivalent; a etoricoxib or equivalent; a valdecoxib or equivalent; a parecoxib or equivalent; a nabumetone or equivalent; a diclofenac (2-(2,6-dichloranilino) phenylacetic acid) or equivalent; or, a lumiracoxib or equivalent; (ii) the neuropathic pain analgesic comprises or consists of gabapentin or pregabalin; or (iii) the antisense or siRNA nucleic acid comprises or consists of oblimersen or GENASENSE™.
3. The therapeutic combination of aspect 2, wherein the etodolac is LODINE™, LODINE SR™ or ECCOXOLAC™; or the celecoxib is CELEBREX™ or CELEBRA™; or the rofecoxib is VIOXX™, CEOXX™ or CEEOXX™; or the etoricoxib is ARCOXIA™, ALGIX™ or TAUXIB™; or the valdecoxib is BEXTRA™; the parecoxib is DYNASTAT™; the naproxen is XENOBID™, ALEVE™, ANAPROX™, MIRANAX™, NAPROGESIC™, NAPROSYN™, NAPRELAN™, PROXEN™ or SYNFLEX™; the nabumetone is RELAFEN™, RELIFEX™ or GAMBARAN™; or, the diclofenac is FLECTOR PATCH™, VOLTAREN™, VOLTAROL™, DICLON™, DICLOFLEX DIFEN™, DIFENE™, CATAFLAM™, PENNSAID™, PANAMOR™, RHUMALGAN™, MODIFENAC™, ABITREN™, OLFEN™, VOVERAN™, ARTHROTEC™, DEDOLOR™, DEFLAMAT™, VETAGESIC™ or ZOLTEROL™.
4. The therapeutic combination of aspect 1, wherein the beta adrenergic receptor antagonist (a beta blocker) comprises propranolol or equivalent.
5. The therapeutic combination of aspect 4, wherein the propranolol is INDERAL™, AVLOCARDYL™, DERALIN™, DOCITON™, INDERALICI™, INNOPRAN XL™, or SUMIAL™
6. The therapeutic combination of aspect 1, wherein the beta adrenergic receptor antagonist (a beta blocker) comprises propranolol or equivalent and the non-steroidal anti-inflammatory drug (a NSAID) comprises etodolac or equivalent.
7. The therapeutic combination of aspect 1, wherein the therapeutic agent for the treatment of cancer comprises or consists of a monoclonal antibody, a peptide, a synthetic polypeptide or peptidomimetic, a nucleic acid, a synthetic nucleic acid, a lipid, a carbohydrate and/or a small molecule.
8. The therapeutic combination of aspect 7, wherein the therapeutic agent for the treatment of cancer comprises or consists of a sorafenib or equivalent, or NEXAVAR™; a sunitinib or equivalent, or SUTENT™; an erlotinib or equivalent, or TARCEVA™; an imatinib or equivalent, or GLEEVEC™; a lapatinib or equivalent, or TYKERB™; a bevacizumab or equivalent, or AVASTIN™; a trastuzumab or equivalent, or HERCEPTIN™; a cetuximab or equivalent, or ERBITUX™; a bevacizumab or equivalent, or AVASTIN™ or BIBW 2992; a gefitinib or equivalent, or IRESSA™; a ranibizumab or equivalent, or LUCENTIS™; a pegaptanib or equivalent, or MACUGEN™; a dasatinib or equivalent, or BMS-354825™; a sunitinib or equivalent, or SUTENT™; a pazopanib or equivalent; a nilotinib or equivalent, or TASIGNA™; a panitumumab or equivalent, or VECTIBIX™; a bandetinib or equivalent; a brivanib or equivalent, or E7080™; a thalidomide or equivalent, or THALOMID™; lenalidomide or equivalent, or REVLIMID™; A bortezomib or equivalent, or VELCADE™; disulfiram or equivalent, or ANTABUSE™ or ANTABUS™; or an epigallocatechin gallate (EGCG) or equivalent; a demecolcine, an etoglucid or elsamitrucin, a lonidamine, a lucanthone, a mitotane or a mitoguazone or equivalent; or any combination thereof.
9. The therapeutic combination of aspect 1, wherein the therapeutic agent for the treatment of cancer comprises or consists of a protein kinase inhibitor or a histone deacetylase inhibitor.
10. The therapeutic combination of aspect 1, wherein the protein kinase inhibitor comprises or consists of a tyrosine kinase inhibitor or a serine/threonine kinase inhibitor; or the histone deacetylase inhibitor comprises or consists of a vorinostat (rINN) or ZOLINZA™, or suberoylanilide hydroxamic acid (SAHA).
11. The therapeutic combination of aspect 1, wherein the therapeutic agent for the treatment of cancer comprises or consists of an angiogenesis inhibitor.
12. The therapeutic combination of aspect 11, wherein the angiogenesis inhibitor comprises or consists of a vascular endothelial growth factor (VEGF)-mediated angiogenesis inhibitor.
13. The therapeutic combination of aspect 1, wherein the therapeutic agent for the treatment of cancer comprises or consists of an inducer of apoptosis or a mitotic and anti-microtubule inhibitor (inhibition of microtubule function).
14. The therapeutic combination of aspect 13, wherein the inducer of apoptosis or mitotic inhibitor or anti-microtubule inhibitor comprises or consists of a raltitrexed or equivalent, or TOMUDEX™; a doxorubicin or equivalent, or ADRIAMYCIN™; a fluorouracil or 5-fluorouracil or equivalent; a paclitaxel or equivalent, or TAXOL™ or ABRAXANE™; a docetaxel or equivalent, or TAXOTERE™; a larotaxel, tesetaxel or ortataxel or equivalent; an epothilone or an epothilone A, B, C, D, E or F or equivalent; an ixabepilone (also known as azaepothilone B) or equivalent, or BMS-247550™; a vincristine (also known as leurocristine) or equivalent, or ONCOVIN™; a vinblastin, vinblastine, vindesine, vinflunine, vinorelbine or NAVELBINE™ or equivalent; or, any combination thereof.
15. The therapeutic combination of aspect 1, wherein the therapeutic agent for the treatment of cancer comprises or consists of an alkylating agent.
16. The therapeutic combination of aspect 15, wherein the alkylating agent comprises or consists of a cisplatin or equivalent; a cisplatinum or equivalent; a *cis-*diamminedichloridoplatinum(II) (CDDP) or equivalent; a carboplatin or equivalent; a oxaloplatin or equivalent; a cyclophosphamide (cytophosphane) or equivalent, or ENDOXAN™, CYTOXAN™, NEOSAR™ or REVIMMUNE™; a mechlorethamine or equivalent; a chlormethine or equivalent; a mustine or equivalent; a nitrogen mustard or equivalent; a chlorambucil or equivalent, or LEUKERAN™; or, a combination thereof.
17. The therapeutic combination of aspect 1, wherein the therapeutic agent for the treatment of cancer comprises or consists of a topoisomerase inhibitor.
18. The therapeutic combination of aspect 17, wherein the topoisomerase inhibitor comprises or consists of an etoposide or equivalent, or EPOSIN™, ETOPOPHOS™, VEPESID™ or VP-16™; an amsacrine or equivalent; a topotecan or equivalent, or HYCAMTIN™; a teniposide or equivalent, or VUMON™ or VM-26™; an epipodophyllotoxin or equivalent; a camptothecin or equivalent; an irinotecan or equivalent, or CAMPTOSAR™; or, a combination thereof.
19. The therapeutic combination of aspect 1, wherein the therapeutic agent for the treatment of cancer comprises or consists of a glycopeptide antibiotic.
20. The therapeutic combination of aspect 19, wherein the glycopeptide antibiotic comprises or consists of a bleomycin or equivalent or a bleomycin A₂ or B₂ or equivalent; a mitomycin or a mitomycin C or equivalent, a plicamycin (also known as mithramycin) or equivalent, or MITHRACIN™; or, a combination thereof.
21. The therapeutic combination of aspect 1, wherein the therapeutic agent for the treatment of cancer comprises or consists of a steroid receptor inhibitor or steroid inhibitor (an anti-steroid).
22. The therapeutic combination of aspect 21, wherein the steroid receptor inhibitor comprises or consists of an estrogen receptor modulator (a SERM).
23. The therapeutic combination of aspect 22, wherein the estrogen receptor modulator comprises or consists of a tamoxifen or equivalent, or NOLVADEX™, ISTUBAL™ or VALODEX™.
24. The therapeutic combination of aspect 22, wherein the steroid inhibitor or an anti-steroid comprises or consists of a finasteride or equivalent, or PROSCAR™, PROPECIA™, FINCAR™, FINPECIA™, FINAX™, FINAST™, FINARA™, FINALO™, PROSTERIDE™, GEFINA™, APPECIA™, FINASTERID IVAX™, FINASTERID or ALTERNOVA™.
25. The therapeutic combination of aspect 1, wherein the therapeutic agent for the treatment of cancer comprises or consists of (a) a matrix metalloproteinase (MMP) inhibitor; (b) an mTOR (mammalian target of rapamycin) inhibitor, or (c) an mTOR inhibitor comprising or consisting of a temsirolimus or equivalent, or TORISEL™.
26. The therapeutic combination of aspect 1, wherein the macrolide or composition comprising a macrolide ring comprises or consists of a macrolide antibiotic.
27. The therapeutic combination of aspect 1 or aspect 26, wherein the macrolide or composition comprising a macrolide ring comprises or consists of a clarithromycin or equivalent, or BIAXIN™, KLARICID™, KLABAX™, CLARIPEN™, CLARIDAR™, FROMILID™ or CLACID™; an azithromycin or equivalent, or ZITHROMAX™, ZITROMAX™ or SUMAMED™; a dirithromycin or equivalent; an erythromycin or equivalent; a roxithromycin or equivalent, or ROXO™, SURLID™, RULIDE™, BIAXSIG™, ROXAR™, ROXIMYCIN™ or COROXIN™; a telithromycin or equivalent or KETEK™; a josamycin or equivalent; a kitasamycin or equivalent; a midecamycin or equivalent; oleandomycin or equivalent; a roxithromycin or equivalent, or ROXO™, SURLID™, RULIDE™, BIAXSIG™, ROXAR™, ROXIMYCIN™ or COROXIN™; a troleandomycin or equivalent; or a tylosin or equivalent; or, any combination thereof.
28. The therapeutic combination of aspect 1, wherein the immunosuppressant composition and/or drug or pharmaceutical comprises or consists of a sirolimus or equivalent (also known as rapamycin), or RAPAMUNE™; a tacrolimus or equivalent, or FK-506™ or FUJIMYCIN™; a ciclosporin (or cyclosporine or cyclosporin) or equivalent; or a cortisone or equivalent.
29. The therapeutic combination of aspect 1, wherein the proton pump inhibitor (a PPI) comprises or consists of an H₂-receptor antagonist (H₂RA).
30. The therapeutic combination of aspect 29, wherein the H₂-receptor antagonist (H₂RA) comprises or consists of a cimetidine or equivalent, or TAGAMET™, TAGAMET HB™ or TAGAMET HB200™; a ranitidine or equivalent, or TRITEC™ or ZANTAC™; a famotidine or equivalent, or PEPCIDINE™ or PEPCID™; a nizatidine or equivalent, or TAZAC™ or AXID™.
31. The therapeutic combination of aspect 1, wherein the proton pump inhibitor (a PPI) comprises or consists of a benzimidazole compound or structure, or an imidazopyridine compound or structure.
32. The therapeutic combination of aspect 31, wherein the imidazopyridine compound or structure comprises or consists of a zolpidem or equivalent, or AMBIEN™, AMBIEN CR™, IVEDAL™, NYTAMEL™, STILNOCT™, STILNOX™, ZOLDEM™, ZOLNOD™ or ZOLPIHEXAL™; an alpidem (also called ananxyl) or equivalent; a saripidem or equivalent; necopidem or equivalent.
33. The therapeutic combination of any of aspects 1 to 32, wherein two or more drugs of the therapeutic combination are formulated as separate compositions, or two or more drugs of the therapeutic combination are formulated into one composition or drug formulation (two or more drugs of the therapeutic combination are formulated together).
34. The therapeutic combination of aspect 33, wherein the beta adrenergic receptor antagonist, or a beta blocker or equivalent, or a propranolol or equivalent; the non-steroidal anti-inflammatory drug, or a NSAID or equivalent, or an etodolac or equivalent; and the therapeutic agent for the treatment of cancer, are formulated in different compositions or formulations, or, are formulated in the same composition or formulation, or are formulated together.
35. The therapeutic combination of any of aspects 1 to 34, wherein one or two or more or all of the drugs of the therapeutic combination are packaged individually, or are packaged together, or packaged in any combination, in a single package, a plurality of packages or packettes, or a blister packet, lidded blister or blister card or packets, or a shrink wrap.
36. The therapeutic combination of aspect 35, wherein the beta adrenergic receptor antagonist, or a beta blocker or equivalent, or a propranolol or equivalent; the non-steroidal anti-inflammatory drug, or a NSAID or equivalent, or an etodolac or equivalent; and the therapeutic agent for the treatment of cancer, are packaged individually in a single package, a plurality of packages or packettes, or a blister packet, lidded blister or blister card or packets, or a shrink wrap.
37. The therapeutic combination of any of aspects 1 to 36, wherein one or two or more or all of the drugs of the therapeutic combination are packaged together or in any combination in a single package, a plurality of packages or packettes, or a blister packet, lidded blister or blister card or packets, or a shrink wrap.
38. The therapeutic combination of aspect 37, wherein two or more or all of the drugs are released upon opening of the single package, plurality of packages or packettes, blister packet, lidded blister, blister card or packets or shrink wrap.
39. The therapeutic combination of aspect 1, wherein the beta adrenergic receptor antagonist, or a beta blocker or equivalent, or a propranolol or equivalent; the non-steroidal anti-inflammatory drug, or a NSAID or equivalent, or an etodolac or equivalent; and the therapeutic agent for the treatment of cancer are packaged together in a single package, a plurality of packages or packettes, or a blister packet, lidded blister or blister card or packets, or a shrink wrap, and two or more or all of the drugs are released upon opening of the single package, plurality of packages or packettes, blister packet, lidded blister, blister card or packets or shrink wrap.
40. The therapeutic combination of any of aspects 1 to 39, wherein one or two or more or all of the drugs of the therapeutic combination are formulated or manufactured as a parenteral formulation, an aqueous solution, a liposome, an injectable solution, a tablet, a pill, a lozenge, a capsule, a caplet, a patch, a spray, an inhalant, a powder, a freeze-dried powder, an inhalant, a patch, a gel, a geltab, a nanosuspension, a nanoparticle, a nanoliposome, a microgel, a pellet, a suppository or any combination thereof.
41. The therapeutic combination of aspect 41, one or two or more or all of the drugs of the therapeutic combination are formulated or manufactured together in one parenteral formulation, one aqueous solution, one liposome, one injectable solution, one freeze-dried powder, one feed, one food, one food supplement, one pellet, one lozenge, one liquid, one elixir, one aerosol, one inhalant, one adhesive, one spray, one powder, one freeze-dried powder, one patch, one tablet, one pill, one capsule, one gel, one geltab, one lozenge, one caplet, one nanosuspension, one nanoparticle, one nanoliposome, one microgel or one suppository.
42. The therapeutic combination of aspect 1, wherein
   (a) the dosage of etodolac ranges from about 200 mg to 400 mg a day, or, about 10, 15, 20, 25, 30, 35, 40, 45, 50, 75, 80, 85, 90, 100, 150, 200, 250, 300, 350, 400, 450, 500, 600, 700, 800, 900 or 1000 mg or more; or
   (b) the dosage of propranolol ranges from 10 to 320 mg per day based on heart rate and blood pressure of the individual, or, about 10, 15, 20, 25, 30, 35, 40, 45, 50, 75, 80, 85, 90, 100, 150, 200, 250, 300, 350, 400, 450, 500, 600, 700, 800, 900 or 1000 mg or more.
43. The therapeutic combination of aspect 1, wherein
   (a) the dosage of clarithromycin comprises or consists of a 250 mg immediate release formulation or tablet every 12 hours, a 500 mg immediate release formulation or tablet every 12 hours, or a 1000 mg extended release formulation or tablet once daily, or any combination thereof;
   (b) the dosage of roxithromycin comprises or consists of a 300 mg formulation or tablet once a day, or a 150 mg formulation or tablet twice a day, or any combination thereof;
   (c) the dosage of rapamycin comprises or consists of 2 mg/day to 20 mg/day, based on tolerability and liver and kidney side effects;
   (d) the dosage of rapamycin of (c), wherein the dose is increased (doubled) every week; or
   (e) the dosage of rapamycin of (d), wherein the dose is doubled every week.
44. The therapeutic combination of aspect 1, the drug combination is packaged in dosages that match a chrono-dosing regimen to match an optimal dose for the time of day.
45. The therapeutic combination of aspect 44, wherein the beta adrenergic receptor antagonist or a beta blocker or equivalent, or a propranolol or equivalent; the non-steroidal anti-inflammatory drug or NSAID or equivalent, or etodolac or equivalent; and the therapeutic agent for the treatment of cancer, are packaged in dosages that match a chrono-dosing regimen to match an optimal dose for the time of day.
46. The therapeutic combination of aspect 45, wherein the beta adrenergic receptor antagonist or beta blocker or equivalent or propranolol or equivalent; the non-steroidal anti-inflammatory drug or NSAID or equivalent or etodolac or equivalent; and the therapeutic agent for the treatment of cancer, are packaged in dosages that match a chrono-dosing regimen comprising:
   (a) in the AM, 20 mg beta adrenergic receptor antagonist (a beta blocker), e.g., a propranolol or equivalent, 200mg NSAID, e.g., an etodolac or equivalent; in the afternoon, 10 mg beta blocker, 200 mg NSAID, e.g., an etodolac or equivalent; in the PM, 10 mg beta blocker, 400 mg NSAID;
   (b) in the AM 40 mg beta adrenergic receptor antagonist (a beta blocker), e.g., a propranolol or equivalent, 200 mg NSAID, e.g., an etodolac or equivalent; in the afternoon 20 mg beta blocker, 200 mg NSAID; in the evening, 20 mg propranolol, 400 mg NSAID;
   (c) in the AM 80 mg beta adrenergic receptor antagonist (a beta blocker), e.g., a propranolol or equivalent, 200 mg NSAID; in the afternoon 40 mg beta blocker, 200 mg NSAID, in the evening 40 mg, NSAID; or
   (d) a dose escalation comprising a regimen of (a) to (b) to (c).
47. The therapeutic combination of aspect 45, wherein the beta adrenergic receptor antagonist or beta blocker or equivalent or propranolol or equivalent; the non-steroidal anti-inflammatory drug or NSAID or equivalent or etodolac or equivalent; and the therapeutic agent for the treatment of cancer, are packaged in dosages that match a chrono-dosing regimen comprising:
   Start: AM, 20 mg propranolol, 200mg etodolac; afternoon, 10 mg propranolol, 200 mg etodolac; PM 5 mg propranolol, 400 mg etodolac;
   Dose Escalation 1: AM 40 mg propranolol, 200 mg etodolac; afternoon 20 mg propranolol, 200 mg etodolac; evening, 10 mg propranolol, 400 mg etodolac;
   Dose escalation 2: AM 80 mg propranolol, 200 mg etodolac; afternoon 40 mg propranolol, 200 mg etodolac, evening 20 mg, etodolac.
48. The therapeutic combination of any of aspects 1 to 47, wherein the therapeutic drug combination is formulated for administration once a day, b.i.d. or t.i.d, or weekly, or biweekly, or monthly.
49. The therapeutic combination of aspect 48, wherein the beta adrenergic receptor antagonist (a beta blocker) or propranolol or equivalent; the non-steroidal anti-inflammatory drug or NSAID or etodolac or equivalent; and the therapeutic agent for the treatment of cancer, are formulated for administration once a day, b.i.d. or t.i.d, or weekly, or biweekly, or monthly.
50. The therapeutic combination of any of aspects 1 to 49, wherein the therapeutic combination of drugs are formulated for administration intravenously, topically, orally, by inhalation, by infusion, by injection, by inhalation, intraperitoneally, intramuscularly, subcutaneously, intra-aurally, for intra-articular administration, for intra-mammary administration, for topical administration or for absorption through epithelial or mucocutaneous linings.
51. The therapeutic combination of any of aspects 1 to 50, further comprising instructions for use in the treatment of a cancer, a cachexia, a cancer cachexia, a mucositis, an oral mucositis, a digestive mucositis, an esophageal mucositis, an intestinal mucositis and/or an anorexia.
52. The therapeutic combination of aspect 51, wherein the cachexia is defined as at least two of the symptoms selected from the group consisting of: 1) a hyper-inflammatory state, 2) altered hormone levels and cytokine levels; 3) decreased heart rate variability; 4) weight loss, and 5) increased heart rate, wherein optionally the increased heart rate is having a sustained elevated heart rate of at least about 6 bpm.
53. The therapeutic combination of aspect 51, wherein the cachexia is defined by an individual having at least a sustained elevated heart rate of at least about 6 bpm and weight loss.
54. A pharmaceutical composition or formulation comprising the therapeutic combination of any one of aspects 1 to 53.
55. The pharmaceutical composition or formulation of aspect 54, further comprising a pharmaceutically acceptable excipient.
56. The pharmaceutical composition or formulation of aspect 54 or aspect 55, wherein the pharmaceutical composition or formulation is formulated or manufactured as a feed, a food, a food or feed concentrate, a pellet, a lozenge, a liquid, a lotion, an implant, a nanoparticle, an elixir, an aerosol, a spray, an aerosol, an inhalant, a powder, a tablet, a pill, a capsule, a gel, a geltab, a nanosuspension, a nanoparticle, a patch, a microgel or a suppository.
57. Use of the therapeutic combination of any of aspects 1 to 53 in the manufacture of a medicament or pharmaceutical composition for treating, ameliorating or preventing a trauma, condition or disease comprising: a cancer or dysfunctional cell condition; any side effect from the treatment of cancer, chronic Systemic Inflammatory Response State (SIRS); chronic systemic inflammatory stress; burns, chronic obstructive pulmonary disease; congestive heart failure; chronic kidney disease; surgery; sepsis; ageing; acute respiratory distress syndrome; acute lung injury; infection; a CNS disorder or injury; anemia; immunosuppression; insulin resistance; anorexia; anxiety; sleep disturbances; weakness; fatigue; gastrointestinal distress; sleep disturbances; wake disturbances; pain; listlessness; shortness of breath; lethargy; depression; malaise; or, a combination thereof.
58. The use of aspect 57, wherein the trauma, condition or disease comprises a maladaptive nutritional state secondary to the SIRS, or the maladaptive nutritional state comprises cachexia, and optionally the cachexia comprises cachexia secondary to cancer.
59. The use of aspect 58, wherein the cancer or dysfunctional cell condition comprises (is) any metastatic or benign tumor, and the use is for treating (killing, eliminating, stopping the growth and/or metastasis of) cancer stem cells or cancer cells from: lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma or pituitary adenoma, and any combination thereof.
60. The use of aspect 58, wherein the cachexia is defined as at least two of the symptoms selected from the group consisting of: 1) a hyper-inflammatory state, 2) altered hormone levels and cytokine levels; 3) decreased heart rate variability; 4) weight loss, and 5) increased heart rate, wherein optionally the increased heart rate is having a sustained elevated heart rate of at least about 6 bpm.
61. The use of aspect 58, wherein the cachexia is defined by an individual having at least a sustained elevated heart rate of at least about 6 bpm and weight loss.
62. The use of aspect 57, wherein the CNS disorder comprises Parkinson's disease or Alzheimer's disease.
63. A method for treating or ameliorating a trauma, condition or disease comprising a cancer or dysfunctional cell condition or a mucositis, any side effect from the treatment of a cancer or dysfunctional cell condition, chronic Systemic Inflammatory Response State (SIRS), chronic systemic inflammatory stress; burns, chronic obstructive pulmonary disease; congestive heart failure; chronic kidney disease; surgery; cancer; sepsis; ageing; acute respiratory distress syndrome; acute lung injury; infection; a CNS disorder or injury; anemia; immunosuppression; insulin resistance; anorexia; anxiety; sleep disturbances; weakness; fatigue; gastrointestinal distress; sleep disturbances; wake disturbances; pain; listlessness; shortness of breath; lethargy; depression; malaise; or, a combination thereof, the method comprising the steps of:
   (a) providing the therapeutic combination of any of aspects 1 to 53, or the pharmaceutical composition or formulation of any of aspects 54 to 56; and,
   (b) administering a therapeutically effective amount of the therapeutic combination or the pharmaceutical composition or formulation of step (a), thereby treating or ameliorating the side effect, trauma, condition or disease.
64. The method of aspect 63, wherein the condition or disease comprises a maladaptive nutritional state secondary to the SIRS.
65. The method of aspect 64, wherein the maladaptive nutritional state comprises cachexia or anorexia, or a cachexia secondary to cancer.
66. The method of aspect 63, wherein the cancer or dysfunctional cell condition comprises (is) any metastatic or benign tumor, and the use is for treating (killing, eliminating, stopping the growth and/or metastasis of) cancer stem cells or cancer cells from: lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, a neoplasm of the central nervous system (CNS), primary CNS lymphoma, spinal axis tumors, brain stem glioma or pituitary adenoma, and any combination thereof.
67. The method of aspect 65, wherein the cachexia is defined as at least two of the symptoms selected from the group consisting of: 1) a hyper-inflammatory state, 2) altered hormone levels and cytokine levels; 3) decreased heart rate variability; 4) weight loss, and 5) increased heart rate, wherein optionally the increased heart rate is having a sustained elevated heart rate of at least about 6 bpm.
68. The method of aspect 65, wherein the cachexia is defined by an individual having at least a sustained elevated heart rate of at least about 6 bpm and weight loss.
69. The method of aspect 63, wherein the mucositis is a mucositis caused by a chemotherapy and/or a radiation therapy; or an oral mucositis, a digestive mucositis, an esophageal mucositis or an intestinal mucositis.
70. A composition or product of manufacture comprising the therapeutic combination of any of aspects 1 to 53, and/or the pharmaceutical composition or formulation of any of aspects 54 to 56.
71. The composition or product of manufacture of aspect 70, comprising a blister pack, clamshell or tray, wherein the therapeutic combination is formulated for unit dosage administration to an individual in need thereof at the same time, and each unit dosage is contained within one blister in the blister pack, or compartment in the clamshell or tray.
72. The composition or product of manufacture of aspect 70 or aspect 71, comprising a caplet, a lozenge, a pill, capsule, tablet, tab, geltab or implant, wherein the therapeutic combination is formulated for unit dosage administration to an individual in need thereof at the same time.
73. The composition or product of manufacture of aspect 72, wherein each unit dosage is contained within one caplet, lozenge, pill, capsule, tablet, tab, geltab or implant.
74. A nanoparticle, microparticle, nanoliposome or liposome comprising the therapeutic combination of any of aspects 1 to 53, or the pharmaceutical composition or formulation of any of aspects 54 to 56.
75. A kit comprising the therapeutic combination of any of aspects 1 to 53, or the pharmaceutical composition or formulation of any of aspects 54 to 56.
76. The kit of aspect 75, comprising at least one blister pack, lidded blister or blister card or packets, or a shrink wrap, comprising the therapeutic combination or the pharmaceutical composition.
77. A kit for the treatment, amelioration or prevention of a mucositis, a cancer or any dysfunctional cell condition, a side effect from the treatment of a cancer or dysfunctional cell condition, a chronic Systemic Inflammatory Response State (SIRS) or a maladaptive nutritional state in a patient population, the kit comprising: the therapeutic combination of any of aspects 1 to 53; the pharmaceutical composition or formulation of any of aspects 54 to 56; the composition or product of manufacture of aspect 70; the nanoparticle, microparticle, nanoliposome or liposome of aspect 74; and, instructions for use of the therapeutic combination or pharmaceutical composition.
78. The kit of aspect 77, wherein the mucositis is a mucositis secondary to radiotherapy (radiation therapy) and/or chemotherapy, or the mucositis is an oral mucositis, a digestive mucositis, an esophageal mucositis, an intestinal mucositis.
79. The kit of aspect 77, wherein the maladaptive nutritional state comprises cachexia, and optionally the cachexia comprises cachexia secondary to cancer.
80. The kit of aspect 79, wherein the cachexia is defined:
   (a) as having (being associated with) at least two of the symptoms selected from the group consisting of: 1) a hyper-inflammatory state, 2) altered hormone levels and/or cytokine levels; 3) decreased heart rate variability; 4) weight loss, and 5) sustained increased heart rate, wherein optionally the sustained increased heart rate is having a sustained elevated heart rate of at least about 6 bpm; or
   (b) by an individual having at least a sustained elevated heart rate of at least about 6 bpm and weight loss.
81. The kit of aspect 77, wherein the cancer or dysfunctional cell condition comprises (is) any metastatic or benign tumor, and the use is for treating (killing, eliminating, stopping the growth and/or metastasis of) cancer stem cells or cancer cells from: lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma or pituitary adenoma, and any combination thereof.
82. A product of manufacture comprising a blister package, a lidded blister or a blister card or packet, a clamshell, a tray or a shrink wrap comprising the therapeutic combination of any of aspects 1 to 53, or the pharmaceutical composition or formulation of any of aspects 54 to 56.
83. A product of manufacture comprising a blister package, a lidded blister or a blister card or packet, a clamshell, a tray or a shrink wrap comprising the therapeutic combination of any of aspects 1 to 53, or the pharmaceutical composition or formulation of any of aspects 54 to 56, wherein the therapeutic combination or pharmaceutical composition or formulation are manufactured and/or formulated for at least two, three, four or five or more dosage administrations; or the therapeutic combination or pharmaceutical composition or formulation are manufactured and/or formulated for once a day, or bid (twice a day), or tid (three times a day), or four times a day, administration.
84. The product of manufacture of aspect 83, wherein the therapeutic combination or pharmaceutical composition or formulation are formulated as one dosage administration in the morning and one dosage administration in the evening; or are formulated as one dosage administration in the morning, one dosage mid-day and one dosage administration in the evening.
85. The product of manufacture of aspect 85, wherein the dosage schedule provides a relatively higher dose of one drug in the morning (the AM) than in the evening, and a relatively higher dose of another medication in the evening than in the morning.
86. A product of manufacture or formulation comprising the therapeutic combination of any of aspects 1 to 53, or the pharmaceutical composition or formulation of any of aspects 54 to 56, and a nutritional supplement, or food supplement or feed supplement.
87. A method for treating, ameliorating or preventing mucositis, a cachexia and/or a chronic Systemic Inflammatory Response State (SIRS), wherein the therapeutic combination of any of aspects 1 to 53, or the pharmaceutical composition or formulation of any of aspects 54 to 56, is administered to an individual in need thereof, and the therapeutic combination or the pharmaceutical composition are formulated for at least two administrations, one in the morning and one in the evening, wherein the dosage schedule provides a relatively higher dose of beta blocker in the morning (the AM) than in the evening, and a relatively higher dose of an anti-inflammatory medication in the evening than in the morning.
88. The method of aspect 87, wherein the administration regimen comprises at least two dosages of beta adrenergic receptor antagonist (a beta blocker) drug and at least two dosages of non-steroidal anti-inflammatory drug (a NSAID), and further comprising administration of an anti-anxiety drug, and the drugs are organized or labeled in a blister package, a lidded blister or a blister card or packet, a clamshell, a tray or a shrink wrap for usage by an individual for at least two administrations, one in the morning and one in the evening, wherein the dosage schedule provides a relatively higher dose of beta blocker in the morning (the AM) than in the evening, and a relatively higher dose of an anti-anxiety and/or an anti-inflammatory medication in the evening than in the morning.
89. The method of aspect 87, wherein the administration regimen comprises doses of propranolol given in 20 or 40 mg tablets immediate release on a bid basis, and in the first dose week the doses for propranolol are 20 mg in the morning and 20 mg at bedtime, and after 1 week the dosage is adjusted to 20 mg of the immediate release product in the morning and 60 mg of the extended release at bedtime, and optionally if after an additional week the subject shows no improvement or has not obtained a 20% reduction in heart rate, without decreasing heart rate below 60 bpm or blood pressure below 90/60, the dose is adjusted to 40 mg of the immediate release propranolol in the morning and 120 mg of the extended release propranolol at bedtime.
90. The method of aspect 87, wherein the administration regimen comprises doses of etodolac are given in 200 mg capsules or 500 mg tablets on a bid basis, and doses for etodolac are started at 200 mg in the morning and at bedtime, and after 1 week the dosage are adjusted to 200 mg in the morning and 500 mg at bedtime.
91. A blister pack or a plurality of blister packettes, a blister package, a lidded blister or a blister card or packet, a clamshell, a tray or a shrink wrap, comprising the therapeutic combination of any of aspects 1 to 53, or the pharmaceutical composition or formulation of any of aspects 54 to 56.
92. The blister pack or a plurality of blister packettes, a blister package, a lidded blister or a blister card or packet, a clamshell, a tray or a shrink wrap of aspect 91, wherein the therapeutic combination of drugs are arranged or clustered in the blister pack or a plurality of blister packettes: (a) in a chrono-dosing arrangement or pattern; or (b) individually.
93. A paper, plastic or cellophane package or a plurality of packettes comprising the therapeutic combination of any of aspects 1 to 53, or the pharmaceutical composition or formulation of any of aspects 54 to 56.
94. The paper, plastic or cellophane package or a plurality of packettes of aspect 93, wherein the therapeutic combination of drugs are arranged or clustered in the paper, plastic or cellophane package or a plurality of packettes: (a) in a chrono-dosing arrangement or pattern; or (b) individually.
95. The blister pack or a plurality of blister packettes, a blister package, a lidded blister or a blister card or packet, a clamshell, a tray or a shrink wrap of aspect 91 or aspect 92, or the paper, plastic or cellophane package or a plurality of packettes of aspect 93 or aspect 94, wherein the drug combination is formulated, packaged or designed for drug regimen compliance of a cancer patient population, a pediatric or geriatric population, or a mentally compromised patient population.
96. The blister pack or a plurality of blister packettes, a blister package, a lidded blister or a blister card or packet, a clamshell, a tray or a shrink wrap of aspect 91 or aspect 92, or the paper, plastic or cellophane package or a plurality of packettes of aspect 93 or aspect 94, wherein the drug combination is formulated, packaged or designed for drug regimen compliance of a cancer patient population having mild or severe mental retardation, slow cognition, dementia, senility, Alzheimer's disease, traumatic brain injury, chemical brain damage, mental diseases (e.g., dissociative disorder, obsessive-compulsive disorder, delusional disorder, schizophrenia, mania, panic disorder and the like) post-traumatic stress disorder, traumatic war neurosis, post-traumatic stress syndrome (PTSS), physical disability (e.g., blindness).
97. A food or food supplement comprising the therapeutic combination of any of any of aspects 1 to 46, or the pharmaceutical composition or formulation of any of aspects 54 to 56.
98. A feed or feed supplement comprising the therapeutic combination of any of any of aspects 1 to 46, or the pharmaceutical composition or formulation of any of aspects 54 to 56.
99. A nutraceutical comprising the therapeutic combination of any of any of aspects 1 to 46, or the pharmaceutical composition or formulation of any of aspects 54 to 56.
100. A composition or product of manufacture comprising:
   (a) a combination of compounds comprising or consisting of
      (i) at least two different compounds, each selected from a separate group selected from the group consisting of Group A, Group B, Group C, Group D, Group E, Group F, Group G, Group H, Group I, Group J, Group K, Group L and Group M, as set forth in Table 1, or,
      (ii) at least two different compounds, wherein at least two of the compounds are selected from the same group selected from the group consisting of Group A, Group B, Group C, Group D, Group E, Group F, Group G, Group H, Group I, Group J, Group K, Group L and Group M,
      wherein
      Group A comprises or consists of geranylgeranyl compounds (acyclic polyisoprenoids);
      Group B comprises or consists of Angiotensin Converting Enzyme Inhibitors (ACE Inhibitors or ACE-Is);
      Group C comprises or consists of Angiotensin Receptor Blockers (ARBs);
      Group D comprises or consists of Peroxisome Proliferator-Activated Receptor (PPAR) ligands;
      Group E comprises or consists of Non-Steroidal Anti-Inflammatory Drugs (NSAIDs);
      Group F comprises or consists of compositions for chemotherapy or radiation therapy;
      Group G comprises or consists of TNF inhibitors;
      Group H comprises or consists of deferrioxamine or deferoxamine;
      Group I comprises or consists of polyunsaturated fatty acids;
      Group J comprises or consists of eflornithine (α-difluoromethylornithine or DFMO);
      Group K comprises or consists of superoxide dismutases (SOD), catalases, superoxide dismutase (SOD)-catalase conjugates or complexes, peroxiredoxins and peroxidases;
      Group L comprises or consists of activators of a heat shock response;
      Group M comprises or consists of drugs that reduce an inflammatory response or a progressive tissue damage response;
   (b) the composition or product of manufacture of (a) comprising
      (i) at least three, four, five, six, seven, eight, nine or ten or more different compounds, each selected from a separate group selected from the group consisting of Group A, Group B, Group C, Group D, Group E, Group F, Group G, Group H, Group I, Group J, Group K, Group L and Group M, as set forth in Table 1, or,
      (ii) at least three, four, five, six, seven, eight, nine or ten or more different compounds, wherein at least three, four, five, six, seven, eight, nine or ten or more of the compounds are selected from the same group selected from the group consisting of Group A, Group B, Group C, Group D, Group E, Group F, Group G, Group H, Group I, Group J, Group K, Group L and Group M,
   (c) the composition or product of manufacture of (a) or (b), wherein the combination of compounds comprise or consist of at least one, three, four, five, six, seven, eight, nine or ten or more compounds selected from at least one, three, four, five, six, seven, eight, nine, ten, eleven, twelve or all of the following groups: Group A, Group B, Group C, Group D, Group E, Group F, Group G, Group H, Group I, Group J, Group K, Group L, and/or Group M (selected from the group consisting of Group A, Group B, Group C, Group D, Group E, Group F, Group G, Group H, Group I, Group J, Group K, Group L, and Group M);
   (d) the composition or product of manufacture of (c), wherein the combination of compounds comprise or consist of at least one compound from each of (selected from the group consisting of)
      Group A and Group B, Group A and Group C, Group A and Group D, Group A and Group E, Group A and Group F, Group A and Group G, Group A and Group H, Group A and Group I, Group A and Group J, Group A and Group K, Group A and Group L, Group A and Group M, Group B and Group C,
      Group B and Group C, Group B and Group D, Group B and Group E, Group B and Group F, Group B and Group G, Group B and Group H, Group B and Group I, Group B and Group J, Group B and Group K, Group B and Group L, Group B and Group M,
      Group C and Group D, Group C and Group E, Group E and Group D; Group C and Group E, Group C and Group F, Group C and Group G, Group B and Group H, Group C and Group I, Group C and Group J, Group C and Group K, Group C and Group L, Group C and Group M,
      Group D and Group E, Group D and Group F, Group D and Group G, Group D and Group H, Group D and Group I, Group D and Group J, Group D and Group K, Group D and Group L, Group D and Group M,
      Group E and Group F, Group E and Group G, Group E and Group H, Group E and Group I, Group E and Group J, Group E and Group K, Group E and Group L, Group E and Group M,
      Group F and Group G, Group F and Group H, Group F and Group I, Group F and Group J, Group F and Group K, Group F and Group L, Group F and Group M,
      Group G and Group H, Group G and Group I, Group G and Group J, Group G and Group K, Group G and Group L, Group G and Group M,
      Group H and Group I, Group H and Group J, Group H and Group K, Group H and Group L, Group H and Group M,
      Group I and Group J, Group I and Group K, Group I and Group L, Group I and Group M,
      Group J and Group K, Group J and Group L, Group J and Group M, or
      Group K and Group L, Group L and Group M;
   (e) the composition or product of manufacture of any of (a) to (d), wherein the combination of compounds comprises or consists of a geranylgeranyl acetone (GGA) or an analog of geranylgeranyl acetone and a non-steroidal anti-inflammatory drug (NSAID), or teprenone or SELBEX™ and a non-steroidal anti-inflammatory drug (NSAID);
   (f) the composition or product of manufacture of (e), wherein the combination of compounds comprises or consists of (i) a GGA or an analog of GGA or a teprenone or a SELBEX™ and (ii) etodolac, aspirin, diclofenac, diflunisal, fenoprofen, flurbiprofen, ibuprofen, indomethacin, ketoprofen, ketorolac tromethamine, meclofenamate, mefenamic acid, meloxicam, nabumetone, naproxen, oxaprozin, piroxicam, salsalate, sulindac, tolmetin, a COX2-selective NSAID, celecoxib, rofecoxib, etoricoxib, valdecoxib, parecoxib, meloxicam or lumiracoxib;
   (g) the composition or product of manufacture of (f), wherein the combination of compounds comprises or consists of a GGA or an analog of GGA or a teprenone or a SELBEX™ and: VOLTAREN™, CATAFLAM™, VOLTAREN-XR™, DOLOBID™, LODINE™, NALFON™, ANSAID™, FROBEN™, MOTRIN™, INDOCIN™, INDOCIN -SR™, ORUDIS™, ORUVAIL™, TORADOL™, MECLOMEN™, PONSTEL™, MOBICOX™, MOBIC™, RELIFEX™, RELAFEN™, ALEVE™, ANAPROX™, MIRANNAX™, NAPROGESIC™, NAPROSYN™, NAPRELAN™, SYNFLEX™, DAYPRO™ or DURAPROX™, FELDENE™, DISALCID™, SALFLEX™, CLINORIL™, TOLECTIN™, CELEBRA™, CELEBREX™, VIOXX™, CEOXX™, ARCOXIA™, BEXTRA™, DYNASTAT™, MOBIC™ or PREXIGE™;
   (h) the composition or product of manufacture of any of (a) to (d), wherein the combination of compounds comprises or consists of a geranylgeranyl acetone (GGA) or an analog of geranylgeranyl acetone and an angiotensin converting enzyme (ACE) inhibitor, or teprenone or SELBEX™ and an angiotensin converting enzyme (ACE) inhibitor;
   (i) the composition or product of manufacture of (h), wherein the combination of compounds comprises or consists of a geranylgeranyl acetone (GGA) or an analog of geranylgeranyl acetone or teprenone or SELBEX™ and: benazepril; captopril; cilazapril; enalapril; enalaprilat; fosinopril, fosinoprilat, imidapril (imidaprilum), lisinopril; moexipril; perindopril (coversyl); quinapril; ramipril; or, trandolapril;
   (j) the composition or product of manufacture of (i), wherein the combination of compounds comprises or consists of a geranylgeranyl acetone (GGA) or an analog of geranylgeranyl acetone or teprenone or SELBEX™ and: LOTENSIN™, CAPOTIN™, RENITEC™, VASOTEC™, MONOPRIL™, UNIVASC™, PERDIX™, ACCUPRIL™, TRITACE™, RAMACE™, ALTACE™, or MAVIK™;
   (k) the composition or product of manufacture of any of (a) to (d), wherein the combination of compounds comprises or consists of a geranylgeranyl acetone (GGA) or an analog of geranylgeranyl acetone and a pentoxifylline, or 1-(5-oxohexyl)-3, 7-dimethylxanthine, or TRENTAL™;
   (l) the composition or product of manufacture of any of (a) to (d), wherein the combination of compounds comprises or consists of a geranylgeranyl acetone (GGA) or an analog of geranylgeranyl acetone and a desferrioxamine (deferoxamine), or DESFERAL™, DESFERAN™, DESFERAL™, DESFEREX™, DESFERIN™, or DESFERRIN™;
   (m) the composition or product of manufacture of any of (a) to (d), wherein the combination of compounds comprises or consists of a geranylgeranyl acetone (GGA) or an analog of geranylgeranyl acetone and a polyunsaturated fatty acid;
   (n) the composition or product of manufacture of (m), wherein the combination of compounds comprises or consists of a geranylgeranyl acetone (GGA) or an analog of geranylgeranyl acetone and a ω-3 fatty acid or omega-3 fatty acid, a ω-6 fatty acid or omega-6 fatty acid, or a as ω-9 fatty acid or omega-9 fatty acid;
   (o) the composition or product of manufacture of (m), wherein the combination of compounds comprises or consists of a geranylgeranyl acetone (GGA) or an analog of geranylgeranyl acetone and α-linolenic acid (ALA), eicosapentaenoic acid (EPA), docosahexaenoic acid (DHA), linoleic acid (LA), oleic acid and/or erucic acid;
   (p) the composition of any of (a) to (d), wherein the combination of compounds comprises or consists of geranylgeranyl acetone (GGA) or an analog of geranylgeranyl acetone and an eflornithine, or α-difluoromethylornithine or DFMO, or ORNIDYL™;
   (q) the composition or product of manufacture of any of (a) to (d), wherein the combination of compounds comprises or consists of geranylgeranyl acetone (GGA) or an analog of geranylgeranyl acetone and a superoxide dismutase (SOD);
   (r) the composition or product of manufacture of any of (a) to (d), wherein the combination of compounds comprises or consists of geranylgeranyl acetone (GGA) or an analog of geranylgeranyl acetone and an activator of a heat shock response;
   (s) the composition or product of manufacture of (r), wherein the combination of compounds comprises or consists of geranylgeranyl acetone (GGA) or an analog of geranylgeranyl acetone and geranylgeranylacetone or gernate, zinc, tin, salicylates, dexamethasone, cocaine, nicotine, alpha-adrenergic agonist, ppar-gamma agonist, a geldanamycin, biomolecular-geldanamycin, cyclopentanone, a prostanoid, a prostaglandin, a thromboxane, a prostacyclins, enprostil, paracetamol or acetaminophen (N-(4-hydroxyphenyl)-acetamide), ketotiphen, levamisole, diazepam, VALIUM™, bromocriptine, PARLODEL™, or dopamine (4-(2-aminoethyl)benzene-1,2-diol);
   (t) the composition or product of manufacture of any of (a) to (s), wherein the combination of compounds comprises or consists of at least one compound selected from each of three groups selected from the group consisting of Group A, Group B, Group C, Group D, Group E, Group F, Group G, Group H, Group I, Group J, Group K, Group L and Group M;
   (u) the composition or product of manufacture of (t), wherein the combination of compounds comprises or consists of at least one compound selected from the group consisting of Group A, Group B and Group C; Group A, Group B and Group D; Group A, Group B and Group E; Group A, Group D and Group E; Group B, Group C and Group D; Group B, Group C and Group E; Group C, Group E and Group F;
   (v) the composition or product of manufacture of any of (a) to (u) formulated as a pharmaceutical composition;
   (w) the composition or product of manufacture of (v) formulated for administration intravenously, topically, orally, by inhalation, by infusion, by injection, intraperitoneally, intramuscularly, subcutaneously, intra-aurally, by intra-articular administration, by intra-mammary administration, rectally, by topical administration or by absorption through epithelial and/or mucocutaneous linings;
   (x) the composition or product of manufacture of (w) formulated as an aqueous suspension, a solid, a liquid, a powder, an emulsion, a lyophilized powder, a spray, a cream, a lotion, a controlled release formulation, a tablet, a pill, a gel, a liposome, on a patch, in an implant, on a tape, a dragee, a capsule, a lozenge, a gel, a syrup, a slurry and/or a suspension, or the composition is formulated with a solid excipient, a carbohydrate, a protein filler, a sugar, a lactose, a sucrose, a mannitol, a sorbitol, a starch, a cellulose, a methyl cellulose, a hydroxypropylmethyl-cellulose, a sodium carboxymethylcellulose, a gum, an arabic gum, a tragacanth, a gelatin, a collagen, a disintegrating or solubilizing agent, a cross-linked polyvinyl pyrrolidone, an agar, an alginic acid or alginic salt, or a sodium alginate;
   (y) the composition or product of manufacture of any of (a) through (x), wherein the composition or product of manufacture comprises or consists of the combination of geranyl geranyl acetone (GGA) and captopril; GGA and CAPOTIN™; an analog of GGA and captopril; an analog of and CAPOTIN™; teprenone (CAS Registry Number 6809-52-5) and captopril; teprenone and CAPOTIN™; SELBEX™ and CAPOTIN™; or VT-211™; or
   (z) the composition or product of manufacture of any of (a) through (x), wherein the composition or product of manufacture comprises or consists of the combination of geranyl geranyl acetone (GGA) and etodolac; GGA and LODINE™); an analog of GGA and etodolac; an analog of GGA and LODINE™; teprenone (CAS Registry Number 6809-52-5) and etodolac; teprenone and LODINE™; SELBEX™ and etodolac; SELBEX™ and LODINE™); or VT-212™.
101. The composition or product of manufacture of aspect 100, comprising a blister pack, clamshell or tray, wherein the combination of drugs is formulated for unit dosage administration to an individual in need thereof at the same time, and each unit dosage is contained within one blister in the blister pack, or compartment in the clamshell or tray.
102. The composition or product of manufacture of aspect 100, comprising a pill, capsule, tablet, tab, geltab or implant, wherein the combination of drugs is formulated for unit dosage administration to an individual in need thereof at the same time, and each unit dosage is contained within one pill, capsule, tablet, tab, geltab or implant.
103. A nanoparticle or microparticle comprising the composition or product of manufacture of aspect 100.
104. Use of the composition or product of manufacture of aspect 100, for the manufacture of a medicament.
105. Use of the composition or product of manufacture of aspect 100, for the manufacture of a medicament for to treat, ameliorate, diminish, improve and/or inhibit unwanted side effects and/or disease state symptoms associated with or caused by anger; anemia; anorexia; anorexia-cachexia; anxiety; atrophy or muscle atrophy; cachexia; cancer cachexia; cognitive impairment; cytoprotection deficiency; depression or despair; difficulties with activities of daily living; discomfort; emesis; erectile or sexual dysfunction or sexual disinterest; excessive sympathoneural drive; fatigue; febrile neutropenia; frustration; hair loss; heart failure; infection, bacterial infection, viral infection, mycobacterium infection, yeast infection, protozoan infection, inflammation; intolerance to a medical therapy; lack of appetite; lack of energy; lack of motivation; mucositis; oral mucositis; digestive mucositis; esophageal mucositis; intestinal mucositis; myeloprotection deficiency; neutropenia; rash; pain; reduced physical activity; wasting; worrying; pruritis; xerostomia; cardiotoxicity; ototoxicity; nephrotoxicity; peripheral neuropathy and/or stress or anxiety related to any of the above.
106. A method for treating, ameliorating, diminishing, improving and/or inhibiting unwanted side effects and/or disease state symptoms associated with or caused by
   (i) anger; anemia; anorexia; anorexia-cachexia; anxiety; atrophy or muscle atrophy; cachexia; cancer cachexia; cognitive impairment; cytoprotection deficiency; depression or despair; difficulties with activities of daily living; discomfort; emesis; erectile or sexual dysfunction or sexual disinterest; excessive sympathoneural drive; fatigue; febrile neutropenia; frustration; hair loss; heart failure; infection, bacterial infection, viral infection, mycobacterium infection, yeast infection, protozoan infection, inflammation; intolerance to a medical therapy; lack of appetite; lack of energy; lack of motivation; oral mucositis; digestive mucositis; esophageal mucositis; intestinal mucositis; myeloprotection deficiency; neutropenia; rash; pain; reduced physical activity; wasting; worrying; pruritis; xerostomia; cardiotoxicity; ototoxicity; nephrotoxicity; peripheral neuropathy and/or stress or anxiety related to any of the above,
   (ii) mucositis or oral mucositis;
   (iii) radiation therapy or chemotherapy; and/or
   (iv) radiation therapy for cancer or cancer chemotherapy;
   the method comprising
   (a) administering to an individual in need thereof an effective amount of at least one composition or product of manufacture of aspect 100,
      thereby treating, ameliorating, diminishing, improving and/or inhibiting the unwanted side effects and/or disease state symptoms associated with or caused by
      (i) anger; anemia; anorexia; anorexia-cachexia; anxiety; atrophy or muscle atrophy; cachexia; cancer cachexia; cognitive impairment; cyto-protection deficiency; depression or despair; difficulties with activities of daily living; discomfort; emesis; erectile or sexual dysfunction or sexual disinterest; excessive sympathoneural drive; fatigue; febrile neutropenia; frustration; hair loss; heart failure; infection, bacterial infection, viral infection, mycobacterium infection, yeast infection, protozoan infection, inflammation; intolerance to a medical therapy; lack of appetite; lack of energy; lack of motivation; oral mucositis; digestive mucositis; esophageal mucositis; intestinal mucositis; myeloprotection deficiency; neutropenia; rash; pain; reduced physical activity; wasting; worrying; pruritis; xerostomia; cardiotoxicity; ototoxicity; nephrotoxicity; peripheral neuropathy and/or stress or anxiety related to any of the above,
      (ii) mucositis or oral mucositis;
      (iii) radiation therapy or chemotherapy; or
      (iv) radiation therapy for cancer or cancer chemotherapy;
   (b) the method of (a), wherein the administered composition comprises or consists of a geranylgeranyl compound, a geranylgeranyl acetone (GGA) or an analog of geranylgeranyl acetone (GGA), at a dosage regimen of between about 0.10 mg to about 20.00 gm per day, or between about 30 mg to 3 gm per day; or
   (c) the method of (a), wherein the administered composition comprises or consists of an angiotensin converting enzyme inhibitor at a dosage regimen of between about 0.10 mg to about 10.00 gm per day, or between about 10 mg to 450 mg per day.
107. A treatment or therapy comprising administering to an individual in need thereof an effective amount of at least one composition or product of manufacture of aspect 100.
108. A treatment or therapy comprising administering to an individual in need thereof an effective amount of at least one composition or product of manufacture of aspect 100, in conjunction (together) with a drug therapy, a chemotherapy or a radiation therapy.
109. A kit comprising (a) at least one composition or product of manufacture of aspect 100; or (b) the kit of (a), further comprising instructions to practice the method of aspect 106, or the treatment or therapy of aspect 107 or aspect 108.
110. A method for increasing drug regimen compliance of stressed, challenged or non-compliant patient population comprising use of
   (a) the therapeutic combination of any of aspects 1 to 53; the pharmaceutical composition or formulation of any of aspects 54 to 56; the composition or product of manufacture of aspect 70; the nanoparticle, microparticle, nanoliposome or liposome of aspect 74; at least one composition or product of manufacture of aspect 100; or a blister pack or a plurality of blister packettes, a blister package, a lidded blister or a blister card or packet, a clamshell, a tray or a shrink wrap of aspect 91 or aspect 92, or the paper, plastic or cellophane package or a plurality of packettes of aspect 93 or aspect 94; or, the kit of aspect 109; or
   (b) the method of (a), further comprising use of instructions to practice the method of aspect 106, or the treatment or therapy of aspect 107 or aspect 108.
111. The method of aspect 110, wherein the stressed, challenged or non-compliant patient population comprises a cancer patient population; or a patient population having mild or severe mental retardation, slow cognition, dementia, senility, Alzheimer's disease, traumatic brain injury, chemical brain damage or a mental disease; or post-traumatic stress disorder, traumatic war neurosis, post-traumatic stress syndrome (PTSS) or a physical disability or blindness.
112. The method of aspect 110, wherein the mental disease comprises a dissociative disorder, an obsessive-compulsive disorder, a delusional disorder, a schizophrenia, a mania, a panic disorder, depression, dyslexia or a learning disability.
113. Use of the therapeutic combination of any of aspects 1 to 53; the pharmaceutical composition or formulation of any of aspects 54 to 56; the composition or product of manufacture of aspect 70; the nanoparticle, microparticle, nanoliposome or liposome of aspect 74; at least one composition or product of manufacture of aspect 100; or a blister pack or a plurality of blister packettes, a blister package, a lidded blister or a blister card or packet, a clamshell, a tray or a shrink wrap of aspect 91 or aspect 92, or the paper, plastic or cellophane package or a plurality of packettes of aspect 93 or aspect 94; or, the kit of aspect 109; for making a deliverable package for increasing drug regimen compliance of a stressed, challenged or non-compliant patient population.
114. The use of aspect 113, wherein the stressed, challenged or non-compliant patient population comprises a cancer patient population; or a patient population having mild or severe mental retardation, slow cognition, dementia, senility, Alzheimer's disease, traumatic brain injury, chemical brain damage or a mental disease; or post-traumatic stress disorder, traumatic war neurosis, post-traumatic stress syndrome (PTSS) or a physical disability or blindness.
115. The use of aspect 114, wherein the mental disease comprises a dissociative disorder, an obsessive-compulsive disorder, a delusional disorder, a schizophrenia, a mania, a panic disorder, depression, dyslexia or a learning disability.

## Claims

1. A therapeutic combination of drugs used for treating, ameliorating or preventing a cancer, wherein the therapeutic combination comprises or consists of:
(1) a beta adrenergic receptor antagonist;
(2) a non-steroidal anti-inflammatory drug (a NSAID); and
(3) an anti-microtubule inhibitor and/or a cisplatin or equivalent, a cisplatinum or equivalent, a *cis*-diamminedichloridoplatinum(II) (CDDP) or equivalent, a carboplatin or equivalent, or an oxaloplatin or equivalent.

2. The therapeutic combination of claim 1, wherein the non-steroidal anti-inflammatory drug (NSAID) comprises:
(a) a cyclooxygenase (COX) or prostaglandin synthase inhibitor;
(b) the COX inhibitor of (a) comprises or consists of an etodolac or equivalent; a naproxen or equivalent; a celecoxib or equivalent; a rofecoxib or equivalent; a etoricoxib or equivalent; a valdecoxib or equivalent; a parecoxib or equivalent; a nabumetone or equivalent; a diclofenac (2-(2,6-dichloranilino) phenylacetic acid) or equivalent; or, a lumiracoxib or equivalent; or
(c) a neuropathic pain analgesic, optionally which comprises or consists of a gabapentin or a pregabalin.

3. The therapeutic combination of claim 1:
(a) wherein the beta adrenergic receptor antagonist (beta blocker) comprises a propranolol or equivalent;
(b) wherein the beta adrenergic receptor antagonist (a beta blocker) comprises a propranolol or equivalent and the non-steroidal anti-inflammatory drug (a NSAID) comprises an etodolac or equivalent;
(c) wherein the anti-microtubule inhibitor comprises at least one of: a paclitaxel or equivalent, a docetaxel or equivalent, a larotaxel, a tesetaxel or an ortataxel or equivalent;
(d) comprising: a propranolol; an etodolac; and at least one of a paclitaxel or equivalent, a docetaxel or equivalent, a larotaxel, a tesetaxel or an ortataxel or equivalent; or
(e) comprising: a propranolol; an etodolac; and, at least one of a cisplatin or equivalent, a cisplatinum or equivalent, a *cis*-diamminedichloridoplatinum(II) (CDDP) or equivalent, a carboplatin or equivalent, an oxaloplatin or equivalent; optionally further comprising at least one of a paclitaxel or equivalent, a docetaxel or equivalent, or a larotaxel, a tesetaxel or an ortataxel or equivalent.

4. The therapeutic combination of claim 1, wherein:
(a) the dosage of etodolac ranges from about 200 mg to 400 mg a day, or, about 10, 15, 20, 25, 30, 35, 40, 45, 50, 75, 80, 85, 90, 100, 150, 200, 250, 300, 350, 400, 450, 500, 600, 700, 800, 900 or 1000 mg or more; or
(b) the dosage of propranolol ranges from 10 to 320 mg per day based on heart rate and blood pressure of the individual, or, about 10, 15, 20, 25, 30, 35, 40, 45, 50, 75, 80, 85, 90, 100, 150, 200, 250, 300, 350, 400, 450, 500, 600, 700, 800, 900 or 1000 mg or more.

5. The therapeutic combination of any of claims 1 to 4, wherein one, two, three, four or more drugs of the therapeutic combination are formulated as separate compositions, or one, two, three, four or more drugs of the therapeutic combination are formulated into one composition or drug formulation.

6. The therapeutic combination of any of claims 1 to 4, wherein:
(a) one, two, three, four or more or all of the drugs of the therapeutic combination are packaged individually, or are packaged together, or packaged in any combination, in a single package, a plurality of packages or packettes, or a blister packet, lidded blister or blister card or packets, or a shrink wrap;
(b) the beta adrenergic receptor antagonist (beta blocker) or equivalent, the propranolol or equivalent; the non-steroidal anti-inflammatory drug (NSAID) or equivalent, the etodolac or equivalent; and the at least one of a cisplatin or equivalent, a cisplatinum or equivalent, a *cis*-diamminedichloridoplatinum(II) (CDDP) or equivalent, a carboplatin or equivalent, an oxaloplatin or equivalent; an oxaloplatin or equivalent; a paclitaxel or equivalent; a docetaxel or equivalent; a larotaxel, a tesetaxel or an ortataxel or equivalent, are packaged individually in a single package, a plurality of packages or packettes, or a blister packet, lidded blister or blister card or packets, or a shrink wrap;
(c) one, two, three, four or more or all of the drugs of the therapeutic combination are packaged together or in any combination in a single package, a plurality of packages or packettes, or a blister packet, lidded blister or blister card or packets, or a shrink wrap; or
(d) the therapeutic combination of any of (a) to (c), wherein one, two, three, four or more or all of the drugs are released upon opening of the single package, plurality of packages or packettes, blister packet, lidded blister, blister card or packets or shrink wrap.

7. The therapeutic combination of any of claims 1 to 4, wherein: the beta adrenergic receptor antagonist (beta blocker) or equivalent, or the propranolol or equivalent; the non-steroidal anti-inflammatory drug (NSAID) or equivalent, or the etodolac or equivalent; the at least one of a cisplatin or equivalent, a cisplatinum or equivalent, a *cis-*diamminedichloridoplatinum(II) (CDDP) or equivalent, a carboplatin or equivalent, an oxaloplatin or equivalent; an oxaloplatin or equivalent; and, the at least one of a paclitaxel or equivalent, a docetaxel or equivalent, or a larotaxel, a tesetaxel or an ortataxel or equivalent, are packaged together in a single package, a plurality of packages or packettes, or a blister packet, lidded blister or blister card or packets, or a shrink wrap, and one, two, three, four or more or all of the drugs are released upon opening of the single package, plurality of packages or packettes, blister packet, lidded blister, blister card or packets or shrink wrap.

8. The therapeutic combination of any of claims 1 to 7, wherein:
(a) one, two, three, four or more or all of the drugs of the therapeutic combination are formulated or manufactured as a parenteral formulation, an aqueous solution, a liposome, an injectable solution, a tablet, a pill, a lozenge, a capsule, a caplet, a patch, a spray, an inhalant, a powder, a freeze-dried powder, an inhalant, a patch, a gel, a geltab, a nanosuspension, a nanoparticle, a nanoliposome, a microgel, a pellet, a suppository or any combination thereof;
(b) one, two, three, four or more or all of the drugs of the therapeutic combination are formulated or manufactured together in one parenteral formulation, one aqueous solution, one liposome, one injectable solution, one freeze-dried powder, one feed, one food, one food supplement, one pellet, one lozenge, one liquid, one elixir, one aerosol, one inhalant, one adhesive, one spray, one powder, one freeze-dried powder, one patch, one tablet, one pill, one capsule, one gel, one geltab, one lozenge, one caplet, one nanosuspension, one nanoparticle, one nanoliposome, one microgel or one suppository;
(c) the therapeutic combination of drugs are formulated for administration intravenously, topically, orally, by inhalation, by infusion, by injection, by inhalation, intraperitoneally, intramuscularly, subcutaneously, intra-aurally, for intra-articular administration, for intra-mammary administration, for topical administration or for absorption through epithelial or mucocutaneous linings;
(d) the therapeutic combination of drugs are formulated, packaged, arranged or clustered: (i) in a chrono-dosing arrangement or pattern; or (ii) individually,
(e) the therapeutic combination of drugs, is formulated, packaged or designed for drug regimen compliance of a cancer patient population, a pediatric or geriatric population, or a mentally compromised patient population; or
(f) the therapeutic combination of drugs, is formulated, packaged or designed for drug regimen compliance of a cancer patient population having a mild or severe mental retardation, slow cognition, a dementia, senility, Alzheimer's disease, a traumatic brain injury, chemical brain damage, a mental disease, a dissociative disorder, obsessive-compulsive disorder, a delusional disorder, schizophrenia, mania, a panic disorder, a post-traumatic stress disorder, traumatic war neurosis, post-traumatic stress syndrome (PTSS), a physical disability or blindness.

9. The therapeutic combination of any of claims 1 to 8, wherein:
(a) the therapeutic combination of drugs is packaged in dosages that match a chrono-dosing regimen to match an optimal dose for the time of day;
(b) the beta adrenergic receptor antagonist (beta blocker) or equivalent, or the propranolol or equivalent; the non-steroidal anti-inflammatory drug (NSAID) or equivalent, or the etodolac or equivalent; the at least one of a cisplatin or equivalent, a cisplatinum or equivalent, a *cis*-diamminedichloridoplatinum(II) (CDDP) or equivalent, a carboplatin or equivalent, an oxaloplatin or equivalent; an oxaloplatin or equivalent; and/or the at least one of a paclitaxel or equivalent, a docetaxel or equivalent, or a larotaxel, a tesetaxel or an ortataxel or equivalent, are packaged in dosages that match a chrono-dosing regimen to match an optimal dose for the time of day;
(c) the beta adrenergic receptor antagonist (beta blocker) or equivalent, or the propranolol or equivalent; the non-steroidal anti-inflammatory drug (NSAID) or equivalent, or the etodolac or equivalent; the at least one of a cisplatin or equivalent, a cisplatinum or equivalent, a *cis*-diamminedichloridoplatinum(II) (CDDP) or equivalent, a carboplatin or equivalent, an oxaloplatin or equivalent; an oxaloplatin or equivalent; and/or the at least one of a paclitaxel or equivalent, a docetaxel or equivalent, or a larotaxel, a tesetaxel or an ortataxel or equivalent, are packaged in dosages that match a chrono-dosing regimen comprising:
(i) in the AM, 20 mg beta adrenergic receptor antagonist (a beta blocker), e.g., a propranolol or equivalent, 200mg NSAID, e.g., an etodolac or equivalent; in the afternoon, 10 mg beta blocker, 200 mg NSAID, e.g., an etodolac or equivalent; in the PM, 10 mg beta blocker, 400 mg NSAID;
(ii) in the AM 40 mg beta adrenergic receptor antagonist (a beta blocker), e.g., a propranolol or equivalent, 200 mg NSAID, e.g., an etodolac or equivalent; in the afternoon 20 mg beta blocker, 200 mg NSAID; in the evening, 20 mg propranolol, 400 mg NSAID;
(iii) in the AM 80 mg beta adrenergic receptor antagonist (a beta blocker), e.g., a propranolol or equivalent, 200 mg NSAID; in the afternoon 40 mg beta blocker, 200 mg NSAID, in the evening 40 mg, NSAID; or
(iv) a dose escalation comprising a regimen of (a) to (b) to (c); or
(d) the beta adrenergic receptor antagonist (beta blocker) or equivalent, or the propranolol or equivalent; the non-steroidal anti-inflammatory drug (NSAID) or equivalent, or the etodolac or equivalent; the at least one of a cisplatin or equivalent, a cisplatinum or equivalent, a *cis*-diamminedichloridoplatinum(II) (CDDP) or equivalent, a carboplatin or equivalent, an oxaloplatin or equivalent; an oxaloplatin or equivalent; and/or the at least one of a paclitaxel or equivalent, a docetaxel or equivalent, or a larotaxel, a tesetaxel or an ortataxel or equivalent, are packaged in dosages that match a chrono-dosing regimen comprising:
Start: AM, 20 mg propranolol, 200mg etodolac; afternoon, 10 mg propranolol, 200 mg etodolac; PM 5 mg propranolol, 400 mg etodolac;
Dose Escalation 1: AM 40 mg propranolol, 200 mg etodolac; afternoon 20 mg propranolol, 200 mg etodolac; evening, 10 mg propranolol, 400 mg etodolac; or
Dose escalation 2: AM 80 mg propranolol, 200 mg etodolac; afternoon 40 mg propranolol, 200 mg etodolac, evening 20 mg, etodolac.

10. The therapeutic combination of any of claims 1 to 9, wherein the therapeutic drug combination is formulated for administration once a day, b.i.d. (twice a day) or t.i.d. (three times a day), or weekly, or biweekly, or monthly.

11. A pharmaceutical composition or formulation comprising the therapeutic combination of any one of claims 1 to 10, and optionally further comprising a pharmaceutically acceptable excipient.

12. A kit, container, blister pack or package, clamshell, shrinkwrap or tray comprising the therapeutic combination of any one of claims 1 to 10,
and optionally the therapeutic combination of drugs are arranged or clustered in the kit, container, blister pack or package, clamshell, shrinkwrap or tray: (a) in a chrono-dosing arrangement or pattern; or (b) individually,
and optionally the kit, container, blister pack or package, clamshell, shrinkwrap or tray, or the therapeutic combination of drugs, is formulated, packaged or designed for drug regimen compliance of a cancer patient population, a pediatric or geriatric population, or a mentally compromised patient population,
and optionally the kit, container, blister pack or package, clamshell, shrinkwrap or tray, or the therapeutic combination of drugs, is formulated, packaged or designed for drug regimen compliance of a cancer patient population having a mild or severe mental retardation, slow cognition, a dementia, senility, Alzheimer's disease, a traumatic brain injury, chemical brain damage, a mental disease, a dissociative disorder, obsessive-compulsive disorder, a delusional disorder, schizophrenia, mania, a panic disorder, a post-traumatic stress disorder, traumatic war neurosis, post-traumatic stress syndrome (PTSS), a physical disability or blindness.

13. Use of the therapeutic combination of any of claims 1 to 10, or the pharmaceutical composition or formulation of claim 11, in the manufacture of a medicament or pharmaceutical composition for treating, ameliorating or preventing a trauma, condition or disease comprising: a cancer or a dysfunctional cell condition.

14. The therapeutic combination of any of claims 1 to 10, or the pharmaceutical composition or formulation of claim 11 for use in a method for treating or ameliorating a cancer or a dysfunctional cell condition.

15. The use according to claim 13, or the therapeutic combination or pharmaceutical composition or formulation for use according to claim 14, wherein the cancer or dysfunctional cell condition comprises (is) any metastatic or benign tumor, and the use is for treating (killing, eliminating, stopping the growth and/or metastasis of) cancer stem cells or cancer cells from: lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, chronic or acute leukemia, lymphocytic lymphomas, cancer of the bladder, cancer of the kidney or ureter, renal cell carcinoma, carcinoma of the renal pelvis, a neoplasm of the central nervous system (CNS), primary CNS lymphoma, spinal axis tumors, brain stem glioma or pituitary adenoma, and any combination thereof.
